# EUROPÄISCHE PATENTANMELDUNG

(11) **EP 2 251 440 A2**
(43) Veröffentlichungstag der Anmeldung: **17.11.2010**
(21) Anmeldenummer: 10007053.1
(22) Anmeldetag: 20.04.1999
(51) Int. Cl.: C12Q 1/68, C07H 1/08, G01N 33/543

(54) **Verfahren und Mittel zur Isolierung und Reinigung von Nukleinsäuren an Oberflächen**

(30) Priorität: 23.10.1998 WO PCT/EP98/06756
(62) Teilanmeldung aus: 99923428.9
(71) Anmelder: Qiagen GmbH, 40724 Hilden (DE)
(72) Erfinder: Oelmüller, Uwe, 40699 Erkrath (DE); Gauch, Simone, 4059 Basel (CH); Bastian, Helge, Creve Coeur, MO 63141 (US); Ullmann, Susanne, 40723 Hilden (DE)
(74) Vertreter: Polypatent

(57) **Zusammenfassung**

Die vorliegende Erfindung betrifft neue Verfahren und Geräte zur Isolierung und Reinigung von Nukleinsäuren an Oberflächen. Die Erfindung ist auf Verfahren gerichtet, die Oberflächen, z.B. poröse Membranen verwenden, an welche die Nukleinsäuren auf einfache Weise aus der die Nukleinsäuren enthaltenden Probe immobilisiert und mittels ebenso einfacher Verfahrensschritte wieder abgelöst werden können, wobei es die erfindungsgemäß einfache Prozeßführung ermöglicht, die Verfahren insbesondere vollautomatisch durchführen zu können. Ein weiterer Aspekt der vorliegenden Erfindung ist darauf gerichet, Nukleinsäuren an eine immobile Phase, insbesondere an eine Membran, in der Art und Weise zu binden, dass sie in einem folgenden Reaktionsschritt ohne weiteres wieder von dieser Phase abgelöst werden können und ggf. in weiteren Anwendungen - wie z. B. Restriktionsverdauung, RT, PCR oder RT-PCR oder in jedweder anderen oben genannten geeigneten Analyse- bzw. Enzymreaktion eingesetzt werden können. Schließlich ist die Erfindung auf spezielle Isoliergefässe gerichtet, mit denen die erfindungsgemäßen Verfahren durchgeführt werden können.

## Beschreibung

Die vorliegende Erfindung betrifft neue Verfahren und Geräte zur Isolierung und Reinigung von Nukleinsäuren an Oberflächen.

Es ist seit langem bekannt, dass die genetische Herkunft und funktionelle Aktivität einer Zelle durch Studien ihrer Nukleinsäuren bestimmt und untersucht werden kann. Die Analysen der Nukleinsäuren ermöglichen den direkten Zugriff auf die Ursache der Aktivitäten von Zellen. Sie sind somit indirekten, konventionellen Methoden, wie z.B. dem Nachweis von Stoffwechselprodukten, potentiell überlegen. Daher ist für die Zukunft mit einer starken Verbreitung von Nukleinsäureanalysen zu rechnen. So werden molekularbiologische Analysen bereits in vielen Bereichen eingesetzt, z.B. in der medizinischen und klinischen Diagnostik, in der Pharmazie bei der Entwicklung und Evaluierung von Arzneimitteln, in der Lebensmittelanalytik sowie bei der Überwachung der Lebensmittelherstellung und der Lebensmittelkontrolle, in der Agrarwirtschaft bei der Züchtung von Nutzpflanzen und Nutztieren sowie in der Umweltanalytik und in vielen Forschungsgebieten. Hierzu zählen zum Beispiel die Vaterschaftsanalyse, Gewebetypisierungen, Identifizierung von Erbkrankheiten, Genomanalyse, molekulare Diagnostik wie z.B. bei der Identifizierung von Infektionskrankheiten, transgene Forschung, Grundlagenforschung auf dem Gebiet der Biologie und der Medizin sowie zahlreiche verwandte Arbeitsgebiete.

Durch die Analyse der RNA, speziell der mRNA in Zellen, lassen sich die Aktivitäten von Genen direkt bestimmen. Die quantitative Analyse von Transkriptmustem (mRNA-Mustem) in Zellen durch moderne molekularbiologische Methoden, wie z.B. Echtzeit-Reverse Transkriptase PCR ("Real-Time RT-PCR") oder Genexpressions-Chip-Analysen ermöglicht z.B. die Erkennung fehlerhaft exprimierter Gene, wodurch z.B. Stoffwechselkrankheiten, Infektionen oder die Entstehung von Krebs erkannt werden können. Die Analyse der DNA aus Zellen durch molekularbiologische Methoden, wie z.B. PCR, RFLP, AFLP oder Sequenzierung ermöglicht z.B. den Nachweis genetischer Defekte oder die Bestimmung des HLA-Typs sowie anderer genetischer Marker.

Die Analyse genomischer DNA und RNA wird auch zum direkten Nachweis von infektiösen Erregern, wie Viren, Bakterien usw. eingesetzt.

Dabei besteht eine generelle Schwierigkeit darin, biologische bzw. klinische Probenmaterialien so aufzubereiten, dass die in ihnen enthaltenen Nukleinsäuren direkt in die jeweilige Analysenmethode eingesetzt werden können. Gerade die direkte Verwendbarkeit bei guter Ausbeute und hoher Qualität der Nukleinsäuren; bei gleichzeitig hoher Reproduzierbarkeit, ist allerdings bei höheren Probenzahlen wichtig, wenn die Analysen automatisch ablaufen sollen.

Aus dem Stand der Technik sind bereits zahlreiche Verfahren zur Reinigung von DNA bekannt. So ist es bekannt, Plasmid-DNA beispielsweise zum Zwecke des Klonierens oder auch für andere experimentelle Vorhaben nach dem Verfahren von Birnboim [Methods in Enzymology 100 (1983), S. 243] zu reinigen. Nach diesem Verfahren wird ein geklärtes Lysat bakteriellen Ursprungs einem Caesiumchlorid Gradienten ausgesetzt und über einen Zeitraum von 4 bis 24 Stunden zentrifugiert. Diesem Verfahrensschritt folgt gewöhnlicherweise die Extraktion und die Präzipitation der DNA. Dieses Verfahren ist mit dem Nachteil verbunden, dass es zum einen apparativ sehr aufwendig und zum anderen sehr zeit- und kostenintensiv sowie nicht automatisierbar ist.

Andere Methoden, bei denen geklärte Lysate eingesetzt werden, um DNA zu isolieren, sind die lonenaustauschchromatographie [Colpan et al., J. Chromatog. 296 (1984), S. 339] und die Gelfiltration [Moreau et al. Analyt. Biochem. 166 (1987), S. 188]. Diese Verfahren bieten sich in erster Linie als Ersatz für den Caesiumchlorid-Gradienten an, machen aber ein aufwendiges System für die Lösungsmittelversorgung sowie die Präzipitation der so gewonnenen DNA-Fraktionen erforderlich, da sie gewöhnlicherweise Salze in hoher Konzentration enthalten und sehr verdünnte Lösungen darstellen.

Marko et al. [Analyt. Biochem. 121 (1982), S. 382] sowie Vogelstein et al. [Proc. Nat. Acad. Sci. 76 (1979), S. 615] erkannten, dass, falls die DNA aus Nukleinsäure-enthaltenden Extrakten hohen Konzentrationen von Natriumiodid oder Natriumperchlorat ausgesetzt wird, nur die DNA an mechanisch fein zerkleinerten Glas-Scintillationsröhrchen sowie zerkleinerten Glasfasermembranen bzw. Glasfiberplatten bindet, während RNA und Proteine nicht binden. Die so gebundene DNA kann ggfs. mit Wasser eluiert werden.

So wird in der WO 87/06621 die Immobilisierung von Nukleinsäuren an einer PVDF-Membran beschrieben. Allerdings werden die an die PVDF-Membran gebundenen Nukleinsäuren anschließend nicht eluiert, sondern die Membran wird samt gebundener Nukleinsäuren direkt in einen PCR-Ansatz eingebracht. Letztendlich wird in dieser internationalen Patentanmeldung und in der weiteren Literatur jedoch die Lehre offenbart, dass hydrophobe Oberflächen bzw. Membranen im allgemeinen zuvor mit Wasser oder Alkohol benetzt werden müssen, um die Nukleinsäuren in halbwegs befriedigenden Ausbeuten immobilisieren zu können.

Für eine Reihe von modernen Applikationen, wie z. B. der PCR-, der Reversed-Transcription-PCR, SunRise, LCR, branched-DNA, NASBA, TaqMan-Technologie und ähnlicher Echtzeitquantifizierungsverfahren für PCR, SDA, DNA-und RNA-Chips und -Arrays zur Genexpressions- und Mutationsanalytik, differential display Analytik, RFLP, AFLP, cDNA-Synthesen, oder subtraktive Hybridisierung, ist es auf der anderen Seite jedoch absolut notwendig, die Nukleinsäuren direkt von der festen Phase lösen zu können. Hierzu ist der WO 87/06621 die Lehre zu entnehmen, dass die Nukleinsäure zwar von den dort eingesetzten Membranen wiedergewonnen werden kann, dass diese Wiedergewinnung jedoch sehr problematisch ist und bei weitem nicht zur quantitativen Isolierung von Nukleinsäuren geeignet ist. Daneben fallen die so gewonnenen Nukeinsäuren in vergleichsweise sehr hoher Verdünnung an - ein Umstand, der weitere Folgeschritte zwecks Konzentrierung und Isolierung zwangsläufig erforderlich macht.

Unter Nukleinsäurenprobe im Sinne der vorliegenden Erfindung sollen alle wäßrigen oder sonstigen Lösungen von Nukleinsäuren und ebenso alle Nukleinsäuren enthaltenden Materialien und Proben, wie biologische Proben und Materialien, Nahrungsmittel etc. verstanden werden. Dabei wird im Sinne der vorliegenden Erfindung eine Nukleinsäure enthaltende Probe oder ein Material durch eine Probe bzw. einen Probenansatz definiert, die bzw. der Nukleinsäuren enthält. Unter biologisches Material bzw. biologischer Probe sollen dabei z. B. zellfreies Probenmaterial, Plasma, Körperflüssigkeiten - wie beispielsweise Blut, Sputum, Urin, Faeces, Sperma, Zellen, Serum, Leukozytenfraktionen, Crusta Phlogistica, Abstriche, Gewebeproben jeder Art, Gewebeteile und Organe, Lebensmittelproben, die freie oder gebundenen Nukleinsäuren oder nukleinsäurehaltige Zellen enthalten, Umweltproben, die freie oder gebundenen Nukleinsäuren oder nukleinsäurehaltige Zellen enthalten, Pflanzen und Pflanzenteile, Bakterien, Viren, Hefen und andere Pilze, andere Eukaryoten und Prokaryoten etc., wie sie beispielsweise in der Europäischen Patentanmeldung Nr. 95909684.3 offenbart sind, auf die hiermit inhaltlich Bezug genommen wird - oder auch freie Nukleinsäuren fallen. Unter Nukleinsäuren fallen im Sinne der vorliegenden Erfindung alle möglichen Arten von Nukleinsäuren, wie z. B. Ribonukleinsäuren (RNA) und Desoxyribonukleinsäuren (DNA) in allen Längen und Konfigurationen, wie Doppelstrang, Einzelstrang, circulär und linear, verzweigt etc.; monomere Nukleotide, Oligomere, Plasmide, virale und bakterielle DNA und RNA, sowie genomische oder sonstige nichtgenomische DNA und RNA aus Tier- und Pflanzenzellen oder anderen Eukaryoten, tRNA, mRNA in prozessierter und unprozessierter Form, hn-RNA, rRNA und cDNA sowie alle anderen denkbaren Nukleinsäuren.

Aus den oben genannten Gründen stellen die aus dem Stand der Technik bekannten Verfahren - insbesondere im Hinblick auf eine Automatisierung des Verfahrensablaufs zur Nukleinsäuregewinnung - keinen geeigneten Ausgangspunkt für eine verfahrenstechnisch möglichst einfache und quantitative Isolierung von Nukleinsäuren dar.

Es ist daher die Aufgabe der vorliegenden Erfindung, die Nachteile der aus dem Stand der Technik bekannten Verfahren zur Isolierung von Nukleinsäuren zu überwinden und Verfahren und Mittel zur Verfügung zu stellen, welche dazu geeignet sind, ohne erheblichen technischen Aufwand durchgeführt bzw. verwendet werden zu können.

Gelöst wird die Aufgabe erfindungsgemäß durch die Verfahren, dem Isoliergefäß bzw. Reaktionsgefäß, der Verwendung sowie dem Automaten und dem Kit gemäß denunten beschriebenen Aspekten der Erfindung und anhängenden Ansprüchen.

Weitere vorteilhafte Aspekte und Ausgestaltungen der Erfindung ergeben sich aus den abhängigen Patentansprüchen, der Beschreibung und den beigefügten Zeichnungen.

Dabei ist die Erfindung auf Verfahren gerichtet, die Oberflächen, z.B. poröse Membranen verwenden, an welche die Nukleinsäuren auf einfache Weise aus der die Nukleinsäuren enthaltenden Probe immobilisiert und mittels ebenso einfacher Verfahrensschritte wieder abgelöst werden können, wobei es die erfindungsgemäß einfache Prozeßführung ermöglicht, die Verfahren insbesondere vollautomatisch durchführen zu können.

Ein weiterer Aspekt der vorliegenden Erfindung ist darauf gerichet, Nukleinsäuren an eine immobile Phase, insbesondere an eine Membran, in der Art und Weise zu binden, dass sie in einem folgenden Reaktionsschritt ohne weiteres wieder von dieser Phase abgelöst werden können und ggf. in weiteren Anwendungen - wie z. B. Restriktionsverdauung, RT, PCR oder RT-PCR oder in jedweder anderen oben genannten geeigneten Analyse- bzw. Enzymreaktion eingesetzt werden können.

Unter einer Oberfläche wird im Sinne der vorliegenden Erfindung jede mikroporöse Trennschicht verstanden. Diese kann z.B. auch unmittelbar auf einem Untergrund aufliegen und somit nur von einer Seite zugänglich sein oder frei im Raum stehen. Von einer Membran im Sinne der vorliegenden Erfindung ist dann die Rede, wenn es sich um eine Trennschicht handelt, die von beiden Seiten zugänglich ist, als nicht mit ihrer gesamten Fläche auf einem undurchlässigen Untergrund aufliegt, sondern ganz frei oder nur an einzelnen Punkten unterstützt ist.

Unter Isolierung wird im Sinne der vorliegenden Erfindung dabei jede Anreicherung von Nukleinsäuren verstanden, bei der also die Konzentration der Nukleinsäuren erhöht und/oder der Anteil an Nichtnukleinsäure in einem Ansatz bzw. einer Probe verringert wird.

Die Erfindung ist gerichtet auf ein Verfahren zur Isolierung von Nukleinsäuren mit den folgenden Schritte:
- Beschicken einer Membran mit zumindest einer Nukleinsäurenprobe;
- Immobilisieren der Nukleinsäuren an der Membran;
- Ablösen der immobilisierten Nukleinsäuren von der Membran; und
- Abnehmen der abgelösten Nukleinsäuren durch die Membran hindurch,
wobei die Membran Nylon, Polysulfon, Polyethersulfon, Polycarbonat, Polyacrylat, Acrylatcopolymer, Polyurethan, Polyamid, Polyvinylchlorid, Polyfluorocarbonat, Polytetrafluorethylen, Polyvinylidendifluorid, Polyethylentetrafluorethylen-Copolymerisat, Polybenzimidazole, Polyethylenchlorotrifluorethylen-Copolymerisat, Polyimide, Polyphenylensulfid, Cellulose, Cellulose-Mischester, Cellulosenitrat, Celluloseacetat, Polyacrylnitrile, Polyacrylnitril-Copolymere, Nitrocellulose, Polypropylen und/oder Polyester enthält.

Auch andere Membranen, beispielsweise solche, die desweiteren in der vorliegenden Beschreibung angegeben sind, können für dieses erfindungsgemäße Verfahren verwendet werden.

Vorzugsweise erfolgt das Beschicken von oben und das Abnehmen nach unten, vorstellbar sind jedoch auch beispielsweise Durchflußverfahren, bei denen eine flachliegende Säule von der einen Seite mit Nukleinsäure enthaltender Lösung beschickt wird, welche nach Immobilisierung der Nukleinsäuren an der Membran durch diese hindurchtritt und am anderen Ende der Säule abgeführt werden kann.

Vorzugsweise kann die Membran in einem Behälter, beispielsweise der oben erwähnten oder einer anderen Säule, mit einer Zuführung und einer Abführung angeordnet sein und den gesamten Querschnitt des Behälters ausfüllen.

Die Membran kann beschichtet sein, wobei sie durch die Beschichtung hydrophob oder hydrophil gemacht sein kann.

Bisherige Isolierungsverfahren, gerade in Isoliersäulen, arbeiten mit relativ dicken Membranen bzw. Vliesen, um eine vollständige Isolierung der Nukleinsäuren zu erreichen. Dies führt jedoch dazu, dass beim Durchsaugen der Lösung durch die Membran ein relativ großes, sog. Totraumvolumen gegeben ist, nämlich das Volumen der Membran, aus der die Nukleinsäuren nur mittels einer größeren Menge eines Elutionspuffers gewonnen werden können. Hierdurch liegen die Nukleinsäuren nach der Elution jedoch stärker verdünnt vor, was für viele Anwendungen unerwünscht oder nachteilig ist. Daher verwendet eine bevorzugte Ausführungsform der Erfindung eine Membran, welche weniger als 1 mm, vorzugsweise weniger als 0,5, besonders bevorzugt weniger als 0,2, bespielsweise 0,1 mm dick ist.

Die Erfindung betrifft weiterhin ein Verfahren zur Isolierung von Nukleinsäuren mit den folgenden Schritten:
- Beschicken einer Oberfläche mit zumindest einer Nukleinsäurenprobe;
- Immobilisieren der Nukleinsäuren auf der Oberfläche; und
- Ablösen der immobilisierten Nukleinsäuren von der Oberfläche mit einem Elutionsmittel.

Dieses Verfahren ist **dadurch gekennzeichnet dass** die Ablösung bei einer Temperatur durchgeführt wird, deren Obergrenze bei 10°C oder darunter liegt und deren Untergrenze beim Gefrierpunkt des zum Ablösen verwendeten Elutionsmittels liegt, so dass das Elutionsmittel nicht einfriert. Es gilt also die Ungleichung 10°C >= T >= T_{S,EM},
wobei T die Temperatur der Ablösung und T_{S,EM} der Gefrierpunkt des Elutionsmittels ist. Es hat sich nämlich gezeigt, dass entgegen landläufiger Meinung eine Ablösung der Nukleinsäuren nahe dem Gefrierpunkt des Elutionsmittels durchaus möglich ist. Eine solche Elution bei niedriger Temperatur hat sogar den unerwarteten Vorteil, dass die Nukleinsäuren schonender behandelt werden und die Aktivität von evtl. im Ansatz noch vorhandenen Nukleasen (DNasen oder RNasen) nahe am Gefriepunkt praktisch zum Erliegen kommt, so dass die Degradierung der Nukleinsäuren vermindert oder gänzlich unterbunden wird.

Demgemäß sollte die Temperatur bei der Elution vorzugsweise noch tiefer liegen, beispielsweise unter 5°C liegt. Die Untergrenze kann auch bei 0°C oder -5°C liegen, wenn der Ansatz aufgrund seines lonengehalts bei dieser Temperatur noch flüssig ist. Auch die Obergrenze der Temperatur sollte möglichst niedrig liegen, so beispielsweise bei etwa 5°C.

Dieses erfindungsgemäße Verfahren erfordert mithin eine Kühlung des Elutionspuffers und kann eine Kühlung der weiteren verwendeten Lösungen und ggfs. auch des Isoliergefässes erfordern. Da gerade bei im Freiland durchgeführten Untersuchungen wie dem Screening von Personen in Entwicklungsländern, eine Kühlung nicht immer zuverlässig gewährleistet werden kann, ist die vorliegende Erfindung desweiteren auf ein Isoliergefäß gerichtet, welches unabhängig von einer externen Kühlung eine Nukleinsäureisolierung bei niedrigen Temperaturen ermöglicht.

Daher ist die Erfindung weiterhin gerichtet auf ein Isoliergefäß zur Isolierung von Nukleinsäuren mit zumindest einem Oberteil mit einer oberen Öffnung, einer unteren Öffnung und einer Membran, die an der unteren Öffnung angeordnet ist und den gesamten Querschnitt des Oberteils ausfüllt; einem Unterteil mit einem absorbierenden Material; und einem das Oberteil zumindest im Bereich der Membran umgebenden Mantel zur Aufnahme eines Kühlmittels. Der das Kühlmittel enthaltende Mantel ermöglicht die Kühlung der Membran und der auf die Membran aufgegebenen Lösungen wie des Lysates, der Waschpuffer und des Elutionspuffers, auf niedrige Temperaturen, so dass die abschließende Elution zuverlässig im gewünschten Temperaturbereich nahe dem Gefrierpunkt des Elutionspuffers erfolgen kann.

Bei einer Ausführungsform dieses Isoliergefässes weist der Mantel zwei Kompartimente auf, die voneinander durch eine mechanisch zerstörbare Trennwand getrennt sind, wobei jedes der Kompartimente eine Lösung enthält und bei Mischung der beiden Lösungen nach Zerstörung der Trennwand das Kühlmittel entsteht. Die Trennwand kann durch den Experimentator zerstört werden, indem er beispielsweise an vorgesehenen Stellen gegen die Aussenwand des Mantels drückt und dadurch die Trennwand zum Zerreissen bringt. Geeignete Lösungen zur Füllung der Kompartimente sind Fachleuten auf dem Gebiet der chemischen Kühltechnik geläufig. Diese können an die gewünschten Temperaturen und an die bei Verwendung des Isoliergefässes zu erwartenden Aussentemperaturen angepasst werden.

Bei der Gewinnung von Nukleinsäuren aus biologischen Proben, wie beispielsweise den oben angegebenen Proben, ist es häufig nötig, Zellen oder Sekrete zunächst zu lysieren, um an die Nukleinsäuren gelangen zu können. Die so hergestellten Lysate können neben den Nukleinsäuren auch große Mengen an unerwünschten Stoffen enthalten, wie z.B. Proteine oder Fette. Wenn die Menge an solchen Stoffen in einem Lysat zu groß ist, kann es beim Beschicken der Membran zu deren Verstopfung kommen, was die Effizienz der Nukleinsäurenisolierung herabsetzt und die Durchgängigkeit der Membran beim Waschen oder bei der Elution vermindert. Um diesen unerwünschten Effekt zu vermeiden, ist die Erfindung daher auch auf ein Verfahren gerichtet, bei dem unerwünschte Stoffe entfernt werden, bevor sie auf die Membran gelangen.

Dieses erfindungsgemäße Verfahren zur Isolierung von Nukleinsäuren umfasst folgende Schritten:
- Einstellen zumindest einer Nukleinsäureprobe auf Bindebedingungen, die eine Immobilisierung von in der zumindest einen Nukleinsäureprobe enthaltenen Nukleinsäuren an eine Oberfläche erlaubt;
- Beschicken der Oberfläche mit der zumindest einen Nukleinsäureprobe; und
- Immobilisieren der Nukleinsäuren auf der Oberfläche,
**dadurch gekennzeichnet, dass** vor und/oder nach dem Einstellen der Bindebedingungen eine Vorreinigung erfolgt.

Die Vorreinigung kann beispielsweise durch Aussalzen oder durch Filtrieren, Zentrifugation, enzymatische Behandlung, Temperatureinwirkung, Fällung, und/oder Extraktion der Nukleinsäure-Lösung und/oder das Binden von Kontaminanten der Nukleinsäure-Lösung an Oberflächen erfolgen. Die Vorreinigung kann ebenso erfolgen durch ein mechanisches Zerhacken oder Homogenisieren der Nukleinsäure-Lösung, wenn es sich beispielsweise um das Lysat einer biologischen Probe handelt.

Die eingestellten Bindebedingungen können dabei eine Immobilisierung von RNA und/oder von DNA ermöglichen.

Eine Vorreinigung kann besonders dann nötig sein, wenn die Isolierung von biologischen Proben mit starken Verunreinigunen vorgenommen wird. Die biologische Probe kann jedes denkbare Material sein, dass entweder unmittelbar verwendet wird oder wiederum gewonnen werden kann aus einer anderen biologischen Probe. Beispielsweise können dies sein Blut, Sputum, Urin, Faeces, Sperma, Zellen, Serum, Leukozytenfraktionen, Crusta Phlogistica, Abstriche, Gewebeproben, Pflanzen, Bakterien, Pilze, Viren und Hefen sowie alle anderen, oben erwähnten Arten von biologischen Proben.

Besonders vorteilhaft kann das erfindungsgemäße Verfahren natürlich eingesetzt werden, wenn die biologische Probe einen hohen Anteil unerwünschter Substanzen enthält.

Nach der Immobilisierung der Nukleinsäuren aus der vorgereinigten Nukleinsäureprobe können sich die üblichen Isolierschritte anschliessen, also:
- Ablösen der immobilisierten Nukleinsäuren von der Oberfläche; und
- Abnehmen der abgelösten Nukleinsäuren von der Oberfläche.

Ein besonderer Vorteil der erfindungsgemäßen Isolierverfahren liegt darin, dass diese mit chemischen Reaktionen verbunden werden können, denen die Nukleinsäuren direkt an der Oberfläche unterworfen werden. Eine Vielzahl von Analysetechniken für Nukleinsäuren kann somit bei den an der Oberfläche isolierten Nukleinsäuren angewendet werden. Hierbei ist es möglich, die Nukleinsäuren vor der Reaktion wieder von der Oberfläche abzulösen, um ihre freie Zugänglichkeit zu gewährleisten. Alternativ kann jedoch eine geeignete Reaktion auch an Nukleinsäuren vorgenommen werden, die direkt an der Oberfläche angebunden sind.

Demgemäß ist die Erfindung in einem Aspekt gerichtet auf ein Verfahren mit Vorreinigung, wie oben skizziert, welches **dadurch gekennzeichnet** ist, dass nach dem Ablöseschritt vorzugsweise zumindest einmal folgender Schritt durchgeführt wird:
- Durchführen zumindest einer chemischen Reaktion an den Nukleinsäuren.

Ein besonderer Vorteil dieses Verfahrens liegt darin, dass vor der chemischen Reaktion kein verlustbehaftetes Umfüllen aus dem Isoliergefäß in ein Reaktionsgefäß erfolgen muß, sondern Isolierung und Reaktion im selben Gefäß stattfinden können.

In einem weiteren, von einer Vorreinigung unabhängigen Aspekt ist die Erfindung gerichtet auf ein Verfahren zur Durchführung einer Nukleinsäuren-Amplifikationsreaktion mit den folgenden Schritten:
- Beschicken einer Oberfläche mit zumindest einer Nukleinsäurenprobe;
- Immobilisieren der Nukleinsäuren auf der Oberfläche; und
- Durchführen einer Amplifikations-Reaktion mit den Nukleinsäuren.

Gerade bei den geringen Mengen an Material, mit denen bei Amplifikationsreaktionen üblicherweise gearbeitet wird oder werden muß, ist es überaus vorteilhaft, wenn der gesamte Ansatz an Nukleinsäuren ohne Umfüllverluste in die Reaktion eingesetzt werden kann. Dies ist besonders auch für eine Automation von Vorteil, da alle Vorgänge in einem Gefäß durchgeführt werden können. Zudem wird die anfallende Abfallmenge verringert und das Verfahren schneller und kostengünstiger.

Die Amplifikations-Reaktion kann dabei eine isothermale oder eine nicht-isothermale Reaktion sein.

Die Amplifikations-Reaktion kann dabei beispielsweise eine SDA-Reaktion ("strand displacement amplification"), eine PCR, RT-PCR, LCR oder eine TMA oder eine Rolling Circle Amplification sein.

Auch eine NASBA Amplifikation-Reaktion ist mit diesem erfindungsgemäßen Verfahren möglich.

Vor dem Durchführen der Amplifikations-Reaktion können die Nukleinsäuren mit einem Reaktionspuffer von der Oberfläche abgelöst werden, wobei sich das Eluat auf oder in der Membran befindet. Alternativ kann die Amplifikations-Reaktion in einem Reaktionspuffer durchgeführt werden, der nicht zu einer Ablösung der Nukleinsäuren von der Oberfläche führt.

Dieses Verfahren weist vorzugsweise die weiteren Schritte auf:
- ggfs. Ablösen der Reaktionsprodukte von der Oberfläche (sofern diese während der Reaktion noch gebunden waren); und
- Abnehmen der abgelösten Reaktionsprodukte von der Oberfläche.

Ein weiterer Aspekt beinhaltet ein Verfahren zur Durchführung von chemischen Reaktionen an Nukleinsäuren mit den folgenden Schritten:
- Beschicken einer Oberfläche mit zumindest einer Nukleinsäurenprobe;
- Immobilisieren der Nukleinsäuren an der Oberfläche;
- Ablösen der immobilisierten Nukleinsäuren von der Oberfläche;
- Durchführen zumindest einer chemischen Reaktion an den Nukleinsäuren; und
- Abnehmen der Nukleinsäuren von der Oberfläche ohne vorherige Immobilisierung.

Bei diesem Verfahren werden die Nukleinsäuren nach der chemischen Reaktion nicht mehr an die Membran gebunden (immobilisiert), sondern ohne Bindung abgenommen. Die Einsparung eines solchen zusätzlichen Schrittes kann zwar die Reinheit des abgenommenen Ansatzes verschlechtern, ist jedoch aufgrund der Zeitersparnis bei zeitkritischen Anwendungen und auch aufgrund der Vereinfachung in bestimmten Anwendungsformen zu bevorzugen. Eine breite Auswahl an chemischen Reaktionen steht durch das erfindungsgemäße Verfahren zur Verfügung. Unter "chemischer Reaktion" im Sinne der Erfindung soll hierbei jede Wechselwirkung der Nukleinsäuren mit weiteren Substanzen verstanden werden (außer mit der Oberfläche, da diese "Reaktion" bei allen hier geschilderten Verfahren auftritt), also enzymatische Modifikationen, Hybridisierung mit Sonden, chemische Sequenzierreaktionen, pH-Wert-Veränderungen, beispielsweise zur basischen Depurinierung von RNA und sauren Depurinierung von DNA, sowie Antikörperbindungen und Proteinanlagerungen. Generell ist jede Reaktion, sei sie auf das Ändern von kovalenten Bindungen oder Wasserstoffbrücken-Bindungen ausgerichtet, miterfasst.

Ein Vorteil des erfindungsgemäßen Verfahrens ist in der permanenten, räumlichen Zusammenführung eines Volumenraumes, in dem unterschiedlichste Vorgänge stattfinden können, und einer Membran, an der Nukleinsäuren binden können, zu sehen. Diese Zusammenführung gestattet in einfachster Weise eine Manipulation der Nukleinsäuren mit einer unmittelbar anschließenden Membranbindung. Dies ist besonders für automatisierte Verfahren von großem Vorteil. Nach Bindung an die Membran stehen die Nukleinsäuren für Weiterbehandlungsschritte zur Verfügung, beispielsweise - wie oben dargestellt - für die Isolierung möglichst reiner Nukleinsäuren oder für das Durchführen chemischer Reaktionen mit den Nukleinsäuren. In einem weiteren Aspekt der Erfindung ist es allerdings auch möglich, die noch an die Membran gebundenen Nukleinsäuren unmittelbar einer weiteren Analyse zu unterwerfen, um bestimmte Eigenschaften der Nukleinsäuren zu bestimmen.

Daher ist die Erfindung weiterhin gerichtet auf ein Verfahren zur Nukleinsäureanalyse in einem Isoliergefaß mit folgenden Schritten:
- Bereitstellen eines Isoliergefässes mit einer darin angeordneten Membran;
- Beschicken des Isoliergefässes mit zumindest einer Nukleinsäurenprobe;
- Immobilisieren der Nukleinsäuren auf der Membran,
- Hindurchführen der flüssigen Bestandteile der Probe durch die Membran; und
- Analysieren von zumindest einer Eigenschaft der Nukleinsäuren auf der im Isoliergefäß angeordneten Membran.

Nach dem Hindurchführen der flüssigen Bestandteile kann in einer weiteren Ausführungsform zumindest eine chemische Reaktion, wie oben dargestellt, an den Nukleinsäuren durchgeführt wird. Diese kann z.B. dazu dienen, die sich anschliessende Analyse der Nukleinsäuren erst zu ermöglichen. Beispiele für Reaktionen in diesem Sinne sind das Hybridisieren von Sonden, das radioaktive Markieren der an die Membran gebundenen Nukleinsäuren oder das Binden spezifischer Antikörper. Auch Hilfsreaktionen wie das Färben von Nukleinsäuren, beispielsweise mit interkalierenden Substanzen wie Ethidiumbromid, soll als chemische Reaktion verstanden werden.

Verschiedene Eigenschaften der Nukleinsäuren stehen einer membrangebundenen Analyse offen und sind bereits für konventionelle Membranen ohne ein kombiniertes Reaktionsgefäß beschrieben worden. Einige der analysierbaren Eigenschaften sind die Radioaktivität der Nukleinsäuren oder ihr Bindevermögen für Moleküle, wobei die Moleküle beispielsweise Antikörper, Nukleinsäure-bindende oder an Nukleinsäuren bindende Farbstoffmoleküle oder Proteine sein können.

Dieses Verfahren stellt eine maßgebliche Vereinfachung der Analyse von Nukleinsäuren dar, da eine Manipulation der freien Membran nicht mehr nötig ist. Diese ist vielmehr im Isoliergefäß angeordnet.

Auch eine irreversible Bindung der Nukleinsäuren an die Membran, beispielsweise für anschließende Analyseschritte, wird vom Rahmen der vorliegenden Erfindung umfasst. Die dauerhafte oder irreversible Bindung ermöglicht die Manipulation der Membran und der daran gebundenen Nukleinsäuren in einem Maße, denen reversibel gebundene Nukleinsäuren nicht offenstehen.

In einem weiteren Aspekt ist die Erfindung auf die quantitative Präzipitation von Nukleinsäuren gerichtet.

Bei vorbekannten, auf Anionenautauscherchromatographie basierenden Methoden zur Plasmid-DNA Aufreinigung von 100 µg und mehr (im folgenden als "Großmaßstab" bezeichnet) wird, im letzten Schritt, die Plasmid DNA in einem Hochsalzpuffer von der Säule eluiert. Um die Plasmid DNA zum einen vom Salz zu trennen und sie zum anderen zu konzentrieren, wird sie mit Hilfe von Alkoholen (z.B. Isopropanol) präzipitiert und in einem geeigneten Gefäß abzentrifugiert. Das erhaltene Zentrifugationspellet wird mit 70%igem Ethanol gewaschen, um restliche Spuren von Salz zu entfernen, und dann nochmals einer Zentrifugation unterzogen. Das Pellet dieser zweiten Zentrifugation wird typischerweise in einer geringen Menge Niedrigsalzpuffer gelöst und die Plasmid-DNA in dieser Form weiterverwendet.

In Stand der Technik sind zudem Verfahren vorgeschlagen worden, die DNA durch Zugabe von chaotropen Salzen in den Hochsalzpuffer in eine Form zu überführen, die an Silikamembranen bindet. Nach einem entsprechenden Waschschritt kann die DNA durch einen Niedrigsalzpuffer wieder von der Membran gelöst werden.

In einer Veröffentlichung (Ruppert, A. et al. , Analytical Biochemistry (1995), 230: 130-134) wird eine ähnliche Anwendung beschrieben, bei der im Kleinmaßstab (Isolierung von weniger als 100 µg Plasmid-DNA) eine Isopropanol gefällte DNA an PVDF-Membranen einer Porengröße von kleiner als 0,2 µm bindet, daraufhin mit Ethanol gewaschen und anschließend mit TE (Tris-EDTA) eluiert wird. Es existiert jedoch keine Beschreibung einer derartigen Methode im Großmaßstab.

Die beschriebene Fällung von DNA mit anschließender Zentrifugation ist extrem zeitaufwendig (etwa 1 Stunde), zudem ist der Einsatz von Zentrifugen notwendig. Neben dem hohen Zeitaufwand für diese Prozedur, ist der beschriebene letzte Schritt der Plasmidpräparation besonders fehleranfällig. Ein teilweiser oder kompletter Verlust des DNA-Pellets tritt auch hin und wieder auf. Eine entscheidende Rolle scheint hierbei auch die Art (Material) des verwendeten Zentrifugationsgefäßes zu spielen.

Die ebenfalls beschriebene Anwendung von chaotropen Salzen und anschließender Bindung der Nukleinsäuren an Silikamembranen ist ebenfalls zeitaufwendig, zudem besteht durch das Einbringen chaotroper Salze in die Präparation die Gefahr einer Kontamination der letztlich isolierten DNA.

Die beschriebene Filtration alkoholischer Präzipitate im Kleinmaßstab besitzt den Nachteil, dass sie nicht linear auf den Großmaßstab übertragbar ist. Im Stand der Technik eingesetzte Membranen erlauben nur die Isolierung kleiner Mengen an Nukleinsäuren, da sich die Membranen rasch mit Nukleinsäuren sättigen und nichts mehr aufnehmen. Beim Entfernen des Präzpitatpuffers und beim Waschen geht daher häufig ein großer Teil der Nukleinsäuren wieder verloren. Um diesen Verlust zu vermeiden, ist die Erfindung auch gerichtet auf ein Verfahren zur Fällung von Nukleinsäuren mit den folgenden Schritten
- Bereitstellen eines Isoliergefässes mit zumindest einer darin angeordneten Membran;
- Beschicken des Isoliergefässes mit einer Nukleinsäurenprobe;
- Präzipitation der in der Probe enthaltenen Nukleinsäuren mit Alkohol, so dass sich die Nukleinsäuren an die zumindest eine Membran binden. Das Verfahren ist **dadurch gekennzeichnet, dass** die Porengröße der zumindest einen Membran größer oder gleich 0,2 Mikrometer beträgt.

Als Alkohole kommen für die Durchführung des erfindungsgemäßen Verfahrens zunächst alle Hydroxylderivate von aliphatischen oder acyclischen gesättigten oder ungesättigten Kohlenwasserstoffen in Betracht.

Unter den vorgenannten Hydroxylverbindungen werden die C1-C5-Alkanole - wie Methanol, Ethanol, n-Propanol, n-Butanol, tert.-Butanol, n-Pentanol oder Mischungen derer bevorzugt. Besonders bevorzugt wird Isopropanol zur Durchführung des erfindungsgemäßen Verfahrens verwendet.

Dabei kann der Alkohol vor oder nach dem Beschicken des Isoliergefässes mit der Nukleinsäure enthaltenden Lösung mit dieser Lösung gemischt werden. Das Volumenverhältnis von Nukleinsäure enthaltender Lösung zu Alkohol, insbesondere Isopropanol, beträgt vorzugsweise 2:1 bis 1:1, besonders bevorzugt 1,67:1 bis 1:1 und beispielsweise 1,43:1.

Die Fläche der Membran ist vorzugsweise so gewählt, dass die gesamten in der Lösung enthaltenden Nukleinsäuren an die Membran binden können.

Die Erfindung ist auch gerichtet auf die Verwendung von Membranen mit einer Porengröße von größer oder gleich 0,2 µm zur Bindung von Alkohol-gefällten Nukleinsäuren, die DNA und/oder RNA sein können.

Als besonders vorteilhaft wird die Verwendung eines 0,45 µm Celluloseacetat- bzw. Cellulosenitratfilters bzw. die Verwendung mehrer Lagen von 0,65 µm Celluloseacetat- oder Cellulosenitratfilter angesehen. Die Prozedur ist sowohl als Vakuumfiltration, als auch als Druckfiltration anwendbar.

Das erfindungsgemäße Verfahren ermöglicht eine zeitsparende Überführung von Nukleinsäuren aus einem Hochsalzpuffersystem in ein Niedrigsalzpuffersystem, die ohne großen apparativen Aufwand möglich ist. Sie ist als Ersatz für die klassische alkoholische Fällung von DNA aus einem Hochsalzpuffer geeignet, die typischerweise mit Hilfe von Zentrifugationsschritten durchgeführt wird. Durch den hohen Wirkungsgrad der Methode (geringer Ausbeuteverlust) ist sie insbesondere für den präparativen Großmaßstab geeignet. Weiterhin werden durch das erfindungsgemäße Verfahren keine Fremdsubstanzen in die bereits aufgereinigte Nukleinsäuren eingeführt. Zudem ist die Fehleranfälligkeit gegenüber der klassischen Methode geringer (Verlust des Zentrifugationssediments während des Waschschritts ist hier nicht möglich).

Vorzugsweise erfolgt bei den verschiedenen, oben erläuterten Verfahren das Beschicken von oben. Grundsätzlich stehen verschiedenste Methoden zur Verfügung, die verschiedenen Lösungen, also nukleinsäurehaltiger Immobilisierungspuffer, Waschpuffer, Eluat, etc, durch die Membranen hindurchzuführen. Es können dies sein Gravitation, Zentrifugation, Vakuum, Überdruck (auf der Beschickungsseite), und Kapillarkräfte.

Zwischen dem Immobilisierungs- und dem Ablöseschritt kann ein Waschen der immobilisierten Nukleinsäuren mit zumindest einem Waschpuffer erfolgen. Das Waschen umfasst für jeden Waschpuffer vorzugsweise folgende Schritte:
- Aufbringen einer vorbestimmten Menge an Waschpuffer auf die Oberfläche, und
- Hindurchführen des Waschpuffers durch die Oberfläche.

Das Beschicken und Immobilisieren der Nukleinsäuren kann wiederum folgende Schritte umfassen:
- Mischen der Nukleinsäurenprobe mit einem Immobilisierungspuffer,
- Beschicken der Nukleinsäurenprobe mit dem Immobilisierungspuffer auf die Oberfläche, und
- Hindurchführen der füssigen Bestandteile durch die Oberfläche in im wesentlichen der Richtung der Beschickung.

Die Verfahren weisen den großen Vorteil auf, leicht automatisierbar zu sein, so dass zumindest einer der Schritte durch einen Automaten vollautomatisch durchgeführt werden kann. Ebenso ist es möglich, dass alle Schritte der Verfahren durch einen Automaten in gesteuerter Abfolge durchgeführt werden.

Speziell in diesen Fällen, aber auch bei manueller Bearbeitung ist es möglich, dass eine Mehrzahl von Nukleinsäuren gleichzeitig der Isolierung unterworfen werden. Beispielsweise können Multi-Isoliergefässe in der Form marktüblicher "Multi-Well"-Gefässe mit 8, 12, 24, 48, 96 oder mehr einzelnen Isoliervertiefungen verwendet werden.

Das Abnehmen der Nukleinsäuren kann in zwei grundsätzlich verschiedene Richtungen erfolgen. Zum einen ist es möglich, die abgenommenen (eluierte) Nukleinsäuren durch die Membran durchzuschleusen (hindurchzuführen) und nach der Seite der Membran abzunehmen, die gegenüber der Seite liegt, auf welche die Nukleinsäure-haltige Lösung bzw. das Lysat zugegeben wurde. In diesem Fall wird die Nukleinsäure in Richtung der Zugabe durch die Membran hindurch abgenommen. Die andere Möglichkeit besteht darin, die Nukleinsäuren von der Membran bzw. der Oberfläche auf der Seite der Zugabe abzunehmen. Die Abnahme erfolgt dann in Gegenrichtung der Zugabe oder "zur selben Richtung hin", in der zugegeben wurde; also auf der Seite der Zugabe. In diesem Fall müssen die Nukleinsäuren die Membran also nicht passieren. Bei einigen der erfindungsgemäßen Verfahren erfolgt das Abnehmen der Nukleinsäuren stets durch die Membran hindurch in Richtung der Zugabe. Falls ein Verfahren mit einer Oberfläche durchgeführt wird, die einem nicht flüssigkeitsdurchlässigem Untergrund, beispielsweise einer Kunststoffwandung aufliegt, kann die Abnahme natürlich nur in Richtung auf die Richtung der Zugabe hin (also in Gegenrichtung) erfolgen. Bei einigen Verfahren ist jedoch eine Abnahme in beiden Richtungen möglich.

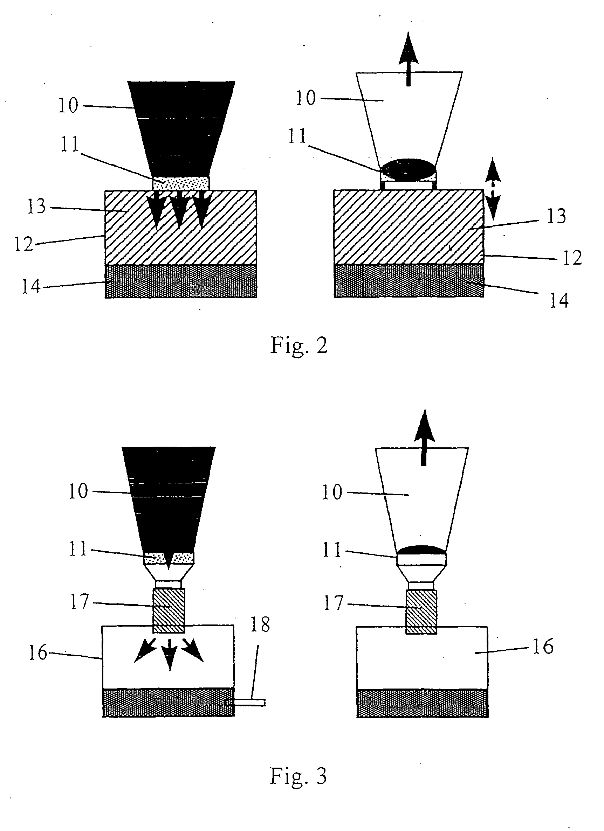

Werden die Nukleinsäuren von der Oberfläche im wesentlichen in Gegenrichtung der Richtung eluiert (abgelöst), in der sie aufgetragen und immobilisiert worden ist, so ist unter "derselben Richtung" dabei im Grunde genommen jede Richtung unter einem Winkel von kleiner oder gleich 180°, bezogen auf die Richtung der Zugabe, zu verstehen, so dass bei der Eluierung die Nukleinsäuren jedenfalls nicht die Oberfläche, beispielsweise eine Membran, durchdringen, sondern in Gegenrichtung der Beschickungsrichtung von der Oberfläche entfernt werden, in der sie auf die Oberfläche aufgebracht worden sind. In bevorzugten Ausführungsformen werden demgegenüber die anderen Puffer, also derjenige Puffer, in dem sich die Nukleinsäuren beim Beschicken befinden, und ggfs. ein Waschpuffer, durch die Oberfläche durchgesaugt oder sonstwie transferiert. Wenn die Isolierung an einer in einem Gefäß befindlichen Membran erfolgt, wobei die Membran den gesamten Querschnitt des Gefässes ausfüllt, ist die bevorzugte Beschickung von oben. Der Abnahmeschritt erfolgt in diesem Fall wiederum nach oben. Fig. 2 zeigt beispielsweise ein trichterförmiges Isoliergefäss, das von oben beschickt wird und bei dem die Abnahme der Nukleinsäuren nach oben erfolgt.

Es versteht sich jedoch, dass auch bei Abnahme in Gegenrichtung der Zugabe andere Anordnungen denkbar sind, so z. B. eine Abnahme der Nukleinsäuren von unten. Es ist beispielsweise vorstellbar, dass ein Nukleinsäuren enthaltender Puffer wie ein Lysatpuffer mittels einer Saugvorrichtung aus einem Reaktionsgefäß unmittelbar in ein Isoliergefaß gesaugt wird, so dass sich die Nukleinsäuren an die Unterseite einer Membran in dem Isoliergefäß binden. In einem solchen Fall könnte die Abnahme der Nukleinsäuren von der Oberfläche dadurch erfolgen, dass ein Elutionspuffer von unten aufgesaugt wird und nach Ablösen der Nukleinsäuren wiederum nach unten in ein Gefäss abgelassen wird. Hierbei erfolgt also die Abnahme der Nukleinsäuren nach unten.

Auch eine seitliche Abnahme der Nukleinsäuren ist möglich, beispielsweise wenn eine flachliegende Säule mit einer darin angeordneten Membran im Durchflußverfahren mit einem Lysat beschickt wird und im Anschluß die liegende Säule auf der Seite der Membran, an der die Nukleinsäuren binden, mit Eluierpuffer gespült wird.

Ein Beispiel für den maximal möglichen Winkel von 180° ist eine Schräge mit einer zur Bindung von Nukleinsäuren geeigneten Oberfläche, über welche die verschiedenen Lösungen bzw. Puffer herabfließen. Wie alle Puffer, kommt auch der Eluierpuffer von einer Seite und fließt zur anderen Seite ab. In diesem Fall bilden Einströmrichtung des Puffers und Abströmrichtung des Puffers mit den darin aufgenommenen Nukleinsäuren einen Winkel von 180°, die Abnahme erfolgt jedoch immer noch auf derselben Seite der Oberfläche wie die Immobilisierung.

Nach den erfindungsgemäßen Verfahren nimmt man die oben beschriebene, Nukleinsäuren enthaltende Probe in einer Lösung auf, die geeignete Salze und/oder Alkohol(e) enthält, schließt anschliessend ggf. den Ansatz auf und führt die so erhaltene Mischung mittels eines Vakuums, auf dem Wege einer Zentrifugation, mittels Überdruck, durch Kapillarkräfte oder durch andere geeignete Verfahren durch eine poröse Oberfläche hindurch, wobei die Nukleinsäuren an der Oberfläche immobilisiert werden.

Als Salze für das Immobilisieren von Nukleinsäuren an Membranen oder anderen Oberflächen und/oder für die Lyse von Nukleinsäureproben kommen Salze von Metallkationen, wie Alkali- oder Erdalkalimetallen, mit Mineralsäuren in Frage; insbesondere Alkali- oder Erdalkalihalogenide bzw. -sulfate oder -phosphate, worunter die Halogenide des Natriums, Lithiums oder Kaliums oder Magnesiumsulfat besonders bevorzugt werden. Auch andere Metallkationen, beispielsweise Mn, Cu, Cs oder Al, oder das Ammoniumkation können verwendet werden, bevorzugt als Salze von Mineralsäuren.

Ferner sind zur Durchführung des erfindungsgemäßen Verfahrens Salze von ein- oder mehrbasigen oder auch polyfunktionellen organischen Säuren mit Alkali- oder Erdalkalimetallen geeignet. Darunter fallen insbesondere Salze des Natriums, des Kaliums oder des Magnesiums mit organischen Dicarbonsäuren - wie z. B. Oxal-, Malonoder Bernsteinsäure - oder mit Hydroxy- bzw. Polyhydroxycarbonsäuren - wie z. B. bevorzugterweise mit Zitronensäure.

Die oben angegebenen Substanzen zur Immobilisierung der Nukleinsäuren auf den Oberflächen und/oder zur Lyse von Nukleinsäureproben können dabei einzeln oder auch in Mischungen verwendet werden, wenn sich diese als für bestimmte Anwendungen geeigneter erweisen.

Als besonders zweckmäßig hat sich dabei der Einsatz von sog. chaotropen Agenzien herausgestellt. Chaotrope Substanzen sind in der Lage, die dreidimensionale Struktur der Wasserstoffbrückenbindungen zu stören. Hierdurch werden auch die intramolekularen Bindungskräfte geschwächt, die bei der Ausbildung der räumlichen Strukturen, wie z. B. Primär-, Sekundär-, Tertiär- oder Quartärstrukturen, bei biologischen Molekülen beteiligt sind. Geeignete chaotrope Agenzien sind dem Fachmann aus dem Stand der Technik hinlänglich bekannt [Römpp, Lexikon der Biotechnologie, Herausgeber H. Dellweg, R.D. Schmid u. W.E. Fromm, Thieme Verlag, Stuttgart 1992].

Als bevorzugte chaotrope Substanzen gelten gemäß der vorliegenden Erfindung beispielsweise Salze aus der Gruppe der Trichloracetate, Thiocyanate, Perchlorate, Iodide oder Guanidinium-Hydrochlorid und Harnstoff.

Die chaotropen Substanzen werden dabei in 0,01 bis 10 molarer wässeriger Lösung, bevorzugt in 0,1 bis 7 molarer wässeriger Lösung und besonders bevorzugt in 0,2 bis 5 molarer wässeriger Lösung eingesetzt. Hierbei können die vorbezeichneten chaotropen Agenzien allein oder in Kombinationen verwendet werden. Insbesondere werden 0,01 bis 10 molare wässerige Lösungen, bevorzugt 0,1 bis 7 molare wässerige Lösungen und besonders bevorzugt in 0,2 bis 5 molare wässerige Lösungen von Natriumperchlorat, Guanidinium-Hydrochlorid, Guanidinium-iso-thiocyanat, Natriumiodid und/oder Kaliumiodid eingesetzt.

Die in den erfindungsgemäßen Verfahren zur Lyse, zur Bindung, zum Waschen und/oder zur Elution eingesetzten Salzlösungen sind vorzugsweise gepuffert. Als Puffersubstanzen kommen alle geeigneten Puffersysteme, wie beispielsweise Karbonsäure-Puffer, insbesondere, Citrat-Puffer, Acetat-Puffer, Succinat-Puffer, Malonat-Puffer sowie Glycin-Puffer, Morpholinopropansulfonsäure (MOPS) oder Tris(hydroxymethyl)aminomethan (Tris) in einer Konzentration von 0,001 - 3 Mol/Liter, bevorzugt 0,005 - 1 Mol/Liter, besonders bevorzugt 0,01 - 0,5 Mol/Liter, insbesondere bevorzugt 0,01 - 0,2 Mol/Liter.

Als Alkohole kommen für die Durchführung des erfindungsgemäßen Verfahrens zunächst alle Hydroxylderivate von aliphatischen oder acyclischen gesättigten oder ungesättigten Kohlenwasserstoffen in Betracht. Dabei ist es zunächst unerheblich, ob diese Verbindungen jeweils eine, zwei, drei oder mehr Hydroxylgruppen - wie mehrwertige C1-C5-Alkanole, beispielsweise Ethylenglykol, Propylenglykol oder Glycerin - enthalten.

Daneben zählen ebenfalls die Zuckerabkömmlinge, die sog. Aldite, wie auch die Phenole - beispielsweise Polyphenole - zu den erfindungsgemäß einsetzbaren Alkoholen.

Unter den vorgenannten Hydroxyverbindungen werden die C1-C5-Alkanole - wie Methanol, Ethanol, n-Propanol, tert.-Butanol und die Pentanole besonders bevorzugt.

Als hydrophil gelten im Sinne der vorliegenden Erfindung solche Stoffe bzw. Membranen, die von ihrem chemischen Charakter her sich leicht mit Wasser mischen bzw. Wasser aufnehmen.

Als hydrophob gelten im Sinne der vorliegenden Erfindung solche Stoffe bzw. Membranen, die von ihrem chemischen Charakter her nicht in Wasser eindringen - bzw. vice versa - und die nicht darin zu bleiben vermögen.

Unter einer Oberfläche wird im Sinne der vorliegenden Erfindung jede mikroporöse Trennschicht verstanden. Im Falle einer Membran wird die Oberfläche durch eine Folie aus einem polymeren Material gebildet. Das Polymer wird bevorzugt aus Monomeren mit polaren Gruppen aufgebaut.

In einer weiteren Ausführungsform des erfindungsgemäßen Verfahrens umfaßt der Begriff Oberfläche im weiteren Sinne auch eine Schicht von Partikeln bzw. auch ein Granulat sowie auch Fasern, wie z. B. Silicagelvliese.

Bei Verwendung hydrophober Membranen gelten als bevorzugt im Sinne der vorliegenden Erfindung Membranen, die aus einem hydrophilen Grundmaterial bestehen und die durch eine entsprechende chemische Nachbehandlung, die an sich aus dem Stand der Technik bekannt ist, hydrophobisiert wurden, wie z. B. hydrophobisierte NylonMembranen, die kommerziell erhältlich sind.

Unter hydrophobisierten Membranen werden erfindungsgemäß allgemein solche Membranen verstanden, die als ursprünglich hydrophile Membran mit den unten erwähnten Hydrophobisierungsmitteln überzogen wurden. Derartige Hydrophobisierungsmittel überziehen hydrophile Substanzen mit einer dünnen Schicht hydrophober Gruppen, wozu beispielsweise längere Alkylketten oder Siloxangruppen gehören. Geeignete Hydrophobisierungsmittel sind aus dem Stand der Technik in großer Zahl bekannt und stellen erfindungsgemäß Paraffine, Wachse, Metallseifen etc., ggf. mit Zusätzen an Aluminium bzw. Zirkoniumsalzen, quartäre organische Verbindungen, Harnstoffderivate, fettstoffmodifizierte Melaminharze, Silicone, zinkorganische Verbindungen, Glutardialdehyde und ähnliche Verbindungen dar.

Daneben gelten als erfindungsgemäß einsetzbare hydrophobe Membranen solche Membranen, die per se hydrophob sind oder die hydrophobisiert sind und deren Grundmaterial polare Gruppen aufweisen kann. Gemäß dieser Kriterien eignen sich beispielsweise - insbesondere hydrophobisierte - Materialien aus der folgenden Gruppe für den erfindungsgemäßen Einsatz:

Nylon, Polysulfone, Polyethersulfone, Cellulosenitrat, Polyproyplen Polycarbonate, Polyacrylate sowie Acrylatcopolymere, Polyurethane, Polyamide, Polyvinylchlorid, Polyfluorcarbonate, Polytetrafluorethylen, Polyvinylidendifluorid, Polyethylentetrafluorethylen-Copolymerisate, Polyethylenchlortrifluorethylen-Coplymerisate oder Polyphenylensulfid sowie Cellulose und Cellulose-Mischester, Celluloseacetat oder Nitrocellulosen wie auch Polybenzimidazole, Polyimide, Polyacrylnitrile, Polyacrylnitril-Copolymere, hydrophobisierte Glasfasermembranen, worunter hydrophobisierte Nylon-Membrane besonders bevorzugt sind.

Bevorzugte hydrophile Oberflächen umfassen per se hydrophile Materialien und auch hydrophobe Materialien, die hydrophilisiert worden sind. Beispielsweise können verwendet werden hydrophiles Nylon, hydrophile Polyethersulfone, hydrophile Polycarbonate, hydrophile Polyester, hydrophile Polytetrafluorethylene auf Polypropylengeweben, hydrophile Polytetrafluorethylene auf Polypropylenvliesen, hydrophilisierte Polyvinylidendifluoride, hydrophilisierte Polytetrafluorethylene, hydrophile Polyamide, Nitrocellulose, hydrophile Polybenzimidazole, hydrophile Polyimide, hydrophile Polyacrylnitrile, hydrophile Polyacrylnitril-Copolymere, hydrophiles Polypropylen, Cellulosenitrat, Cellulosemischester und Celluloseacetat.

Die oben geschilderten Membranen sind in ihrer Anwendung bei Nukleinsäurenbindung zum Teil aus dem Stand der Technik vorbekannt, wenn auch nicht im erfindungsgemäßen Kontext. Eine Reihe von Materialien ist jedoch aus dem Stand der Technik nicht für diese Verwendung bekannt. Die umfangreichen Versuche der Erfinder haben jedoch gezeigt, dass es weitere, zur Nukleinsäurebindung geeignete Membranen gibt.

Die vorliegende Erfindung ist daher auch gerichtet auf die Verwendung von Celluloseacetat, nicht carboxyliertem, hydrophoben Polyvinylidendifluorid, oder massivem, hydrophobem Polytetrafluorethylen als Material zur Anlagerung und Isolierung von Nukleinsäuren.

Unter "massiv" soll hierbei ein Material verstanden werden, dass durchgängig aus der entsprechenden chemischen Verbindung besteht und weder beschichtet noch als Beschichtung auf einem Trägermaterial aufgebracht ist.

Das Material kann in Membranform, als Granulat, in Faserfom oder in anderen geeigneten Formen, verwendet wird. Die Fasern können beispielsweise als Vlies angeordnet sein und das Granulat als gepresste Fritte.

Die Membranen, die in den oben beschriebenen, erfindungsgemäßen Verfahren eingesetzt werden (mit Ausnahme der Isopropanol-Fällung), haben dabei beispielsweise einen Porendurchmesser von 0,001 bis 50 µm, vorzugsweise 0,01 bis 20 µm und besonders bevorzugt 0,05 bis 10 µm. Im Falle der Fällung von Nukleinsäuren mit Isopropanol nach dem oben angegebenen Verfahren muß die Porengröße über 0,2 µm betragen.

Als Waschpuffer kommen ebenfalls die oben beschriebenen Salze oder Alkohole bzw. Phenole oder Polyphenole in Frage. Auch Detergenzien und Naturstoffe im weitesten Sinne, wie etwa Albumin oder Milchpulver, können für die Waschschritte verwendet werden. Die Zugabe chaotroper Substanzen ist ebenfalls möglich. Polymere können ebenso wie lösende Detergentien und ähnliche Stoffe zugegeben werden. Der Waschpuffer und die darin enthaltenen Substanzen sollten jedenfalls allgemein in der Lage sein, unerwünschte Verunreinigungen zu binden, zu solubilisieren, oder mit ihnen zu reagieren, so dass diese Verunreinigungen oder ihre Abbauprodukte zusammen mit dem Waschpuffer entfernt werden können.

Die Temperaturen liegen im Waschschritt in einem Intervall von üblicherweise 10° bis 30 °C, vorzugsweise bei Raumtemperatur, wobei auch höhere oder niedrigere Temperaturen erfolgreich angewandt werden können. So bietet es sich beispielsweise an, bei einer Elution bei niedrigen Temperaturen, z.B. 2°C, auch den Waschpuffer zu kühlen, um die Temperatur des Isoliergefässes und der Oberfläche bzw. Membran auf den gewünschten Temperaturwert vorzukühlen. Eine Anwendung für niedrige Temperaturen ist die cytoplasmatische Lyse, bei der die Zellkerne zunächst unbeschädigt bleiben. Höhere Temperaturen des Waschpuffers bewirken auf der anderen Seite eine bessere Solubilisierung der auszuwaschenden Verunreinigungen.

Zur Elution der gebundenen Nukleinsäuren eignen sich erfindungsgemäß Wasser oder wässerige Salzlösungen als Elutionsmittel. Als Salzlösungen werden Pufferlösungen eingesetzt, die aus dem Stand der Technik bekannt sind, wie beispielsweise Morpholinopropansulfonsäure (MOPS), Tris(hydroxymethyl)aminomethan (TRIS), 2-[4-(2-Hydroxyethyl)-1-piperazino]ethansulfonsäure (HEPES) in einer Konzentration von 0,001 bis 0,5 Mol/Liter, bevorzugt 0,01 bis 0,2 Mol/Liter, besonders bevorzugt 0,01 bis 0,05 molare Lösungen. Daneben werden bevorzugt wässerige Lösungen von Alkali- oder Erdalkalimetallsalzen, insbesondere deren Halogenide, eingesetzt, darunter 0,001 bis 0,5 molare, bevorzugt 0,01 bis 0,2 molare, besonders bevorzugt 0,01 bis 0,05 molare wässerige Lösungen von Natriumchlorid, Lithiumchlorid, Kaliumchlorid oder Magnesiumdichlorid. Daneben können bevorzugt auch Lösungen von Salzen der Alkalimetalle oder Erdalkalimetalle mit Carbon- oder Dicarbonsäuren wie Oxalsäure oder Essigsäure, wie Lösungen von Natriumacetat oder -oxalat in Wasser eingesetzt werden, beispielsweise in dem zuvor genannten Konzentrationsbereich, wie z. B. 0,001 bis 0,5 molar, bevorzugt 0,01 bis 0,2 molar, besonders bevorzugt 0,01 bis 0,05 molar.

Auch die Zugabe von Hilfsstoffen wie Detergenzien oder DMSO ist möglich. Soll mit den eluierlen Nukleinsäuren eine chemische Reaktion durchgeführt werden, sei es unmittelbar an der Membran oder in einem weiteren Reaktionsgefäß, ist es auch möglich, solche Substanzen oder andere Hilfsstoffe dem Elutionspuffer zuzugeben, die in den verwendeten Reaktionsansätzen verwendet werden sollen. So ist beispielsweie die Zugabe von DMSO in niedrigen Konzentrationen bei vielen Reaktionsansätzen üblich.

Nach einer an Nukleinsäuren durchgeführten chemischen Reaktion können diese auch mit dem Reaktionspuffer eluiert werden. Beispielsweise kann die Nukleinsäure nach einer SDA- oder einer NASBA-Reaktion mit dem Reaktionspuffer oder dem Reaktions-Master-Mix eluiert werden.

Ganz besonders wird reines Wasser als Elutionsmittel bevorzugt, beispielsweise demineralisiertes, bidestiliertes, oder ultrareines Millipore-Wasser.

Die Eluierung kann bei Temperaturen von unter 0°C bis 90°C, bespielsweise bei 10° bis 30°C oder auch bei höheren Temperaturen erfolgreich durchgeführt werden. Auch ein Eluieren mit Wasserdampf ist möglich. Die untere Grenze der Elutionstemperatur ist, wie oben ausgeführt, beim Gefrierpunkt des Elutionspuffers zu sehen.

Aufgrund der problemlosen Durchführbarkeit der erfindungsgemäßen Verfahren, die auch "im Feld", d.h. abseits etablierter Laboreinrichtungen, und somit ohne umfangreiche, strombetriebene Ausrüstung, durchführbar sind, ist die Erfindung auch auf die Bereitstellung von Isoliergefäßen gerichtet, mit denen das erfindungsgemäße Verfahren mit einem Minimum an weiteren Hilfsmitteln durchgeführt werden kann. Hierfür kann ein Reaktionsgefäß verwendet werden, das eine Membran enthält. Diese kann in Kontakt mit einem absorbierenden Material, wie einem Schwamm, gebracht werden, um die verschiedenen, verwendeten Puffer durch die Membran durchzusaugen. Der Schwamm funktioniert daher wie die Kombination einer Vakuumpumpe oder einer Zentrifuge in Verbindung mit einem Abfallbehältnis. Zur Gewinnung des Eluats wird der Kontakt des absorbierenden Materials mit der Membran unterbunden, so dass das Eluat nicht verloren gehen kann, sondern abgenommen oder/und weiter untersucht werden kann.

Die Erfindung ist in diesem Aspekt spezifisch gerichtet auf ein Isoliergefäß zur Isolierung von Nukleinsäuren mit zumindest einem zylinderförmigen Oberteil mit einer oberen Öffnung, einer unteren Öffnung und einer Membran, die an der unteren Öffnung angeordnet ist und den gesamten Querschnitt des Oberteils ausfüllt; einem Unterteil mit einem absorbierenden Material; und einem Mechanismus zur Verbindung zwischen Oberteil und Unterteil, wobei bei hergestellter Verbindung die Membran in Kontakt mit dem absorbierenden Material ist und bei nichthergestellter Verbindung die Membran nicht in Kontakt mit dem absorbierenden Material ist.

Vorzugsweise ist das Unterteil ein Zylinder gleichen Durchmessers wie das Oberteil. Auf diese Weise erhält man ein einfaches Röhrchen vom im wesentlichem konstanten Durchmesser, das wie herkömmliche Reaktionsgefässe handhabbar ist. Speziell wenn das Oberteil oder Oberteil plus Unterteil ein Röhrchen ist bzw. bilden, welches in Reaktionsgefäßhalter einstellbar ist, wie sie in Laboratorien Verwendung finden, ist dieser Effekt erzielbar. Der Mechanismus kann ein Anschluß sein, der eine räumliche Trennung von Oberteil und Unterteil erlaubt, beispielsweise ein Bajonettanschluß, ein Steckanschluß oder ein Schraubanschluß. Ein Bajonettanschluß hat dabei den Vorteil, leichter ver- und entriegelbar zu sein, während der Schraubverschluß die bessere, wasserdichtere Verbindung von Ober- und Unterteil erlaubt. Alternativ kann auch eine Sollbruchstelle zwischen Ober- und Unterteil vorgesehen sein, die zumindest die einmalige Trennung der beiden Teile gestattet und die besonders preisgünstig herstellbar ist.

Der Mechanismus kann allerdings auch ein Schieber sein, der zwischen absorbierendem Material und Membran einschiebbar ist. Auch durch diese Ausführung kann eine Trennung von Membrann und absorbierenden Material erreicht werden.

Um die Verarbeitungskapazität zu erhöhen und die erfindungsgemäßen Verfahren noch ökonomischer ausführen zu können, bietet sich desweiteren an, das oben beschriebene, erfindungsgemäße Isoliergefäß so zu modifizieren, dass mehrere Oberteile auf einem Unterteil angeordnet sind. Das Unterteil kann dann gleichzeitig als Halter der Anordnung dienen und zudem so dimensioniert sein, dass eine Vielzahl von Isolierungsvorgängen, jedenfalls mehr als Anschlüsse für Oberteil im Unterteil vorhanden sind, durchgeführt werden kann, bevor die Saugkapazität des absorbierenden Materials im Unterteil erschöpft ist.

Das absorbierende Material im Unterteil kann einen Schwamm oder/und ein Granulat aufweisen. Das Granulat kann beispielsweise aus superabsorbierendem Material bestehen, wie es dem Fachmann auf dem Gebiet der Absorptionstechnik (z.B. bei Hygieneartikeln) geläufig ist.

Die Erfindung ist gleichermaßen auf die Verwendung dieses erfindungsgemäßen Isoliergefässes zur Analyse der Eigenschaften von Nukleinsäuren und zur Isolierung von Nukleinsäuren gerichtet.

Hinsichtlich der einzelnen Schritte werden die erfindungsgemäßen Verfahren allgemein typischerweise wie folgt durchgeführt:

Wenn von biologischen Proben ausgegangen wird, sind diese zunächst in einem geeigneten Puffer zu lysieren. Hierbei können weitere Verfahren zur Lyse zum Einsatz kommen, beispielsweise eine mechanische Einwirkung, wie Homogenisierung oder Ultraschall, enzymatische Einwirkung, Temperaturveränderug oder Additiva. Falls erforderlich oder gewünscht, kann sich an diese Lyse ein Vorreinigungsschritt anschliessen, um Debris aus dem Lysat zu entfernen. Im Anschluß daran werden, falls noch nicht geschehen, die Bedingungen im Lysat eingestellt, unter denen eine Immobilisierung der Nukleinsäuren an die Oberfläche erfolgen kann. Auch nach Einstellung der Bindebedingungen kann sich, kumulativ oder alternativ zur obigen Vorreinigung, ein Vorreinigungsschritt anschliessen.

Dieses vorbehandelte Lysat der zur Gewinnung der Nukleinsäuren dienenden Probe oder die ursprünglich freie(n) Nukleinsäure(n) (falls nicht von einer biologischen Probe ausgegangen wird) wird/werden beispielsweise in eine (Plastik-)Säule pipettiert, in der - beispielsweise auf dem Boden - die Membran fixiert wird. Zweckmäßigerweise kann die Membran auf einer Fritte fixiert werden, die als mechanische Unterstützung dient. Anschließend wird das Lysat durch die Membran geführt, was durch Anlegen eines Vakuums am Ausgang der Säule erreicht werden kann. Auf der anderen Seite kann der Transport durch einen Lysat-seitigen Überdruck erfolgen. Daneben kann - wie schon zuvor erwähnt - der Lysattransport auf dem Wege der Zentrifugation oder durch die Einwirkung von Kapillarkräften bewerkstelligt werden; letzteres kann zum Beispiel mit einem schwammartigen Material geschehen, das unterhalb der Membran mit dem Lysat bzw. Filtrat in Kontakt gebracht wird. Bei einer Zentrifugation kann das unten offene Isoliergefäß in einen Auffangbehälter für die hindurchgeführte Flüssigkeit verwendet werden.

Der in bevorzugten Ausführungsformen eingefügte Waschschritt, kann erfolgen, indem der Waschpuffer durch die Oberfläche bzw. Membran hindurchtransportiert wird oder auf derselben Seite der Oberfläche verbleibt wie die Nukleinsäuren. Wird der Waschpuffer hindurchtansportiert bzw. gesaugt, so kann dies auf unterschiedliche Weisen geschehen, z. B. durch einen auf der anderen Seite der Membran angeordneten Schwamm, eine Saug- oder Überdruckvorrichtung oder durch Zentrifugation oder Gravitation.

Der Vorteil einer Anordnung mit einem absorbierenden, beispielsweise schwammartigen Material besteht in einer einfachen, sicheren und bequemen Möglichkeit der Filtrat-Entsorgung - es muß in diesem Fall nur der Schwamm, der nunmehr mit dem Filtrat mehr oder weniger vollgesogen ist, ausgetauscht werden. Es wird an dieser Stelle deutlich, dass die Säule kontinuierlich oder auch batch-weise betrieben werden kann und dass beide Betriebsarten vollautomatisiert durchgeführt werden können, bis die Membran mit Nukleinsäure gesättigt ist. Im letzten Schritt erfolgt ggfs. die Elution der Nukleinsäure, welche beispielsweise von der Membran abpipettiert bzw. abgehoben oder in sonstiger Weise nach oben entfernt werden kann, wenn keine In-situ Analyse der noch gebundenen Nukleinsäuren vorgenommen werden soll.

Die gewünschten Nukleinsäuren fallen in schwach oder nicht-salzhaltigen Lösungen in sehr kleinen Volumina an, was von großem Vorteil für alle molekularbiologischen Analyseverfahren ist, da hier gewünscht ist, reine Nukleinsäuren in möglichst kleinen Volumina bei gleichzeitig hoher Konzentration einzusetzen. Um möglichst kleine Volumina an Eluat erzielen zu können, werden als Oberflächen insbesondere Membranen bevorzugt, die möglichst dünn sind, so dass sich nur wenig Flüssigkeit in ihnen ansammeln kann.

Ferner bietet die vorliegende Erfindung den Vorteil, dass bei einer vertikalen Anordnung des Gefässes (Membran dann horizontal orientiert) das über der Membran befindliche Volumen als Reaktionsraum genutzt werden kann. So ist es z. B. möglich, nach dem Isolieren und Ablösen der nach dem erfindungsgemäßen Verfahren gewonnenen Nukleinsäuren, diese zunächst nicht abzunehmen, sondern im Isoliergefäß zu belassen und einer molekularbiologischen Applikation - wie Restriktionsverdauung, RT, PCR, RT-PCR, in vitro Transkription, NASBA, rolling circle, LCR (ligase chain reaction), SDA (strand displacement amplification) oder Enzymreaktionen wie RNase und DNase-Verdau zur vollständigen Entfernung der jeweils nicht erwünschten Nukleinsäuren, zu unterwerfen, die aus diesen Reaktionen hervorgehenden Nukleinsäuren gemäß dem erfindungsgemäßen Verfahren erneut an die Membran zu binden oder im Überstand zu belassen, ggfs. wie beschrieben zu waschen und anschließend zu eluieren, zu isolieren, bzw. zu analysieren, beispielsweise mittels Chromatographie, Spektroskopie, Fluorometrie, Elektrophorese oder ähnlichen Meßverfahren.

Die erfindungsgemäß isolierten Nukleinsäuren sind frei von nukleinsäureabbauenden Enzymen und haben eine derartig hohe Reinheit, dass sie unmittelbar in verschiedensten Weisen weiterbehandelt und bearbeitet werden können.

Die erfindungsgemäß hergestellten Nukleinsäuren können für Klonierungen verwendet werden und als Substrate für verschiedenste Enzyme dienen, wie beispielsweise DNA-Polymerasen, RNA-Polymerasen wie z.B. T7-Polymerase oder T3-Polymerase, DNA-Restriktionsenzyme, DNA-Ligase, reverse Transkriptase und andere.

Die durch die erfindungsgemäßen Verfahren bereitgestellten Nukleinsäuren eignen sich in besonders guter Weise zur Amplifikation, insbesondere für die PCR, Strand Displacement Amplifikation, Rolling Circle Verfahren, Ligase Chain Reaction (LCR), SunRise, NASBA und ähnlicher Verfahren.

Die erfindungsgemäßen Verfahren eignen sich weiterhin in besonders guter Weise zur Bereitstellung von Nukleinsäuren für die Verwendung in der Diagnostik, beispielsweise der Lebensmittelanalyse, bei toxikologischen Untersuchungen, in der medizinischen und klinischen Diagnostik, der Erreger-Diagnostik, der Genexpressionsanalyse und in der Umweltanalytik. Die Verfahren eignen sich insbesondere für ein Diagnoseverfahren, welches **dadurch gekennzeichnet** ist, dass die durch das erfindungsgemäße Verfahren gereinigten Nukleinsäuren in einem Folgeschritt amplifiziert werden und anschließend und/oder gleichzeitig die so amplifizierten Nukleinsäuren detektiert werden (z. B. Holland, P.M. et al., 1991. Proc. Natl. Acad. Sci. 88, 7276 - 7280. Livak, K.J. et al., 1995. PCR Methods Applic. 4, 357 - 362; Kievits, T. et al., 1991. J. Virol. Meth. 35, 273 - 286; Uyttendaele, M. et al., 1994. J. Appl. Bacteriol. 77, 694 - 701).

Desweiteren eignen sich die erfindungsgemäßen Verfahren in besonders guter Weise zur Bereitstellung von Nukleinsäuren, welche in einem Folgeschritt einem auf einer Hybridisierungsreaktion basierenden Signalamplifikationsschritt unterzogen werden, der insbesondere **dadurch gekennzeichnet** ist, dass die durch das erfindungsgemäße Verfahren bereitgestellten Nukleinsäuren mit "Verzweigten Nukleinsäuren", insbesondere Branched DNA und/oder Branched RNA und/oder entsprechende Dendrimer Nukleinsäuren, wie in den folgenden Literaturstellen beschrieben (z. B. Bresters, D. et al., 1994. J. Med. Virol. 43 (3), 262 - 286; Collins M.L. et al., 1997. Nucl. Acids Res. 25 (15), 2979 - 2984; ), in Kontakt gebracht werden und das entstehende Signal detektiert wird.

Im folgenden wird ein Beispiel für die Automatisierbarkeit des erfindungsgemäßen Verfahrens erläutert und werden Beispiele für die Durchführung des Verfahrens mit verschiedenen Oberflächen und Nukleinsäuren angegeben. Hierbei wird Bezug genommen auf die beigefügten Zeichnungen, in denen folgendes dargestellt ist.
Fig. 1 zeigt einen zur Durchführung des erfindungsgemäßen Verfahrens geeigneten Automaten in einer schematisierten Darstellung.
Fig. 2 zeigt eine erste Ausführungsform eines Isoliergefässes und Abfallbehälters zur Durchführung des erfindungsgemäßen Verfahrens.
Fig. 3 zeigt eine zweite Ausführungsform eines Isoliergefässes und Abfallbehälters zur Durchführung des erfindungsgemäßen Verfahrens.
Fig. 4 zeigt eine dritte Ausführungsform eines Isoliergefässes und Abfallbehälters zur Durchführung des erfindungsgemäßen Verfahrens.
Fig. 5 zeigt Ausführungsformen von erfindungsgemäßen Isoliergefässen mit Oberteil.
Fig. 6 zeigt das Ethidiumbromid-gefärbte Gel einer elektrophoretischen Auftrennung verschiedener Proben nach dem erfindungsgemäßen Verfahren.
Fig. 7 zeigt ein weiteres Ethidiumbromid-gefärbtes Gel einer elektrophoretischen Auftrennung verschiedener Proben nach dem erfindungsgemäßen Verfahren.
Fig. 8 zeigt ein weiteres Ethidiumbromid-gefärbtes Gel einer elektrophoretischen Auftrennung verschiedener Proben nach dem erfindungsgemäßen Verfahren.
Fig. 9 zeigt noch ein weiteres Ethidiumbromid-gefärbtes Gel einer elektrophoretischen Auftrennung verschiedener Proben nach dem erfindungsgemäßen Verfahren.
Fig. 10 zeigt wieder ein weiteres Ethidiumbromid-gefärbtes Gel einer elektrophoretischen Auftrennung verschiedener Proben nach dem erfindungsgemäßen Verfahren.
Fig. 11 schließlich zeigt ein weiteres Ethidiumbromid-gefärbtes Gel einer elektrophoretischen Auftrennung verschiedener Proben nach dem erfindungsgemäßen Verfahren.

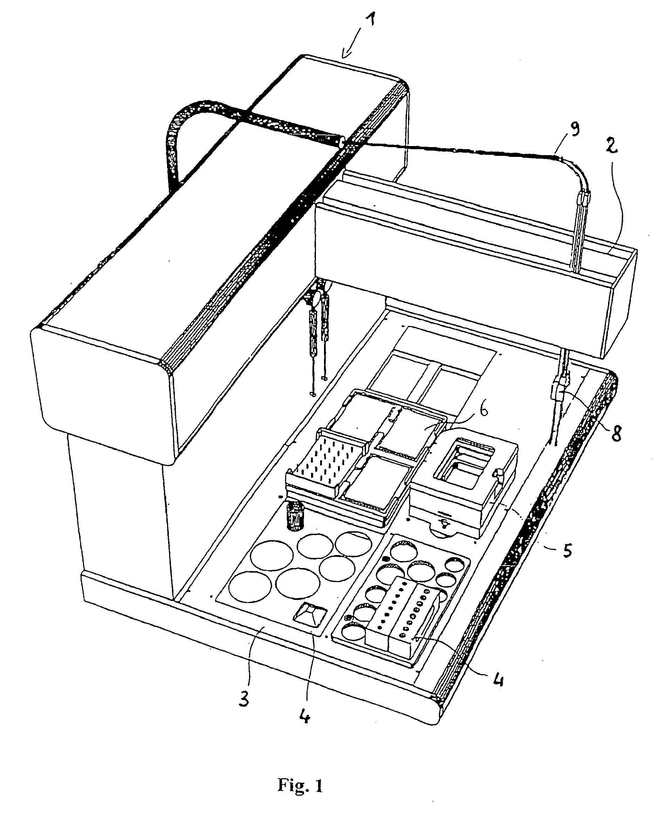

Die erfindungsgemäßen Verfahren werden vorzugsweise teilweise oder vollständig, d.h. in allen Schritten, automatisiert durchgeführt. Ein Beispiel für einen geeigneten Automaten ist in Fig. 1 dargestellt, bei dem ein Hauptteil 1 mit Steuerungselektronik und Antriebsmotoren mit einer Arbeitsplattform 3 und einem fahrbaren Arm 2 ausgestattet ist. Auf der Arbeitsplattform sind verschiedene Elemente positioniert, so Bereiche 4 zur Halterung von verschiedenen Gefässen. Eine Absaugvorrichtung 5 dient zum Absaugen von Flüssigkeiten aus darüber positionierten, nach unten offenen Isoliergefässen, oder sonst mit der Absaugvorrichtung verbundenen Gefässen. Ein Rüttler 6 ist ebenfalls vorgesehen, der beispielsewise zum Lysieren von biologischen Proben verwendet werden kann. Die verwendeten Anordnungen von Isoliergefässen sind beispielsweise Spritzgußteile mit integrierten Isoliergefässen, in die erfindungsgemäße Oberflächen eingelegt sind. Es können typischerweise 8, 12, 24, 48, 96 oder bis zu 1536 Isoliergefässe vorgesehen sein, wie sie z.B. in den Formaten moderner Multi-Well-Platten zur Verfügung gestellt werden. Auch noch höhere Isoliergefäßzahlen in einer Anordnung sind vorstellbar, wenn entsprechende Standards zur Verfügung stehen. Mit Hilfe von Luer-Adaptern ist es jedoch auch möglich, die Böden der Anordnungen separat bereitzustellen und je nach Bedarf mit einem oder mehreren Isoliergefässen zu bestücken. Auch einzeln gearbeitete Isoliergefässe ohne Luer-Adapter werden von der Erfindung miterfasst.

Unter eine Saug- und Dispensiervorrichtung 8 werden die Anordnungen von Isoliergefässen in den Automaten eingesetzt und über diese können Flüssigkeiten aufgenommen und abgegeben werden. Hierbei können mehrere einzelne Saugrohre vorgesehen sein, um mehr als ein Isolier- oder Reaktionsgefäß gleichzeitig bearbeiten zu können. Die Saug- und Dispensiervorrichtung 8 erfüllt also die Funktion einer Pipette. Sog und Druck werden der Saug- und Dispensiervorrichtung 8 über Schläuche 9 vermittelt.

Zur Nukleinsäureisolierung können beispielsweise Reaktionsgefässe mit Zellen in den Rüttler/Halter 6 eingesetzt werden, in die mit der Dispensiervorrichtung 8 Lysepuffer eingefüllt wird. Nach Mischen werden die Zelllysate in Isoliergefässe überführt. Der Lysepuffer wird daraufhin durch die Oberflächen in den Isoliergefässen durchgesaugt. Im Anschluß können die Oberflächen mit einem Waschpuffer gespült werden, um Reste der Zelllysate zu entfernen, wobei auch der Waschpuffer nach unten abgesaugt wird. Schließlich wird Elutionspuffer in die Isoliergefässe dispensiert und nach eventuellem nochmaligen Rütteln werden die abgelösten Nukleinsäuren nach oben entnommen und in Aufbewahrungsgefässe überführt.

Üblicherweise werden Wechselspitzen an der Saug- und Dispensiervorrichtung 8 verwendet, um eine Kontamination der Proben zu verhindern.

Die Fig. 2 bis 4 zeigen verschiedene schematische Beispiele für geeignete Isoliergefässe zur Verwendung in der vorliegenden Erfindung.

In Fig. 2 ist ein trichterförmiges Isoliergefäß 10 mit einer Oberfläche 11, beispielsweise einer Membran, versehen, die auf einen Auffangbehälter 12 aufgesetzt ist, der ein schwammartiges Material 13 enthält, das der Aufnahme von Lysepuffer und Waschpuffer dient. Unter dem schwammartigen Material 13 kann eine Schicht Superabsorbens 14 angeordnet sein, um die Saugleistung zu verbessern. Alternativ kann die Schicht 14 auch ein Material enthalten, welches Wasser chemisch umzusetzen vermag, beispielsweise Acrylat. Das Wasser wird dadurch ebenfalls dem prozeß entzogen. In den Trichter wird Lysat oder eine sonstige Aufbereitung von Nukleinsäuren gegeben. Das schwammartige Material 13 saugt die aufgetragene Flüssigkeit durch die Membran 11 hindurch. Vor der Zugabe des Eluierpuffers wird der Schwamm etwas von der Membran beabstandet, beispielsweise durch eine im Auffangbehälter 12 angeordnete Mechanik (nicht dargestellt). So wird beim letzten Schritt verhindert, dass der Eluatpuffer auch durch die Membran 11 gesaugt wird. Dieser verbleibt vielmehr auf der Oberfläche (Fig. 1b) und kann zusammen mit den Nukleinsäuren nach oben entnommen werden. Mit dieser Anordnung wird die Absaugvorrichtung 5 im Automaten nicht benötigt.

Fig. 3 zeigt ein weiteres Beispiel eines Isoliergefässes, dass über einen an seinem unteren Ende angeordneten Luer-Anschluß mittels eines Luer-Adapters 17 mit einem Auffangbehälter 16 verbunden ist, der in diesem Fall keinen Schwamm enthält, sondern mittels eines Stutzen 18 mit einer Absaugvorrichtung verbunden ist. Lyse- und Waschpuffer können hier also durch Anlegen eines Vakuums durch die Membran 11 durchgesaugt werden. Beim Auftragen des Eluatpuffers bleibt das Vakuum abgeschaltet, so dass sich das Eluat nach oben entnehmen lässt. Durch die Verwendung eines Luer-Anschlusses sind individuelle Isoliergefässe der Anordnung von Isoliergefässen entnehmbar. Es versteht sich jedoch, dass der Vakuumauffangbehälter auch mit fest angebrachten Isoliergefässen, beispielsweise Multi-Well Gefässen von 8, 12, 24, 48, 96 oder mehr Einzelgefässen kombinierbar ist.

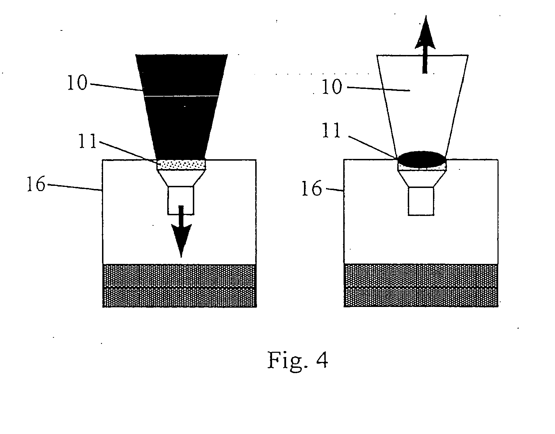

Fig. 4 zeigt schließlich eine Ausführungsform, bei der ein Auffangbehälter vorgesehen ist, in den die Puffer mittels Schwerkraft hineingesaugt oder zentrifugiert werden. Der Eluatpuffer, der in geringem Volumen verwendet wird, hat kein hinreichendes Eigengewicht, um die Membran 11 zu durchdringen und kann wiederum nach oben abgenommen werden.

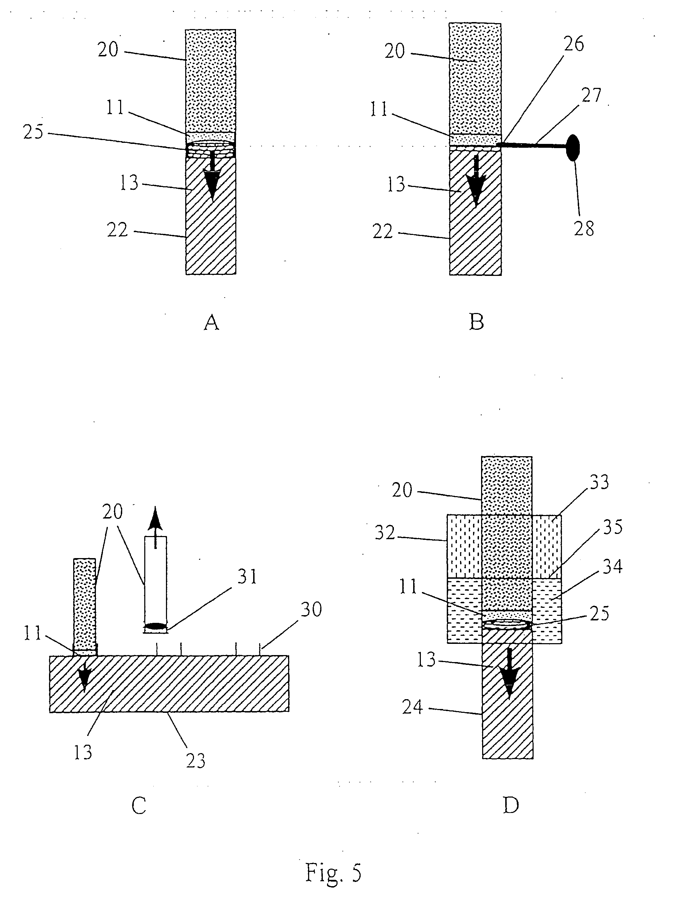

Fig. 5 zeigt Ausführungsformen der erfindungegemäßen Isoliergefässe.

In Fig. 5 A ist ein Isoliergefäß mit einem zylinderförmigen Oberteil 20 dargestellt. Dieses Oberteil ist mittels eines Schraubanschlusses 25 mit einem Unterteil 22 verbunden. Anstelle des Schraubanschlusses können auch andere Verbindungsformen verwendet werden, sofern diese eine flüssigkeitsdichte Verbindung von Ober- und Unterteil zulassen und für ein Aufliegen der Membran 11 sorgen. Die Membran 11 ist in diesem Ausführungsbeispiel direkt an der unteren Öffnung des Oberteils 20 angebracht. Sie kann jedoch auch nach innen versetzt sein oder in einem anderen Winkel als 90° zur Oberteilwandung stehen. Das Unterteil weist ebenfalls eine zylinderförmige Form auf, kann jedoch in anderen Ausführungsformen anders ausgebildet sein. So könnte eine viereckige Form verwendet werden, welche die Standsicherheit des Oberteils 20 auf einem Untergrund verbessert. Eine Ausweitung des Unterteils 22 im Verhältnis zum Oberteil 20 ist ebenfalls möglich, beispielsweise falls in bestimmten Ausführungsformen der erfindungsgemäßen Verfahren ein größerer Hohlraum im Unterteil 22 benötigt wird, um die verwendeten Lösungen vollständig in das absorbierende Material 13 aufnehmen zu können.

Eine zu der in Fig. 5 A gezeigten Auführungsform, alternative Ausführungsform ist in Teil B dargestellt. Hier sind Oberteil 20 und Unterteil 22 fest miteinander verbunden bzw. können auch einstückig ausgeformt sein. Zwischen dem absorbierenden Material 13 und der Membran 11 kann ein Schieber 27 über eine Öffnung 26 in das Isoliergefäß eingeschoben werden, um Membran 11 und absorbierendes Material 13 voneinander trennen zu können. Am Schieber 27 ist in diesem Beispiel ein zusätzlicher Griffbereich 28 angeordnet, der ein einfaches Herausziehen des Schiebers 27 gestattet. Der Schieber kann jedoch auch ohne diesen Griffbereich ausgeführt sein. Wie in der Fig. 5 B gezeigt, expandiert das absorbierende Material 13 etwas, um den vom Schieber eingenommenen Raum überbrücken zu können und dadurch die Membran zu kontaktieren.

Fig. 5 C zeigt eine weitere Ausführungsform des erfindungsgemäßen Isoliergefäßes. Das Unterteil 23 ist hierbei mit mehreren Anschlüssen 30 zur Aufnahme von Oberteilen 20 ausgestattet, was die gleichzeitige Bearbeitung mehrerer Ansätze erlaubt. Die Oberteile 20 werden in diesem Beispiel mittels Schraubanschlüssen 31 mit dem Unterteil 23 verbunden. Obwohl in der Abbildung kleiner dargestellt als die Oberteile 20 der Figs. 5 A und B versteht es sich, dass die Oberteile von gleicher Größe (oder größer bzw. kleiner) sein können wie bzw. als in jenen Ausführungsformen.

Fig. 5 D zeigt schließlich ein erfindungsgemäßes Isoliergefäß mit einem die Membran 11 außen umgebenden Mantel 32 mit einem Kühlmittel. Oberteil 20 und Unterteil 24 sind in diesem Beispiel mittels eines Steckanschlusses miteinander verbunden. Eine andere Anschlußart oder eine einteilige Ausführung sind jedoch ebenso möglich. Der Mantel 32 besteht aus zwei Kompartimenten 33 und 34, die durch eine zerstörbare Trennwand 35 miteinander in Verbindung gebracht werden können. Beide Kompartimente 33, 34 sind mit Substanzen, beispielsweise Lösungen, gefüllt, bei deren Mischung nach Zerstörung der Trennwand 35 die Temperatur des Gemischs sinkt.

Die vorbeschriebene Erfindung wird durch die nachfolgenden Beispiele erläutert. Verschiedenartige, andere Ausgestaltungen der Verfahren werden für den Fachmann aus der vorstehenden Beschreibung und den Beispielen ersichtlich. Es wird jedoch ausdrücklich darauf hingewiesen, dass diese Beispiele und die diesen Beispielen zugeordnete Beschreibung lediglich der Erläuterung dienen und nicht als Einschränkung der Erfindung anzusehen sind.

### Beispiel 1

### Isolierung von Gesamt-RNA aus HeLa-Zellen

In einer Plastiksäule werden kommerziell erhältliche hydrophobe Nylon-Membranen (beispielsweise ein Material der Fa. MSI: Magna SH mit einem Porendurchmesser von 1,2 µm oder ein Material der Fa. Pall: Hydrolon mit einem Porendurchmesser von 1,2 µm), die durch chemische Nachbehandlung hydrophobisiert wurden, einlagig eingebracht. Die Membranen werden auf einer Polypropylenfritte, die als mechanische Unterstützung dient, plaziert. Die Membranen werden in der Plastiksäule durch einen Spannring fixiert.
Die so vorbereitete Säule wird über eine Luerverbindung mit einer Vakuumkammer verbunden - alle Isolierungsschritte werden mit Hilfe eines angelegten Vakuums durchgeführt.

Zur Isolierung werden 5x10⁵ HeLa-Zellen durch Zentrifugation pelletiert und der Überstand entfernt. Die Zellen werden durch Zugabe von 150 µl eines handelsüblichen Guanidinium-Isothiocyanat-Puffers - wie z.B. RLT-Puffer der Fa. QIAGEN - auf an sich aus dem Stand der Technik bekannte Weise lysiert. Dabei wird die Lyse durch mehrmaliges Auf- und Abpipettieren oder durch Vortexen über einen Zeitraum von 5 s unterstützt. Anschließend werden 150 µl 70 %-iges Ethanol zugefügt und durch mehrmaliges Auf- und Abpipettieren oder durch Vortexen über einen Zeitraum von ca. 5 s gemischt.
Das Lysat wird anschließend in die Plastiksäule pipettiert und durch Evakuierung der Vakuumkammer durch die Membran gesaugt. Unter den so eingestellten Bedingungen bleibt die RNA an der Membran gebunden. Anschließend wird mit einem ersten handelsüblichen Guanidinium-Isothiocyanat-haltigen Waschpuffer - beispielsweise mit dem Puffer RW1 der Fa. QIAGEN - und danach mit einem zweiten Tris-haltigen bzw. Tris- und alkoholhaltigen Waschpuffer - z. B. Puffer RPE der Fa. QIAGEN - gewaschen. Dabei werden die Waschpuffer jeweils durch Evakuierung der Vakuumkammer durch die Membran gesaugt. Nach dem letzten Waschschritt wird das Vakuum über einen Zeitraum von ca. 10 Minuten aufrechterhalten, um die Membran zu trocknen. Danach wird die Vakuumkammer belüftet.
Zur Elution werden 70 µl RNase-freies Wasser auf die Membran pipettiert, um die gereinigte RNA von der Membran abzulösen. Nach einer Inkubation von 1 Minute bei einer Temperatur im Bereich von 10 - 30 °C wird das Eluat mittels einer Pipette von oben von der Membran abpipettiert und der Elutionsschritt wird zum Zweck einer vollständigen Elution noch einmal wiederholt.
Die Menge an isolierter Gesamt-RNA wird anschließend durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt.
Die Ergebnisse der zwei Isolierungen mit hydrophoben Nylonmembranen (Nr. 1 und 2) sind in der nachfolgenden Tabelle 1 Vergleichsversuchen, in denen zum einen hydrophiles Nylon (Nylaflo) (Nr. 3) sowie eine Silica-Membran eingesetzt wurde (Nr. 4), gegenübergestellt. Die dort wiedergegebenen Werte liefern einen überzeugenden Beleg für die beeindruckende Isolierungsleistung sowie Trennwirkung der erfindungsgemäß eingesetzten Materialien. Sie zeigen weiterhin, dass Silicagel-Vliese eine deutlich geringere Ausbeute erbringen, was vermutlich auf ihre vliesartige Struktur und die damit verbundene Absorption des größten Teils des Eluatpuffers zurückzuführen ist.

**Tabelle 1: RNA-Ausbeute und Reinheit der nach Beispiel 1 isolierten Gesamt-RNA**

| Nr. | Membrantypus | Ausbeute an Gesamt-RNA (µg) | E₂₆₀/E₂₈₀ |
|---|---|---|---|
| 1 | Magna SH 1,2 µm (hydrophobes Nylon) | 6,0 | 1,97 |
| 2 | Hydrolon 1,2 µm (hydrophobes Nylon) | 7,1 | 2.05 |
| 3 | Nylaflo (hydrophiles Nylon) | < 0,2 | nicht bestimmt |
| 4 | hydrophile Silica-Membran | < 0,2 | nicht bestimmt |

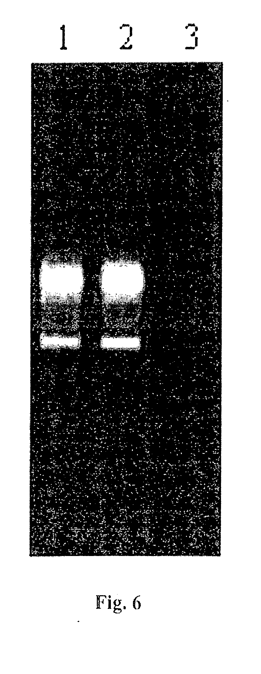

Die isolierte RNA kann ferner auf Agarosegelen, die mit Ethidiumbromid angefärbt sind, analysiert werden. Hierzu werden beispielsweise 1,2 %-ige Formaldehyd-Agarose-Gele angefertigt. Das Ergebnis ist in Fig. 6 wiedergegeben.
In Fig. 6 verkörpert Spur 1 eine Gesamt-RNA, die über eine hydrophobe Nylon-Membran des Ursprungs Magna SH mit einem Porendurchmesser von 1,2 µm isoliert wurde.
Spur 2 stellt eine Gesamt-RNA dar, die über eine hydrophobe Nylon-Membran des Ursprungs Hydrolon mit einem Porendurchmesser von 1,2 µm isoliert wurde.
Spur 3 stellt das Chromatogramm einer Gesamt-RNA dar, die über eine Silica-Membran isoliert wurde.
Dabei wurden jeweils 50 µl der Gesamt-RNA Isolate analysiert.

Fig. 6 liefert einen deutlichen Beleg dafür, dass unter Verwendung der Silica-Membran kein meßbarer Anteil an Gesamt-RNA isoliert werden konnte.

### Beispiel 2

Isolierung freier RNA durch die Bindung der RNA an hydrophobe Membranen mittels verschiedener Salz/AlkoholGemische

Bei diesem Beispiel werden Lysat und Waschlösung durch Anlegen eines Vakuums über die hydrophobe Membran geführt.
In Plastiksäulen, die mit einer Vakuumkammer in Verbindung stehen, werden hydrophobe Nylon-Membranen (beispielsweise Hydrolon 1,2 µm der Fa. Pall) analog Beispiel 1 eingebracht.
100 µl einer Gesamt-RNA enthaltenden wäßrigen Lösung werden jeweils mit 350 µl eines kommerziell erhältlichen Guanidinium-Isothiocyanat enthaltenden Lysepuffers (beispielsweise Puffer RLT der Fa. QIAGEN), 350 µl 1,2 M Natriumacetat-Lösung, 350 µl 2 M Natriumchlorid-Lösung, 350 µl 4 M Lithiumchlorid-Lösung durch Auf- und Abpipettieren gemischt.
Anschließend werden jeweils 250 µl Ethanol zugegeben und ebenfalls durch Auf- und Abpipettieren gemischt. Die RNA-haltigen Lösungen werden danach in die Plastiksäulen einpipettiert und durch Evakuierung der Vakuumkammer durch die Membran gesaugt. Unter den beschriebenen Bedingungen bleibt die RNA an den Membranen gebunden. Die Membranen werden anschließend - wie in Beispiel 1 beschrieben - gewaschen.
Abschließend wird die RNA - ebenfalls wie in Beispiel 1 beschrieben - von oben von der Membran mittels einer Pipette entnommen.
Die Menge an isolierter Gesamt-RNA wurde durch photometrische Messung der Lichtabsorption bei 260 nm ermittelt. Die Qualität der so gewonnenen RNA wird durch die photometrische Bestimmung der Verhältnisse der Lichtabsorption bei 260 nm zu der bei 280 nm bestimmt.

**Tabelle 2: Isolierung freier RNA durch die Bindung der RNA an hydrophobe Membranen mittels verschiedener Salz/Alkohol-Gemische**

| Nr. | Salz/Alkohol-Gemisch | Ausbeute an Gesamt-RNA (µg) | E₂₆₀/E₂₈₀ |
|---|---|---|---|
| 1 | RLT-Puffer QIAGEN/35 %iges Ethanol | 9,5 | 1,92 |
| 2 | 0,6 M Natriumacetat/35 %iges Ethanol | 8,5 | 1,98 |
| 3 | 1 M Natriumchlorid/35 %iges Ethanol | 7,9 | 1,90 |
| 4 | 2 M Lithiumchlorid/35 %iges Ethanol | 4,0 | 2,01 |

### Beispiel 3

### Isolierung von Gesamt-RNA aus HeLa-Zellen

Entsprechend dem Beispiel 1 werden Plastiksäulen mit verschiedenen hydrophoben Membranen hergestellt. Die so vorbereitete Säule wird in ein Collection Tube gesetzt, die folgenden Isolierungsschritte werden mittels einer Zentrifugation durchgeführt.
Zur Isolierung werden 5x10⁵ HeLa-Zellen durch Zentrifugation pelletiert und der Überstand abgenommen. Die Zellen werden durch Zugabe von 150 µl eines handelsüblichen Guanidinium-Isothiocyanat-Puffers - wie z.B. RLT-Puffer der Fa. QIAGEN - auf an sich aus dem Stand der Technik bekannte Weise lysiert. Dabei wird die Lyse durch mehrmaliges Auf- und Abpipettieren oder durch Vortexen über einen Zeitraum von 5 s unterstützt. Anschließend werden 150 µl 70 %-iges Ethanol zugefügt und durch mehrmaliges Auf- und Abpipettieren oder durch Vortexen über einen Zeitraum von 5 s gemischt.
Das Lysat wird anschließend in die Plastiksäule pipettiert und durch Zentrifugation bei 10000 x g für 1 Minute durch die Membran geführt. Anschließend wird mit einem handelsüblichen Guanidinium-Isothiocyanat-haltigen Waschpuffer - beispielsweise mit dem Puffer RW1 der Fa. QIAGEN - und danach mit einem zweiten Tris- und alkoholhaltigen Waschpuffer - beispielsweise Puffer RPE der Fa. QIAGEN - gewaschen. Dabei werden die Waschpuffer jeweils durch Zentrifugation durch die Membran geführt. Der letzte Waschschritt wird bei 20000 x g für 2 Minuten durchgeführt, um die Membran zu trocknen.
Zur Elution werden 70 µl RNase-freies Wasser auf die Membran pipettiert, um die gereinigte RNA von der Membran zu lösen. Nach einer Inkubation von 1 - 2 Minuten bei einer Temperatur im Bereich von 10 - 30 °C wird das Eluat mittels einer Pipette von oben von der Membran abpipettiert. Der Elutionsschritt wird einmal wiederholt, um eine vollständige Elution zu erreichen.
Die Menge an isolierter Gesamt-RNA wird anschließend durch photometrische Messung der Lichtabsorption bei einer Wellenlänge von 260 nm ermittelt. Die Qualität der RNA wird durch die photometrische Bestimmung des Verhältnisses der Lichtabsorption bei 260 nm zu derjenigen bei 280 nm bestimmt.
Die Ergebnisse der Isolierungen mit den verschiedenen hydrophoben Membranen sind in der nachfolgenden Tabelle 3 aufgeführt. Es werden 3 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 3: RNA-Ausbeute der nach Beispiel 3 isolierten Gesamt-RNA durch Bindung an hydrophobe Membranen**

| Hersteller | Membran | Material | RNA(µg) | E₂₆₀/E₂₈₀ |
|---|---|---|---|---|
| Pall | Hydrolon, 1,2 µm | hydrophobes Nylon | 6,53 | 1,7 |
| Pall | Hydrolon, 3 µm | hydrophobes Nylon | 9,79 | 1,72 |
| Pall | Fluoro Trans G | hydrophobes Polyvinylidendifluorid | 6,16 | 1,72 |
| Pall | Fluoro Trans W | hydrophobes Polyvinylidendifluorid | 5,4 | 1,9 |
| Pall | Bio Trace | hydrophobes Polyvinylidendifluorid | 4,3 | 1,97 |
| Pall | Supor-450 PR | hydrophobes Polyethersulfon | 3,96 | 1,76 |
| Pall | V-800 R | hydrophobes Acrylat-Copolymer | 6,26 | 1,72 |
| Pall | Versapor- 1200 R | hydrophobes Acrylat-Copolymer | 6,23 | 1,68 |
| Pall | Versapor - 3000 R | hydrophobes Acrylat-Copolymer | 3,54 | 1,74 |
| Gore-Tex | OH 9335 | hydrophobes Polytetrafluorethylen | 1,59 | 1,72 |
| Gore-Tex | OH 9336 | hydrophobes Polytetrafluorethylen | 2,15 | 1,65 |
| Gore-Tex | OH 9337 | hydrophobes Polytetrafluorethylen | 3,6 | 1,59 |
| Gore-Tex | QH 9316 | hydrophobes Polytetrafluorethylen | 3,61 | 1,69 |
| Gore-Tex | QH 9317 | hydrophobes Polytetrafluorethylen | 2,87 | 1,70 |
| Millipore | Mitex Membrane | hydrophobes Polytetrafluorethylen | 1,98 | 1,62 |
| Millipore | Durapore | hydrophobes Polyvinylidendifluorid | 7,45 | 1,72 |
| MSI | Magna-SH, 1,2 µm | hydrophobes Nylon | 4,92 | 1,69 |
| MSI | Magna-SH, 5 µm | hydrophobes Nylon | 10,2 | 1,71 |
| MSI | Magna-SH, 10 µm | hydrophobes Nylon | 7,36 | 1,76 |
| MSI | Magna-SH, 20 µm | hydrophobes Nylon | 7,04 | 1,65 |
| Sartorius | Typ 118 | hydrophobes Polytetrafluorethylen | 7,6 | 1,61 |
| Mupor | PM12A | hydrophobes Polytetrafluorethylen | 6,7 | 1,77 |
| Mupor | PM3VL | hydrophobes Polytetrafluorethylen | 6,6 | 1,77 |

### Beispiel 3b

### Isolierung von Gesamt-RNA aus HeLa-Zellen durch Bindung an hydrophile Membranen

Entsprechend dem Beispiel 1 werden Plastiksäulen mit verschiedenen hydrophilen Membranen hergestellt. Die so vorbereitete Säule wird in ein Collection Tube gesetzt, die folgenden Isolierungsschritte werden mittels einer Zentrifugation durchgeführt.
Zur Isolierung werden 5x10⁵ HeLa-Zellen eingesetzt. Die folgenden Isolierungsschritte und die Elution der Nukleinsäure werden wie in Beispiel 3 beschrieben durchgeführt.
Die Menge an isolierter Gesamt-RNA wird anschließend durch photometrische Messung der Lichtabsorption bei einer Wellenlänge von 260 nm ermittelt. Die Qualität der RNA wird durch die photometrische Bestimmung des Verhältnisses der Lichtabsorption bei 260 nm zu derjenigen bei 280 nm bestimmt.
Die Ergebnisse der Isolierungen mit den verschiedenen hydrophilen Membranen sind in der nachfolgenden Tabelle 3b aufgeführt. Es werden 2 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 3b: RNA-Ausbeute der nach Beispiel 3b isolierten Gesamt-RNA durch Bindung an hydrophile Membranen**

| Hersteller | Membran | Material | RNA (µg) | E₂₆₀/E₂₈₀ |
|---|---|---|---|---|
| Pall | Loprodyne | hydrophiles Nylon | 3,1 | 1,8 |
| Pall | Loprodyne | hydrophiles Nylon | 3,1 | 1,78 |
| Pall | Biodyne A | hydrophiles Nylon | 3,1 | 1,8 |
| Pall | Biodyne A | hydrophiles Nylon | 3,6 | 1,83 |
| Pall | Biodyne B | hydrophiles Nylon | 2,6 | 1,84 |
| Pall | Biodyne B | hydrophiles Nylon | 4,2 | 1,84 |
| Pall | Biodyne C | hydrophiles Nylon | 6,1 | 1,88 |
| Pall | Biodyne C | hydrophiles Nylon | 5,2 | 1,91 |
| Pall | Biodyne plus | hydrophiles Nylon | 3,3 | 1,87 |
| Pall | I.C.E.-450 | hydrophiles Polyethersulfon | 6,36 | 1,8 |
| Pall | I.C.E.-450sup | hydrophiles Polyethersulfon | 3,07 | 1,71 |
| Pall | Supor - 800 | hydrophiles Polyethersulfon | 4,12 | 1,7 |
| Pall | Supor - 450 | hydrophiles Polyethersulfon | 4,69 | 1,69 |
| Pall | Supor - 100 | hydrophiles Polyethersulfon | 3,25 | 1,71 |
| Pall | Hemasep V | hydrophiles Polyester | 4,16 | 1,74 |
| Pall | Hemasep L | hydrophiles Polyester | 6,67 | 1,65 |
| Pall | Leukosorb | hydrophiles Polyester | 1,5 | 1,84 |
| Pall | Premium Release | hydrophile Polyestermembran | 1,66 | 1,63 |
| Pall | Polypro - 450 | hydrophiles Polypropylen | 5,09 | 1,78 |
| Gore-Tex | OH 9339 | hydrophiles Polytetrafluorethylen | 1,08 | 1,65 |
| Gore-Tex | OH 9338 | hydrophiles Polytetrafluorethylen | 3,97 | 1,67 |
| Gore-Tex | QH 9318 | hydrophiles Polytetrafluorethylen | 3,61 | 1,69 |
| Millipore | Durapore | hydrophilisiertes Polyvinylidendifluorid | 5,6 | 1,69 |
| Millipore | Durapore | hydrophilisiertes Polyvinylidendifluorid | 3,12 | 1,68 |
| Millipore | LCR | hydrophilisiertes Polytetrafluorethylen | 3,14 | 1,66 |
| Sartorius | Typ 250 | hydrophiles Polyamid | 4,3 | 1,66 |
| Sartorius | Typ 113 | hydrophiles Cellulose-Nitrat | 1,8 | 1,86 |
| Sartorius | Typ 113 | hydrophiles Cellulose-Nitrat | 1,9 | 1,74 |
| Infiltec | Polycon, 0,01 | hydrophiles Polycarbonat | 0,17 | 1,64 |
| Infiltec | Polycon, 0,1 | hydrophiles Polycarbonat | 0,73 | 1,68 |
| Infiltec | Polycon, 1 | hydrophiles Polycarbonat | 3,33 | 1,86 |

### Beispiel 4

### Isolierung freier RNA aus wäßriger Lösung

Entsprechend dem Beispiel 1 werden Plastiksäulen mit verschiedenen hydrophoben Membranen hergestellt. 100 µl einer Gesamt-RNA enthaltenden wäßrigen Lösung werden mit 350 µl eines kommerziell erhältlichen Guanidinium-Isothiocyanat enthaltenden Lysepuffers - z.B. RLT-Puffer der Fa. QIAGEN - vermischt. Anschließend werden 250 µl Ethanol zugegeben und durch Auf- und Abpipettieren gemischt. Dieses Gemisch wird dann auf die Säule aufgetragen und durch Zentrifugation (10000 x g; 1 Minute) durch die Membran geführt. Die Membranen werden anschließend zweimal mit einem Puffer - z.B. RPE der Fa. QIAGEN - gewaschen. Der Puffer wird jeweils durch Zentrifugation durch die Membranen geführt. Der letzte Waschschritt wird bei 20000 x g durchgeführt, um die Membranen zu trocknen.
Abschließend wird die RNA wie bereits im Beispiel 1 beschrieben mit RNase-freiem Wasser eluiert und mittels einer Pipette von der Membran von oben abgenommen.
Die Menge an isolierter Gesamt-RNA wird anschließend durch photometrische Messung der Lichtabsorption bei einer Wellenlänge von 260 nm ermittelt und die Qualität der RNA durch die photometrische Bestimmung des Verhältnisses der Lichtabsorption bei 260 nm zu derjenigen bei 280 nm bestimmt.
Die Ergebnisse der Isolierungen mit den verschiedenen hydrophoben Membranen sind in der nachfolgenden Tabelle 4 aufgeführt. Es werden 3 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 4: Isolierung freier RNA aus wäßriger Lösung durch Bindung an hydrophobe Membranen**

| Hersteller | Membran | Material | RNA (µg) | E₂₆₀/E₂₈₀ |
|---|---|---|---|---|
| Pall | Hydrolon, 1,2 µm | hydrophobes Nylon | 5,15 | 1,75 |
| Pall | Hydrolon, 3 µm | hydrophobes Nylon | 0,22 | 1,79 |
| Pall | Fluoro Trans G | hydrophobes Polyvinylidendifluorid | 5,83 | 1,79 |
| Pall | Fluoro Trans W | hydrophobes Polyvinylidendifluorid | 5,4 | 1,84 |
| Pall | Bio Trace | hydrophobes Polyvinylidendifluorid | 4 | 1,79 |
| Pall | Emflon | hydrophobes Polytetrafluorethylen | 0,2 | 1,7 |
| Pall | Supor-450 PR | hydrophobes Polyethersulfon | 5,97 | 1,71 |
| Pall | Supor-200 PR | hydrophobes Polyethersulfon | 2,83 | 1,66 |
| Pall | V-800 R | hydrophobes Acrylat-Copolymer | 2,74 | 1,77 |
| Gore-Tex | OH 9335 | hydrophobes Polytetrafluorethylen | 4,35 | 1,63 |
| Gore-Tex | OH 9336 | hydrophobes Polytetrafluorethylen | 7,43 | 1,71 |
| Gore-Tex | OH 9337 | hydrophobes Polytetrafluorethylen | 5,96 | 1,62 |
| Gore-Tex | QH 9316 | hydrophobes Polytetrafluorethylen | 5,92 | 1,67 |
| Gore-Tex | QH 9317 | hydrophobes Polytetrafluorethylen | 8,7 | 1,66 |
| Millipore | Fluoropore | hydrophobes Polytetrafluorethylen | 8,46 | 1,70 |
| Millipore | Durapore, 0,65 µm | hydrophobes Polyvinylidendifluorid | 4,23 | 1,8 |
| MSI | Magna-SH, 1,2 µm | hydrophobes Nylon | 1,82 | 1,76 |
| MSI | Magna SH, 5 µm | hydrophobes Nylon | 0,6 | 1,78 |
| Sartorius | Typ 118 | hydrophobes Polytetrafluorethylen | 0,9 | 1,82 |
| Sartorius | Typ 118 | hydrophobes Polytetrafluorethylen | 5,4 | 1,74 |
| Mupor | PM12A | hydrophobes Polytetrafluorethylen | 1,1 | 1,98 |

### Beispiel 4b

### Isolierung freier RNA aus wäßriger Lösung durch Bindung an hydrophile Membranen

Entsprechend dem Beispiel 1 werden Plastiksäulen mit verschiedenen hydrophilen Membranen hergestellt.
100 µl einer Gesamt-RNA enthaltenden wäßrigen Lösung werden mit 350 µl eines kommerziell erhältlichen Guanidinium-Isothiocyanat enthaltenden Lysepuffers - z.B. RLT-Puffer der Fa. QIAGEN - vermischt. Anschließend werden 250 µl Ethanol zugegeben und durch Auf- und Abpipettieren gemischt. Dieses Gemisch wird dann auf die Säule aufgetragen und entsprechend Beispiel 4 durch die Membran geführt, gewaschen und getrocknet.
Abschließend wird die RNA wie bereits im Beispiel 1 beschrieben mit RNase-freiem Wasser eluiert und mittels einer Pipette von der Membran abgenommen.
Die Menge an isolierter Gesamt-RNA wird anschließend durch photometrische Messung der Lichtabsorption bei einer Wellenlänge von 260 nm ermittelt und die Qualität der RNA durch die photometrische Bestimmung des Verhältnisses der Lichtabsorption bei 260 nm zu derjenigen bei 280 nm bestimmt.
Die Ergebnisse der Isolierungen mit den verschiedenen hydrophilen Membranen sind in der nachfolgenden Tabelle 4b aufgeführt. Es werden 2 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 4b: Isolierung freier RNA aus wäßriger Lösung durch Bindung an hydrophile Membranen**

| Hersteller | Membran | Material | RNA (µg) | E₂₆₀/E₂₈₀ |
|---|---|---|---|---|
| Pall | Loprodyne | hydrophiles Nylon | 2 | 1,8 |
| Pall | Loprodyne | hydrophiles Nylon | 1,4 | 1,87 |
| Pall | Biodyne A | hydrophiles Nylon | 4,5 | 1,93 |
| Pall | Biodyne A | hydrophiles Nylon | 3,1 | 1,9 |
| Pall | Biodyne B | hydrophiles Nylon | 1,7 | 1,94 |
| Pall | Biodyne B | hydrophiles Nylon | 1,2 | 1,94 |
| Pall | Biodyne C | hydrophiles Nylon | 3,7 | 1,93 |
| Pall | Biodyne C | hydrophiles Nylon | 3,1 | 1,93 |
| Pall | Biodyne plus | hydrophiles Nylon | 1,1 | 1,87 |
| Pall | I.C.E.-450 | hydrophiles Polyethersulfon | 1,92 | 1,82 |
| Pall | I.C.E.-450sup | hydrophiles Polyethersulfon | 0,87 | 1,67 |
| Pall | Supor - 800 | hydrophiles Polyethersulfon | 3,93 | 1,74 |
| Pall | Supor - 450 | hydrophiles Polyethersulfon | 1,78 | 1,74 |
| Pall | Supor - 100 | hydrophiles Polyethersulfon | 1,04 | 1,68 |
| Pall | Hemasep V | hydrophiles Polyester | 4 | 1,79 |
| Pall | Hemasep L | hydrophiles Polyester | 0,47 | 2,1 |
| Pall | Polypro - 450 | hydrophiles Polypropylen | 5,09 | 1,78 |
| Gore-Tex | OH 9339 | hydrophiles Polytetrafluorethylen | 0,43 | 1,48 |
| Gore-Tex | OH 9338 | hydrophiles Polytetrafluorethylen | 3,63 | 1,64 |
| Gore-Tex | QH 9318 | hydrophiles Polytetrafluorethylen | 5,92 | 1,67 |
| Millipore | Durapore | hydrophilisiertes Polyvinylidendifluorid | 1,18 | 1,79 |
| Millipore | LCR | hydrophilisiertes Polytetrafluorethylen | 2,84 | 1,72 |
| Sartorius | Typ 250 | hydrophiles Polyamid | 2,7 | 1,7 |
| Sartorius | Typ 111 | hydrophiles Cellulose-Acetat | 1,6 | 1,85 |
| Sartorius | Typ 111 | hydrophiles Cellulose-Acetat | 2,2 | 2,1 |
| Sartorius | Typ 111 | hydrophiles Cellulose-Acetat | 0,3 | 2,01 |
| Sartorius | Typ 113 | hydrophiles Cellulose-Nitrat | 4 | 1,88 |
| Sartorius | Typ 113 | hydrophiles Cellulose-Nitrat | 3,8 | 1,87 |

### Beispiel 5

### Isolierung von Gesamt-RNA aus HeLa-Zellen in Abhängigkeit von der Porengröße der Membran

Entsprechend dem Beispiel 1 werden Plastiksäulen mit verschiedenen hydrophoben Membranen unterschiedlicher Porengröße hergestellt.
Nach Beispiel 3 wird ein Zell-Lysat aus 5x10⁵ HeLa-Zellen hergestellt und über die Säulen geführt. Anschließend werden die Membranen mit den kommerziell erhältlichen Puffern RW1 und RPE der Fa. QIAGEN mittels Zentrifugation gewaschen. Der letzte Zentrifugationsschritt wird bei 20000 x g für 2 Minuten durchgeführt, um die Membranen zu trocknen. Die Elution wird wie in Beispiel 1 beschrieben durchgeführt.
Die Ergebnisse sind in der nachfolgenden Tabelle 5 aufgeführt. Es werden 3 - 5 Parallelversuche pro Membran durchgeführt und jeweils der Mittelwert errechnet.

**Tabelle 5: RNA-Ausbeute der isolierten Gesamt-RNA durch Bindung an hydrophobe Membranen unterschiedlicher Porengröße**

| Hersteller | Membran | Material | Porengr. (µm) | RNA (µg) | E₂₆₀/E₂₈₀ |
|---|---|---|---|---|---|
| Infiltec | Polycon 0,01 | hydrophiles Polycarbonat | 0,01 | 0,17 | 1,64 |
| Pall | Fluoro Trans G | hydrophobes Polyvinylidendifluorid | 0,2 | 6,16 | 1,72 |
| Pall | Supor-450 PR | hydrophobes Polyethersulfon | 0,45 | 3,96 | 1,76 |
| Millipore | Durapore | hydrophobes Polyvinylidendifluorid | 0,65 | 7,45 | 1,72 |
| MSI | Magna-SH | hydrophobes Nylon | 1,2 | 4,92 | 1,69 |
| MSI | Magna-SH | hydrophobes Nylon | 5 | 10,2 | 1,71 |
| MSI | Magna-SH | hydrophobes Nylon | 10 | 7,36 | 1,76 |
| MSI | Magna-SH | hydrophobes Nylon | 20 | 7,04 | 1,65 |

### Beispiel 6

### Stabilität und Qualität isolierter Gesamt-RNA aus HeLa-Zellen

Entsprechend dem Beispiel 1 werden Plastiksäulen mit einer kommerziell erhältlichen Membran (Fa. Pall, Hydrolon der Porengröße 3 µm) hergestellt.
Nach Beispiel 3 wird ein Zell-Lysat aus 5x10⁵ HeLa-Zellen hergestellt und über die Säulen geführt. Anschließend werden die Membranen mit den kommerziell erhältlichen Puffern RW1 und RPE der Fa. QIAGEN mittels Zentrifugation gewaschen. Der letzte Zentrifugationsschritt wird bei 20000 x g für 2 Minuten durchgeführt, um die Membranen zu trocknen. Die Elution wird wie in Beispiel 1 beschrieben durchgeführt.
Die isolierte Gesamt-RNA wird für 16 Stunden bei 37 °C inkubiert und anschließend auf ein nicht denaturierendes Agarosegel aufgetragen und analysiert. Es zeigte sich, dass die RNA keiner Degradation unterlag. Die mit der oben beschriebenen Methode isolierte RNA zeigt keine Verunreinigungen mit Nukleinsäure abbauenden Enzymen und hat somit eine hohe Qualität.

### Beispiel 7

### Isolierung freier RNA aus wäßriger Lösung durch Bindung an eine hydrophile Membran in einer 96-Well-Platte

Es wird eine 96-Well-Platte mit einer hydrophilen Polyvinylidendifluorid Membran (Durapore, 0,65 µm der Fa. Millipore) verwendet.
5,3 ml einer Gesamt-RNA enthaltenden wäßrigen Lösung werden mit 18,4 ml eines kommerziell erhältlichen Guanidinium-Isothiocyanat enthaltenden Lysepuffers - z.B. RLT-Puffer der Fa. QIAGEN - vermischt. Anschließend werden 13,1 ml Ethanol zugegeben und durch Auf- und Abpipettieren gemischt. Pro Well werden 350 µl dieses Gemisches aufgetragen und durch Anlegen eines Vakuums durch die Membran geführt. Die Membranen werden anschließend zweimal mit einem Puffer - z.B. RPE der Fa. QIAGEN - gewaschen. Der Puffer wird jeweils durch das Anlegen eines Vakuums durch die Membranen geführt. Nach dem letzten Waschschritt wird die Platte einmal auf einem Papiertuch abgetupft und anschließend für 5 Minuten durch Anlegen eines Vakuums getrocknet.
Abschließend wird die RNA wie bereits im Beispiel 1 beschrieben mit RNase-freiem Wasser eluiert und mittels einer Pipette von der Membran abgenommen.

Die Menge an isolierter Gesamt-RNA wird anschließend durch photometrische Messung der Lichtabsorption bei einer Wellenlänge von 260 nm ermittelt und der Mittelwert sowie die Standardabweichung für die gesamte Platte berechnet. Der Mittelwert beträgt 8,4 µg mit einer Standardabweichung von 0,7 µg.

### Beispiel 8

### Isolierung von Gesamt-RNA mittels Kapillarkräfte

Es wird eine 96-Well-Platte mit einer hydrophilen Polyvinylidendifluorid Membran (Durapore, 0,65 µm der Fa. Millipore) verwendet.
33 µl einer Gesamt-RNA enthaltenden wäßrigen Lösung werden mit 110 µl eines kommerziell erhältlichen Guanidinium-Isothiocyanat enthaltenden Lysepuffers - z.B. RLT-Puffer der Fa. QIAGEN - vermischt. Anschließend werden 78 µl Ethanol zugegeben und durch Auf- und Abpipettieren gemischt. Pro Well werden 45 µl dieses Gemisches aufgetragen. Ein saugstarker Haushaltsschwamm wird mit Wasser befeuchtet und die 96-Well-Platte mit der Membranunterseite auf den Schwamm gesetzt. Das RNA-Gemisch wird mittels Kapillarkräfte durch die Membran geführt. Die Membranen werden anschließend zweimal mit einem Puffer - z.B. RPE der Fa. QIAGEN - gewaschen. Der Puffer wird ebenfalls durch Aufsetzen der Platte auf den Schwamm durch die Membran geführt. Nach dem letzten Waschschritt wird die Platte für 5 Minuten an der Luft getrocknet.
Abschließend wird die RNA wie bereits im Beispiel 1 beschrieben mit RNase-freiem Wasser eluiert und mittels einer Pipette von der Membran abgenommen.
Die Menge an isolierter Gesamt-RNA wird anschließend durch photometrische Messung der Lichtabsorption bei einer Wellenlänge von 260 nm ermittelt und der Mittelwert sowie die Standardabweichung für die gesamte Platte berechnet. Der Mittelwert beträgt 5,9 µg mit einer Standardabweichung von 0,7 µg.

### Beispiel 9

Isolierung von genomischer DNA aus wäßriger Lösung mittels eines Guanidinium-Hydrochlorid-haltigen Puffers

Entsprechend Beispiel 1 werden Plastiksäulen mit hydrophoben Membranen (beispielsweise Magna-SH, 5 µm der Fa. MSI) hergestellt. Die Durchführung der Aufreinigung wird mit handelsüblichen Puffern der Fa. QIAGEN durchgeführt.
200 µl einer wäßrigen Lösung genomischer DNA aus Lebergewebe werden in PBS-Puffer hergestellt. Zu dieser Lösung werden 200 µl eines Guanidinium-Hydrochlorid-haltigen Puffers - z.B. AL der Fa. QIAGEN - gegeben und vermischt. Anschließend werden 210 µl Ethanol zugegeben und durch Vortexen vermischt. Das Gemisch wird entsprechend Beispiel 3 auf die Säule gegeben und durch Zentrifugation durch die Membran geführt. Die Membran wird anschließend mit einem alkoholhaltigen Puffer - z.B. AW der Fa. QIAGEN - gewaschen und getrocknet. Die Elution wird wie in Beispiel 1 beschrieben durchgeführt. Es werden drei Parallelversuche durchgeführt und der Mittelwert errechnet.
Die Menge an isolierter DNA wird anschließend durch photometrische Messung der Lichtabsorption bei einer Wellenlänge von 260 nm ermittelt und beträgt ca. 30 % der Ausgangsmenge. Das Verhältnis der Absorption bei 260 nm zu derjenigen bei 280 nm beträgt 1,82.

### Beispiel 10

Isolierung von genomischer DNA aus wäßriger Lösung durch Bindung an hydrophobe Membranen mittels eines Guanidinium- Isothiocyanat enthaltenden Puffers.

Entsprechend dem Beispiel 1 werden Plastiksäulen mit verschiedenen Membranen hergestellt.
100 µl einer Gesamt-DNA enthaltenden wäßrigen Lösung werden mit 350 µl eines Guanidinium-Isothiocyanat enthaltenden Lysepuffers (4 M GITC, 0,1 M MgSO₄, 25 mM Na-Citrate, pH 4) vermischt. Anschließend werden 250 µl Ethanol zugegeben und durch Auf- und Abpipettieren gemischt. Dieses Gemisch wird dann auf die Säule aufgetragen und durch Zentrifugation (10000 x g; 1 Minute) durch die Membran geführt. Die Membranen werden anschließend zweimal mit einem Puffer - z.B. RPE der Fa. QIAGEN - gewaschen. Der Puffer wird jeweils durch Zentrifugation durch die Membranen geführt. Der letzte Waschschritt wird bei 20000 x g durchgeführt, um die Membranen zu trocknen. Die Elution wird wie in Beispiel 1 durchgeführt. Es werden 3 Parallelversuche pro Membran durchgeführt und jeweils der Mittelwert errechnet.
Die Ergebnisse sind in Tabelle 6 aufgeführt.

**Tabelle 6: DNA-Ausbeute aus wäßriger Lösung durch Bindung an hydrophobe Membranen**

| Hersteller | Membran | Material | DNA (µg) |
|---|---|---|---|
| Pall | Hydrolon, 1,2 µm | hydrophobes Nylon | 1,3 |
| Pall | Supor-450 PR | hydrophobes Polyethersulfon | 2,2 |
| Millipore | Fluoropore | hydrophobes Polytetrafluorethylen | 1,1 |
| Millipore | Durapore | hydrophobes Polyvinylidendifluorid | 1,2 |

### Beispiel 11

### Isolierung von genomischer DNA aus Gewebe

Entsprechend Beispiel 1 werden Plastiksäulen mit hydrophoben Membranen (beispielsweise Magna-SH, 5 µm der Fa. MSI) hergestellt. Die Durchführung der Aufreinigung wird mit handelsüblichen Puffern der Fa. QIAGEN durchgeführt.
10 mg Nierengewebe (Maus) werden mit 180 µl Puffer ATL versetzt und durch einen mechanischen Homogenisator zermahlen. Anschließend wird Proteinase K (ca. 0,4 mg gelöst in 20 µl Wasser) zum Ansatz gegeben und bei 55 °C für 10 Minuten inkubiert. Nach Zusatz von 200 µl eines Guanidinium-Hydrochlorid-haltigen Puffers - z.B. AL der Firma QIAGEN - Mischen und einer 10 minütigen Inkubation bei 70 °C werden 200 µl Ethanol unter den Ansatz gemischt. Dieses Gemisch wird auf die Säule gegeben und durch Zentrifugation über die Membran geführt. Die Membran wird mit alkoholhaltigen Puffern - z.B. AW1 und AW2 der Fa. QIAGEN - gewaschen und anschließend durch Zentrifugation getrocknet. Die Elution wird wie in Beispiel 1 beschrieben durchgeführt. Es werden drei Parallelversuche durchgeführt und der Mittelwert errechnet.
Die Menge an isolierter DNA wird anschließend durch photometrische Messung der Lichtabsorption bei einer Wellenlänge von 260 nm ermittelt und beträgt im Durchschnitt 9,77 µg. Das Verhältnis der Absorption bei 260 nm zu derjenigen bei 280 nm beträgt 1,74.

### Beispiel 12

### Isolierung von genomischer DNA aus Blut

Entsprechend Beispiel 1 werden Plastiksäulen mit hydrophoben Membranen (beispielsweise Magna-SH, 5 µm der Fa. MSI) hergestellt. Die Durchführung der Aufreinigung wird mit handelsüblichen Puffern der Fa. QIAGEN durchgeführt. 200 µl Blut werden mit 200 µl AL und 20 µl QIAGEN Protease versetzt, gründlich durchmischt und 10 Minuten bei 56 °C inkubiert. Nach Zusatz von 200 µl Ethanol wird der Ansatz gemischt, auf die Säule gegeben und durch Zentrifugation über die Membran geführt. Die Membran wird mit alkoholhaltigen Puffern - z.B. AW1 und AW2 der Fa. QIAGEN - gewaschen und anschließend durch Zentrifugation getrocknet. Die Elution wird wie in Beispiel 1 beschrieben durchgeführt.
Die Menge an isolierter DNA wird anschließend durch photometrische Messung der Lichtabsorption bei einer Wellenlänge von 260 nm ermittelt und beträgt 1,03 µg. Das Verhältnis der Absorption bei 260 nm zu derjenigen bei 280 nm beträgt 1,7.

### Beispiel 13

### Isolierung von Gesamt-RNA aus einem RNA-DNA-Gemisch

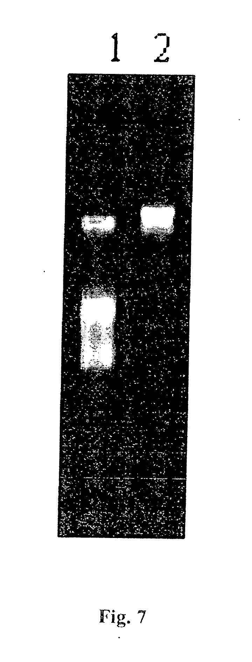

Entsprechend dem Beispiel 1 werden Plastiksäulen mit hydrophoben Membranen (beispielsweise Hydrolon 1,2 µm der Fa. Pall) hergestellt.
275 µl einer Gesamt-RNA und genomische DNA enthaltenden wäßrigen Lösung werden mit 175 µl eines kommerziell erhältlichen Guanidinium-Isothiocyanat enthaltenden Lysepuffers - z.B. RLT-Puffer der Fa. QIAGEN - vermischt. Anschließend werden 250 µl Ethanol zugegeben und durch Auf- und Abpipettieren gemischt. Dieses Gemisch wird dann auf die Säule aufgetragen und entsprechend Beispiel 4 durch die Membran geführt, gewaschen und getrocknet. Der Durchbruch aus dem ersten Zentrifugationsschritt wird auf eine kommerziell erhältliche Mini-Spin-Säule (beispielsweise OIAamp Mini-Spin-Säule der Fa. QIAGEN) aufgetragen und durch Zentrifugation durch die Membran gebracht. Die weiteren Waschschritte werden wie in Beispiel 4 durchgeführt.
Abschließend werden die Nukleinsäuren mit 140 µl RNase-freiem Wasser mittels einer Zentrifugation (10000 x g, 1 Minute) eluiert und in einem nicht denaturierenden Agarosegel analysiert (Fig. 7). Mit der hier beschriebenen Methode läßt sich die Gesamt-RNA von der genomischen DNA weitgehend trennen.

Fig. 7 zeigt ein Ethidiumbromid-gefärbtes Gel einer elektrophoretischen Auftrennung zweier verschiedener Eluate.

Spur 1: Isolierung von total RNA mittels einer hydrophoben Nylonmembran;
Spur 2: Isolierung von genomischer DNA aus dem Durchbruch mittels einer QIAamp Mini-Spin-Säule der Fa. QIAGEN.

### Beispiel 14

### Isolierung von Plasmid-DNA aus wäßriger Lösung durch Bindung an hydrophobe und hydrophile Membranen

Entsprechend dem Beispiel 1 werden Plastiksäulen mit verschiedenen Membranen hergestellt.
100 µl einer Plasmid enthaltenden wäßrigen Lösung (pCMV? der Fa. Clontech) werden mit 350 µl eines Guanidinium-Isothiocyanat enthaltenden Lysepuffers (4 M GITC, 0,1 M MgSO₄, 25 mM Natrium-Acetat, pH 4) vermischt. Anschließend werden 250 µl Isopropanol zugegeben und durch Auf- und Abpipettieren gemischt. Dieses Gemisch wird dann auf die Säule aufgetragen und entsprechend Beispiel 4 durch die Membran geführt, gewaschen und getrocknet.
Abschließend wird die Plasmid-DNA wie bereits im Beispiel 1 beschrieben mit RNase-freiem Wasser eluiert und mittels einer Pipette von der Membran abgenommen.
Die Menge an isolierter Plasmid-DNA wird anschließend durch photometrische Messung der Lichtabsorption bei einer Wellenlänge von 260 nm ermittelt.

Die Ergebnisse der Isolierungen mit den verschiedenen Membranen sind in der nachfolgenden Tabelle aufgeführt. Es werden 3 Parallelversuche pro Membran durchgeführt und jeweils der Mittelwert errechnet.

**Tabelle 7: Plasmid-DNA-Ausbeute aus wäßriger Lösung durch Bindung an Membranen**

| Hersteller | Membran | Material | Plasmid-DNA (µg) |
|---|---|---|---|
| Pall | Hydrolon, 1,2 µm | hydrophobes Nylon | 1,9 |
| Pall | Fluoro Trans G | hydrophobes Polyvinylidendifluorid | 2,2 |
| Pall | I.C.E.-450 | hydrophiles Polyethersulfon | 0,8 |
| Pall | I.C.E.-450sup | hydrophiles Polyethersulfon | 1,5 |
| Pall | Supor-450 PR | hydrophobes Polyethersulfon | 4,7 |
| Pall | Supor-200 PR | hydrophobes Polyethersulfon | 4 |
| Pall | Supor-800 | hydrophiles Polyethersulfon | 0,5 |
| Pall | Supor-450 | hydrophiles Polyethersulfon | 0,9 |
| Pall | Supor-100 | hydrophiles Polyethersulfon | 1 |
| Pall | V-800 R | hydrophobes Acrylat-Copolymer | 1,5 |
| Pall | Versapore - 1200R | hydrophobes Acrylat-Copolymer | 0,2 |
| Pall | Polypro - 450 | hydrophiles Polypropylen | 1,4 |
| Gore-Tex | QH 9318 | hydrophiles Polytetrafluorethylen | 4,9 |
| Gore-Tex | OH 9335 | hydrophobes Polytetrafluorethylen | 4,3 |
| Millipore | Durapore, 0,65 µm | hydrophilisiertes Polyvinylidendifluorid | 1,8 |
| Millipore | Durapore, 0,65 µm | hydrophobes Polyvinylidendifluorid | 1,7 |
| MSI | Magna-SH, 1,2 µm | hydrophobes Nylon | 1,1 |

### Beispiel 15

### Immobilisierung von Gesamt-RNA aus wäßriger Lösung mittels unterschiedlicher chaotroper Agenzien

Entsprechend Beispiel 1 werden Plastiksäulen mit hydrophoben Membranen hergestellt.
Jeweils 100 µl einer Gesamt-RNA enthaltenden wäßrigen Lösung werden mit 350 µl verschiedener Lysepuffer, die Guanidinium-Isothiocyanat (GITC) bzw. Guanidinium-Hydrochlorid (GuHCl) in unterschiedlichen Konzentrationen enthalten, vermischt. Anschließend werden 250 µl Ethanol zugegeben und durch Auf- und Abpipettieren gemischt. Dieses Gemisch wird dann auf die Säule aufgetragen und durch Zentrifugation (10000 x g; 1 Minute) durch die Membran geführt. Die Membranen werden anschließend zweimal mit einem alkoholhaltigen Puffer - z.B. RPE der Fa. QIAGEN - gewaschen. Der Puffer wird jeweils durch Zentrifugation durch die Membran geführt. Der letzte Waschschritt wird bei 20000 x g durchgeführt, um die Membran zu trocknen. Die Elution wird wie in Beispiel 1 durchgeführt. Es werden Doppelbestimmungen durchgeführt und jeweils der Mittelwert angegeben.
Die Ergebnisse sind in Tabelle 8 aufgeführt.

**Tabelle 8: RNA-Ausbeute aus wäßriger Lösung mittels chaotroper Agenzien**

| Membran | chaotropes Agens und Konzentration im Bindeansatz | Ausbeute an Gesamt-RNA (µg) |
|---|---|---|
| Hydrolon, 1,2 µm | GITC, 500 mM | 2,3 |
| Hydrolon, 1,2 µm | GITC, 1 M | 0,8 |
| Hydrolon, 1,2 µm | GITC, 3 M | 0,9 |
| Fluoro Trans G | GITC, 500 mM | 0,4 |
| Fluoro Trans G | GITC, 1 M | 1,25 |
| Fluoro Trans G | GITC, 3 M | 0,6 |
| Hydrolon, 1,2 µm | GuHCl, 500 mM | 2,6 |
| Hydrolon, 1,2 µm | GuHCl, 1 M | 6,7 |
| Hydrolon, 1,2 µm | GuHCl, 3 M | 2,9 |
| Fluoro Trans G | GuHCl, 500 mM | 0,4 |
| Fluoro Trans G | GuHCl, 1 M | 1,25 |
| Fluoro Trans G | GuHCl, 3 M | 0,6 |

### Beispiel 16

### Immobilisierung von Gesamt-RNA aus wäßriger Lösung mittels unterschiedlicher Alkohole

Entsprechend Beispiel 1 werden Plastiksäulen mit hydrophoben Membranen hergestellt.
100 µl einer Gesamt-RNA enthaltenden wäßrigen Lösung werden mit 350 µl eines Guanidinium-Isothiocyanat enthaltenden Lysepuffers (Konzentration 4 M) vermischt. Anschließend werden unterschiedliche Mengen an Ethanol bzw. Isopropanol zugegeben und mit RNase-freiem Wasser auf 700 µl aufgefüllt und gemischt. Dieses Gemisch wird dann auf die Säule aufgetragen und entsprechend Beispiel 4 durch die Membran geführt und gewaschen. Die Elution erfolgte wie in Beispiel 1. Es werden Doppelbestimmungen durchgeführt und jeweils der Mittelwert angegeben.
Die Ergebnisse sind in Tabelle 9 aufgeführt.

**Tabelle 9: RNA-Ausbeute aus wäßriger Lösung mit unterschiedlichen Alkoholen im Bindeansatz**

| Membran | Alkohol und Konzentration im Bindeansatz | Ausbeute an Gesamt-RNA (µg) |
|---|---|---|
| Hydrolon, 1,2 µm | Ethanol, 5 % | 0,7 |
| Hydrolon, 1,2 µm | Ethanol, 30 % | 2,85 |
| Hydrolon, 1,2 µm | Ethanol, 50 % | 4,5 |
| Durapore, 0,65 µm | Ethanol, 5 % | 0,4 |
| Durapore, 0,65 µm | Ethanol, 30 % | 1,25 |
| Durapore, 0,65 µm | Ethanol, 50 % | 0,6 |
| Hydrolon, 1,2 µm | Isopropanol, 5 % | 0,35 |
| Hydrolon, 1,2 µm | Isopropanol, 30 % | 4,35 |
| Hydrolon, 1,2 µm | Isopropanol, 50 % | 3,2 |
| Durapore, 0,65 µm | Isopropanol, 10 % | 1,35 |
| Durapore, 0,65 µm | Isopropanol, 30 % | 4,1 |
| Durapore, 0,65 µm | Isopropanol, 50 % | 3,5 |

### Beispiel 17

### Immobilisierung von Gesamt-RNA aus wäßriger Lösung mit unterschiedlichen pH-Werten

Entsprechend Beispiel 1 werden Plastiksäulen mit hydrophoben Membranen hergestellt.
100 µl einer Gesamt-RNA enthaltenden wäßrigen Lösung werden mit 350 µl eines Guanidinium-Isothiocyanat enthaltenden Lysepuffers (Konzentration 4 M) vermischt. Der Puffer enthält 25 mM Natrium-Citrat und wird auf unterschiedliche pH-Werte mittels HCl bzw. NaOH eingestellt. Anschließend werden 250 µl Ethanol zugegeben und gemischt. Dieses Gemisch wird dann auf die Säule aufgetragen und entsprechend Beispiel 4 durch die Membran geführt und gewaschen. Die Elution erfolgte wie in Beispiel 1. Es werden Doppelbestimmungen durchgeführt und jeweils der Mittelwert angegeben.
Die Ergebnisse sind in Tabelle 10 aufgeführt.

**Tabelle 10: RNA-Ausbeute aus wäßriger Lösung mit unterschiedlichen pH-Werten im Bindeansatz**

| Membran | pH-Wert im Bindeansatz | Ausbeute an Gesamt-RNA (µg) |
|---|---|---|
| Hydrolon, 1,2 µm | pH 3 | 0,15 |
| Hydrolon, 1,2 µm | pH 9 | 1,6 |
| Hydrolon, 1,2 µm | pH 11 | 0,05 |
| Fluoro Trans G | pH 1 | 0,45 |
| Fluoro Trans G | pH 9 | 2,85 |
| Fluoro Trans G | pH 11 | 0,25 |

### Beispiel 18

### Immobilisierung von Gesamt-RNA aus wäßriger Lösung mittels unterschiedlicher Salze

Entsprechend Beispiel 1 werden Plastiksäulen mit einer hydrophoben Membran hergestellt. 100 µl einer Gesamt-RNA enthaltenden wäßrigen Lösung werden mit 350 µl eines salzhaltigen Lysepuffers (NaCl, KCI, MgSO₄) vermischt. Anschließend werden 250 µl H₂O oder Ethanol zugegeben und gemischt. Dieses Gemisch wird dann auf die Säule aufgetragen und entsprechend Beispiel 4 durch die Membran geführt, gewaschen und eluiert. Es werden Doppelbestimmungen durchgeführt und jeweils der Mittelwert angegeben.
Die Ergebnisse sind in Tabelle 11 aufgeführt.

**Tabelle 11: RNA-Ausbeute aus wäßriger Lösung mit verschiedenen Salzen im Bindeansatz**

| Membran | Salz-Konzentration im Bindeansatz | Ausbeute an Gesamt-RNA (µg) |
|---|---|---|
| Hydrolon, 1,2 µm | NaCl, 100 mM; ohne Ethanol | 0,1 |
| Hydrolon, 1,2 µm | NaCl, 1 M; ohne Ethanol | 0,15 |
| Hydrolon, 1,2 µm | NaCl, 5 M; ohne Ethanol | 0,3 |
| Hydrolon, 1,2 µm | KCI, 10 mM; ohne Ethanol | 0,2 |
| Hydrolon, 1,2 µm | KCI, 1 M; ohne Ethanol | 0,1 |
| Hydrolon, 1,2 µm | KCI, 3 M; ohne Ethanol | 0,25 |
| Hydrolon, 1,2 µm | MgSO₄, 100 mM; ohne Ethanol | 0,05 |
| Hydrolon, 1,2 µm | MgSO₄, 750 mM; ohne Ethanol | 0,15 |
| Hydrolon, 1,2 µm | MgSO₄, 2 M; ohne Ethanol | 0,48 |
| Hydrolon, 1,2 µm | NaCl, 500 mM; mit Ethanol | 2,1 |
| Hydrolon, 1,2 µm | NaCl, 1 M; mit Ethanol | 1,55 |
| Hydrolon, 1,2 µm | NaCl, 2,5 M; mit Ethanol | 1,35 |
| Hydrolon, 1,2 µm | KCI, 500 mM; mit Ethanol | 1,6 |
| Hydrolon, 1,2 µm | KCI, 1 M; mit Ethanol | 2,1 |
| Hydrolon, 1,2 µm | KCI, 1,5 M; mit Ethanol | 3,5 |
| Hydrolon, 1,2 µm | MgSO₄, 10 mM; mit Ethanol | 1,9 |
| Hydrolon, 1,2 µm | MgSO₄, 100 mM; mit Ethanol | 4,6 |
| Hydrolon, 1,2 µm | MgSO₄, 500 M; mit Ethanol | 2 |

### Beispiel 19

### Immobilisierung von Gesamt-RNA aus wäßriger Lösung mittels unterschiedlicher Pufferbedingungen

Entsprechend Beispiel 1 werden Plastiksäulen mit hydrophoben Membranen hergestellt.
100 µl einer Gesamt-RNA enthaltenden wäßrigen Lösung werden mit 350 µl eines Guanidinium-Isothiocyanat enthaltenden Lysepuffers (Konzentration 2,5 M) vermischt. Der Lysepuffer wird mit verschiedenen Konzentrationen an Natrium-Citrat, pH 7, bzw. Natrium-Oxalat, pH 7,2 versetzt. Anschließend werden 250 µl Ethanol zugegeben und gemischt. Dieses Gemisch wird dann auf die Säule aufgetragen und entsprechend Beispiel 4 durch die Membran geführt, gewaschen und eluiert.
Die Ergebnisse sind in Tabelle 12 aufgeführt. Es werden Doppelbestimmungen durchgeführt und jeweils der Mittelwert angegeben.

**Tabelle 12: RNA-Ausbeute aus wäßriger Lösung mit verschiedenen Pufferkonzentrationen im Bindeansatz**

| Membran | Na-Citrat/Na-Oxalat im Lysepuffer | Ausbeute an Gesamt-RNA (µg) |
|---|---|---|
| Hydrolon, 1,2 µm | Na-Citrat, 10 mM | 2,2 |
| Hydrolon, 1,2 µm | Na-Citrat, 100 mM | 2,4 |
| Hydrolon, 1,2 µm | Na-Citrat, 500 mM | 3,55 |
| Supor-450 PR | Na-Citrat, 10 mM | 1,1 |
| Supor-450 PR | Na-Citrat, 100 mM | 1,15 |
| Supor-450 PR | Na-Citrat, 500 mM | 0,2 |
| Hydrolon, 1,2 µm | Na-Oxalat, 1 mM | 1,5 |
| Hydrolon, 1,2 µm | Na-Oxalat, 25 mM | 1,05 |
| Hydrolon, 1,2 µm | Na-Oxalat, 50 mM | 0,9 |
| Supor-450 PR | Na-Oxalat, 1 mM | 1,9 |
| Supor-450 PR | Na-Oxalat, 25 mM | 1,3 |
| Supor-450 PR | Na-Oxalat, 50 mM | 1,7 |

### Beispiel 20

### Immobilisierung von Gesamt-DNA aus wäßriger Lösung mittels unterschiedlicher Puffersubstanzen

Entsprechend Beispiel 1 werden Plastiksäulen mit hydrophoben Membranen (beispielsweise Hydrolon 1,2 µm der Fa. Pall) hergestellt.
100 µl einer Gesamt-DNA enthaltenden wäßrigen Lösung werden mit 350 µl eines Guanidinium-Isothiocyanat enthaltenden Lysepuffers (4 M GITC, 0,1 M MgSO₄) vermischt. Der Lysepuffer wird mit verschiedenen Puffersubstanzen (Konzentration 25 mM) versetzt und auf verschiedene pH-Werte eingestellt. Anschließend werden 250 µl Ethanol zugegeben und gemischt. Dieses Gemisch wird dann auf die Säule aufgetragen und entsprechend Beispiel 4 durch die Membran geführt, gewaschen und eluiert.
Die Ergebnisse sind in Tabelle 13 aufgeführt. Es werden Dreifachbestimmungen durchgeführt und jeweils der Mittelwert angegeben.

**Tabelle 13: DNA-Ausbeute aus wäßriger Lösung mit verschiedenen Puffersubstanzen im Bindeansatz**

| Puffersubstanz | pH im Lysepuffer | Ausbeute an DNA (µg) |
|---|---|---|
| Natrium-Citrat | pH 4 | 1,3 |
| Natrium-Citrat | pH 5 | 0,6 |
| Natrium-Citrat | pH 6 | 1,4 |
| Natrium-Citrat | pH 7 | 0,5 |
| Natrium-Acetat | pH 4 | 0,9 |
| Natrium-Acetat | pH 5 | 1 |
| Natrium-Acetat | pH 6 | 0,6 |
| Natrium-Acetat | pH 7 | 0,5 |
| Kalium-Acetat | pH 4 | 0,6 |
| Kalium-Acetat | pH 5 | 0,9 |
| Kalium-Acetat | pH 6 | 1,2 |
| Kalium-Acetat | pH 7 | 1,4 |
| Ammonium-Acetat | pH 4 | 0,7 |
| Ammonium-Acetat | pH 5 | 0,3 |
| Ammonium-Acetat | pH 6 | 5,7 |
| Ammonium-Acetat | pH 7 | 1,5 |
| Glycin | pH 4 | 0,5 |
| Glycin | pH 5 | 1,1 |
| Glycin | pH 6 | 1,6 |
| Glycin | pH 7 | 1,1 |
| Malonat | pH 4 | 1,5 |
| Malonat | pH 5 | 0,3 |
| Malonat | pH 6 | 3,1 |
| Malonat | pH 7 | 1,6 |
| Succinat | pH 4 | 2,8 |
| Succinat | pH 5 | 2,3 |
| Succinat | pH 6 | 2,5 |
| Succinat | pH 7 | 4,7 |

### Beispiel 21

### Immobilisierung von Gesamt-RNA aus wäßriger Lösung durch Phenol

Entsprechend Beispiel 1 werden Plastiksäulen mit hydrophoben Membranen (beispielsweise Hydrolon, 1,2 µm der Fa. Pall) hergestellt.
Wäßrige RNA-Lösung wird mit 700 µl Phenol vermischt und über die Membranen mittels Zentrifugation geführt. Entsprechend Beispiel 4 werden die Membranen gewaschen und die RNA eluiert. Es werden Doppelbestimmungen durchgeführt und jeweils der Mittelwert angegeben.
Die Menge an isolierter DNA wird anschließend durch photometrische Messung der Lichtabsorption bei einer Wellenlänge von 260 nm ermittelt und beträgt im Durchschnitt 10,95 µg. Das Verhältnis der Absorption bei 260 nm zu derjenigen bei 280 nm beträgt 0,975.

### Beispiel 22

### Waschen der immobilisierten Gesamt-RNA unter verschiedenen Salzkonzentrationen

Entsprechend Beispiel 1 werden Plastiksäulen mit hydrophoben Membranen hergestellt.
100 µl einer Gesamt-RNA enthaltenden wäßrigen Lösung werden mit 350 µl eines Guanidinium-Isothiocyanat enthaltenden Lysepuffers (Konzentration 4 M) vermischt. Anschließend werden 250 µl Ethanol zugegeben und gemischt. Dieses Gemisch wird dann auf die Säule aufgetragen und entsprechend Beispiel 4 durch die Membran geführt und gewaschen. Die Membranen werden anschließend zweimal mit einem Puffer, der unterschiedliche Konzentrationen an NaCl und 80 % Ethanol enthält, gewaschen. Der Puffer wird jeweils durch Zentrifugation durch die Membranen geführt. Der letzte Waschschritt wird bei 20000 x g durchgeführt, um die Membranen zu trocknen. Die Elution erfolgt wie in Beispiel 1. Es werden Doppelbestimmungen durchgeführt und jeweils der Mittelwert angegeben.
Die Ergebnisse sind in Tabelle 14 aufgeführt.

**Tabelle 14: RNA-Ausbeute aus wäßriger Lösung mit NaCl im Waschpuffer**

| Membran | NaCl im Waschpuffer | Ausbeute an Gesamt-RNA (µg) |
|---|---|---|
| Hydrolon, 1,2 µm | NaCl, 10 mM | 1,4 |
| Hydrolon, 1,2 µm | NaCl, 50 mM | 3,15 |
| Hydrolon, 1,2 µm | NaCl, 100 mM | 3 |
| Durapore, 0,65 µm | NaCl, 10 mM | 2,7 |
| Durapore, 0,65 µm | NaCl, 50 mM | 2,85 |
| Durapore, 0,65 µm | NaCl, 100 mM | 2,7 |

### Beispiel 23

### Elution der immobilisierten Gesamt-RNA unter verschiedenen Salz- und Pufferbedingungen

Entsprechend Beispiel 1 werden Plastiksäulen mit hydrophoben Membranen hergestellt.
100 µl einer Gesamt-RNA enthaltenden wäßrigen Lösung werden mit 350 µl eines Guanidinium-Isothiocyanat enthaltenden Lysepuffers (Konzentration 4 M) vermischt. Anschließend werden 250 µl Ethanol zugegeben und gemischt. Dieses Gemisch wird dann auf die Säule aufgetragen und entsprechend Beispiel 4 durch die Membran geführt und gewaschen.
Zur Elution werden 70 µl einer NaCl-haltigen Lösung, eines Tris/HCl-Puffers (pH 7) oder einer Natrium-Oxalat Lösung (pH 7,2) auf die Membran pipettiert, um die gereinigte RNA von der Membran zu lösen. Nach einer Inkubation von 1 - 2 Minuten bei einer Temperatur im Bereich von 10 - 30 °C wird das Eluat mittels einer Pipette von oben von der Membran abpipettiert. Der Elutionsschritt wird einmal wiederholt, um eine vollständige Elution zu erreichen. Es werden Doppelbestimmungen durchgeführt und jeweils der Mittelwert angegeben.
Die Ergebnisse sind in Tabelle 15 zusammengefaßt.

**Tabelle 15: RNA-Ausbeute aus wäßriger Lösung mit NaCl, Tris/HCl bzw. Na-Oxalat im Elutionspuffer**

| Membran | NaCl bzw. Tris im Elutionspuffer | Ausbeute an Gesamt-RNA (µg) |
|---|---|---|
| Hydrolon, 1,2 µm | NaCl, 1 mM | 1,35 |
| Hydrolon, 1,2 µm | NaCl, 50 mM | 1,2 |
| Hydrolon, 1,2 µm | NaCl, 250 mM | 0,45 |
| Durapore, 0,65 µm | NaCl, 1 mM | 0,9 |
| Durapore, 0,65 µm | NaCl, 50 mM | 0,35 |
| Durapore, 0,65 µm | NaCl, 500 mM | 0,15 |
| Hydrolon, 1,2 µm | Tris/HCl, 1 mM | 0,35 |
| Hydrolon, 1,2 µm | Tris/HCl, 10 mM | 0,75 |
| Durapore, 0,65 µm | Tris,/HCl 1 mM | 1,5 |
| Durapore, 0,65 µm | Tris/HCl, 50 mM | 1 |
| Durapore, 0,65 µm | Tris/HCl, 250 mM | 0,1 |
| Hydrolon, 1,2 µm | Na-Oxalat, 1 mM | 0,45 |
| Hydrolon, 1,2 µm | Na-Oxalat, 10 mM | 0,65 |
| Hydrolon, 1,2 µm | Na-Oxalat, 50 mM | 0,3 |
| Durapore, 0,65 µm | Na-Oxalat, 1 mM | 2 |
| Durapore, 0,65 µm | Na-Oxalat, 10 mM | 1,55 |
| Durapore, 0,65 µm | Na-Oxalat, 50 mM | 0,15 |

### Beispiel 24

### Elution der immobilisierten RNA bei verschiedenen Temperaturen

Entsprechend Beispiel 1 werden Plastiksäulen mit einer hydrophoben Membran (beispielsweise Hydrolon, 3 µm der Fa. Pall) hergestellt.
Zur Isolierung werden 5x10⁵ HeLa-Zellen eingesetzt. Die folgenden Isolierungsschritte werden wie in Beispiel 3 beschrieben durchgeführt.
Zur Elution werden 70 µl RNase-freies Wasser unterschiedlicher Temperatur auf die Membran pipettiert, um die gereinigte RNA von der Membran zu lösen. Nach einer Inkubation von 1 - 2 Minuten bei der entsprechenden Elutionstemperatur wird das Eluat mittels einer Pipette von oben von der Membran abpipettiert. Der Elutionsschritt wird einmal wiederholt, um eine vollständige Elution zu erreichen.
Es werden Dreifachbestimmungen durchgeführt und jeweils der Mittelwert angegeben.
Die Ergebnisse sind in Tabelle 16 zusammengefaßt.

**Tabelle 16: RNA-Ausbeute bei verschiedenen Elutionstemperaturen**

| Membran | Elutionstemperatur | Ausbeute an Gesamt-RNA (µg) |
|---|---|---|
| Hydrolon, 3 µm | eiskalt | 2,2 |
| Hydrolon, 3 µm | 40 °C | 3,2 |
| Hydrolon, 3 µm | 50 °C | 3,9 |
| Hydrolon, 3 µm | 60 °C | 3,7 |
| Hydrolon, 3 µm | 70 °C | 3,7 |
| Hydrolon, 3 µm | 80 °C | 2,9 |

### Beispiel 25

### Elution der immobilisierten RNA mittels Zentrifugation

Entsprechend dem Beispiel 1 werden Plastiksäulen mit einer hydrophoben Membran (beispielsweise Hydrolon 1,2 µm der Fa. Pall) hergestellt.
100 µl einer Gesamt-RNA enthaltenden wäßrigen Lösung werden mit 350 µl eines kommerziell erhältlichen Guanidinium-Isothiocyanat enthaltenden Lysepuffers - z.B. RLT-Puffer der Fa. QIAGEN - vermischt. Anschließend werden 250 µl Ethanol zugegeben und durch Auf- und Abpipettieren gemischt. Dieses Gemisch wird dann auf die Säule aufgetragen und durch Zentrifugation (10000 x g; 1 Minute) durch die Membran geführt. Die Membranen werden anschließend zweimal mit einem Puffer - z.B. RPE der Fa. QIAGEN - gewaschen. Der Puffer wird jeweils durch Zentrifugation durch die Membranen geführt. Der letzte Waschschritt wird bei 20000 x g durchgeführt, um die Membranen zu trocknen.
Zur Elution werden 70 µl RNase-freies Wasser auf die Membran pipettiert, um die RNA von der Membarn abzulösen. Nach einer Inkubation von 1 Minute bei einer Temperatur im Bereich von 10 - 30 °C wird das Eluat mittels einer Zentrifugation (10000 x g, 1 Minute) durch die Membran geführt. Der Elutionsschritt wird zum Zweck der vollständigen Elution noch einmal wiederholt und die Eluate vereint. Es werden fünf Parallelversuche durchgeführt und der Mittelwert errechnet.
Die Menge an isolierter Gesamt-RNA wird anschließend durch photometrische Messung der Lichtabsorption bei einer Wellenlänge von 260 nm ermittelt und beträgt im Durchschnitt 6,4 µg. Das Verhältnis der Absorption bei 260 nm zu derjenigen bei 280 nm beträgt 1,94.

### Beispiel 26

Einsatz von Gesamt-RNA in einer "Real Time" Quantitativen RT-PCR unter Verwendung des 5'-Nuklease PCR-Assays zur Amplifikation und Detektion von β-Aktin mRNA

Entsprechend dem Beispiel 3 werden Plastiksäulen mit einer kommerziell erhältlichen Membran (Fa. Pall: Hydrolon mit einer Porengröße von 3 µm) hergestellt.
Zur Isolierung von RNA werden 1 x 10⁵ HeLa-Zellen eingesetzt und die Aufreinigung der Gesamt-RNA wird wie in Beispiel 1 beschrieben durchgeführt. Die Elution erfolgt mit 2 x 70 µl H₂O wie in Beispiel 1 beschrieben. Zur vollständigen Entfernung restlicher geringer Mengen an DNA wird die Probe mit einer DNase vor der Analyse behandelt.
Es wird eine "Ein-Gefäß 'Real Time' Quantitative RT-PCR" unter Verwendung des kommerziell erhältlichen Reagenzsystems von Perkin-Elmer (TaqMan™ PCR Reagent Kit) unter Einsatz einer M-MLV Reversen Transkriptase durchgeführt. Durch den Einsatz spezifischer Primer und einer spezifischen TaqMan-Sonde für β-Aktin (TaqMan™ β-Actin Detection Kit der Fa. Perkin Elmer) werden die β-Aktin mRNA-Moleküle in der Gesamt-RNA Probe zunächst in β-Aktin cDNA umgeschrieben und anschließend direkt ohne Unterbrechung der Gesamtreaktion in dem gleichen Reaktionsgefäß amplifiziert und detektiert. Die Reaktionsansätze werden entsprechend den Anweisungen des Herstellers hergestellt. Es werden drei verschiedene Mengen an isolierter Gesamt-RNA verwendet (1, 2, 4 µl Eluat) und eine dreifache Bestimmung durchgeführt. Als Kontrolle werden drei Ansätze ohne RNA mitgeführt.
Die cDNA Synthese findet für 1 Stunde bei 37 °C statt, direkt gefolgt von einer PCR, die 40 Zyklen umfaßt. Die Reaktionen und die Analysen werden auf einem ABI PRISM™ 7700 Sequence Detector der Fa. Perkin Elmer Applied Biosystems durchgeführt. Jedes während eines PCR-Zyklus entstehende Amplikon erzeugt ein lichtemitierendes Molekül, das durch Abspaltung von der TaqMan-Sonde entsteht. Damit ist das insgesamt entstehende Lichtsignal direkt proportional zur entstehenden Amplikonmenge und damit zur ursprünglich in der Gesamt-RNA Probe vorhandenen Transkriptmenge. Das emitierte Licht wird von dem Gerät gemessen und durch ein Computerprogramm ausgewertet. Der PCR-Zyklus, bei dem das Lichtsignal das erste Mal sicher oberhalb des Hintergrundrauschens detektiert wird, wird als "Threshold Cycle" (ct) bezeichnet. Dieser Wert ist ein Maß für die in der Probe vorhandene Menge an der spezifisch amplifizierter RNA.
Für die eingesetzte Menge von 1 µl an mit dem hier beschriebenen Verfahren isolierter Gesamt-RNA ergibt sich ein durchschnittlicher ct-Wert von 17,1, für 2 µl an Gesamt-RNA ein ct-Wert von 16,4 und für 4 µl an Gesamt-RNA ein ct-Wert von 15,3. Hieraus ergibt sich ein linearer Zusammenhang von eingesetzter Gesamt-RNA und dem ct-Wert. Dies zeigt, dass die Gesamt-RNA frei von Substanzen ist, die die Amplifikationsreaktion hemmen könnten. Die Kontrollansätze, die keine RNA enthalten, erzeugen keine Signale.

### Beispiel 27

### Einsatz von Gesamt-RNA in einer RT-PCR zur Amplifikation und Detektion von β-Aktin mRNA

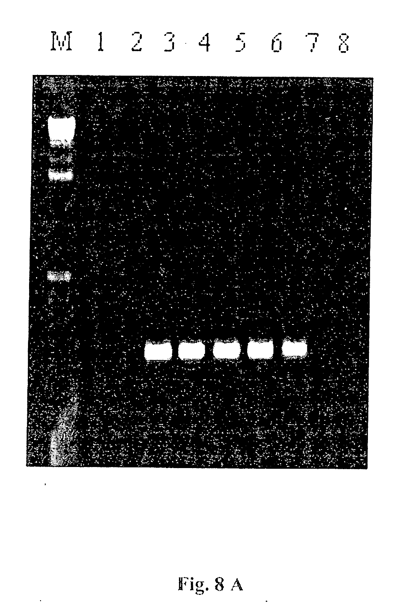

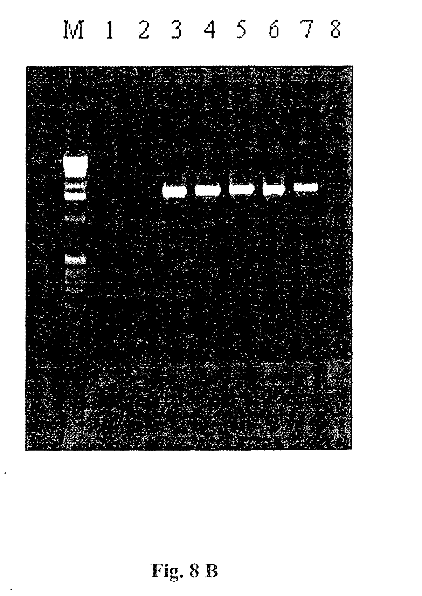

Entsprechend dem Beispiel 1 werden Plastiksäulen mit kommerziell erhältlichen Membranen (Fa. Pall, Hydrolon der Porengröße 1,2 bzw. 3 µm; Fa. Sartorius, Sartolon der Porengröße 0,45 µm) hergestellt.
Zur Isolierung von RNA werden zwei verschiedene Ausgangsmaterialien eingesetzt: 1) Gesamt-RNA aus Leber (Maus) in wäßriger Lösung, Aufreinigung und Elution erfolgt wie in Beispiel 4 beschrieben und 2) 5 x 10⁵ HeLa-Zellen, die Aufreinigung der Gesamt-RNA sowie die Elution wird wie in Beispiel 3 beschrieben durchgeführt.
Es werden jeweils 20 ng der isolierten Gesamt-RNA eingesetzt. Als Kontrolle wird RNA, die mittels des RNeasy-Kits (Fa. QIAGEN) aufgereinigt wurde, und ein Ansatz ohne RNA mitgeführt.
Es wird eine RT-PCR unter Standardbedingungen mit diesen Proben durchgeführt. Für die Amplifikation werden zwei verschiedene Primerpaare für die β-Aktin-mRNA verwendet. Ein 150 Bp großes Fragment dient als Nachweis der Sensitivität, ein 1,7 kBp großes Fragment dient der Integrität der RNA. Aus der RT-Reaktion wird 1 µl entnommen und in der anschließenden PCR eingesetzt. Es werden für das kleine Fragment 25 Zyklen, für das große Fragment 27 Zyklen durchgeführt. Die Anlagerungstemperatur beträgt 55 °C. Die Amplifikate werden anschließend auf einem nicht denaturierenden Gel aufgetragen und analysiert (Fig. 8).
Für die eingesetzte Menge von 20 ng an mit dem hier beschriebenen Verfahren isolierter Gesamt-RNA lassen sich in der RT-PCR die entsprechenden DNA-Fragmente nachweisen. Bei Verwendung von Gesamt-RNA aus Maus Leber läßt sich kein Transkript nachweisen, da die hier verwendeten Bedingungen an humane β-Aktin-mRNA angepaßt sind. Die Kontrollansätze, die keine RNA enthalten, erzeugen keine Signale.

Fig. 8 zeigt Ethidiumbromid-gefärbte Agarose-Gele einer elektrophoretischen Auftrennung der RT-PCR-Reaktionsprodukte.
A: Spur 1 bis 8: RT-PCR eines 150 Bp-Fragmentes;
Spur 1, 2: RNA aus Mausleber in wäßriger Lösung mit der Membran Hydrolon 1,2 µm aufgereinigt;
Spur 3, 4: RNA aus HeLa-Zellen mit der Membran Sartolon aufgereinigt;
Spur 5, 6: RNA aus HeLa-Zellen mit der Membran Hydrolon 3 µm aufgereinigt;
Spur 7: RNA aufgereinigt mittels RNeasy-Mini-Kit;
Spur 8: Kontrolle ohne RNA.
B: Spur 1 bis 8: RT-PCR eines 1,7 kBp-Fragmentes;
Spur 1, 2: RNA aus Mausleber in wäßriger Lösung mit der Membran Hydrolon 1,2 µm aufgereinigt;
Spur 3, 4: RNA aus HeLa-Zellen mit der Membran Sartolon aufgereinigt;
Spur 5, 6: RNA aus HeLa-Zellen mit der Membran Hydrolon 3 µm aufgereinigt;
Spur 7: RNA aufgereinigt mittels RNeasy-Mini-Kit;
Spur 8: Kontrolle ohne RNA.

### Beispiel 28

Einsatz von Gesamt-RNA in einer NASBA-Reaktion (nucleic acid sequence based amplification) zur Amplifikation und Detektion von β-Aktin mRNA

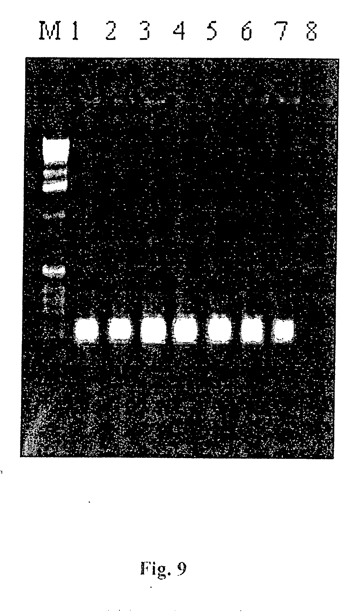

Entsprechend dem Beispiel 1 werden Plastiksäulen mit einer kommerziell erhältlichen Membran (Fa. Pall, Hydrolon der Porengröße 1,2 bzw. 3 µm; Fa. Sartorius, Sartolon der Porengröße 0,45 µm) hergestellt.
Zur Isolierung von RNA werden zwei verschiedene Ausgangsmaterialien eingesetzt: 1) Gesamt-RNA aus Leber (Maus) in wäßriger Lösung, Aufreinigung und Elution erfolgt wie in Beispiel 4 beschrieben und 2) 5 x 10⁵ HeLa-Zellen, die Aufreinigung der Gesamt-RNA sowie die Elution wird wie in Beispiel 3 beschrieben durchgeführt.
Es wird eine NASBA-Reaktion unter Standardbedingungen durchgeführt (Fahy, E. et al., 1991. PCR Methods Amplic. 1, 25 - 33). Für die Amplifikation werden β-Aktin spezifische Primer eingesetzt.
Es werden jeweils 20 ng der isolierten Gesamt-RNA eingesetzt. Als Kontrolle wird RNA, die mittels des RNeasy-Kits (Fa. QIAGEN) aufgereinigt wurde, und ein Ansatz ohne RNA mitgeführt. Es wird zunächst für 5 Minuten bei 65 °C und für 5 Minuten bei 41 °C inkubiert. Im Anschluß an diesen Schritt wird ein Enzymgemisch bestehend aus RNaseH, T7-Polymerase und AMVV-RT zugegeben und 90 Minuten bei 41 °C inkubiert. Die Amplifikate werden anschließend auf ein nicht denaturierendes Gel aufgetragen und analysiert.
Für die eingesetzte Menge von 20 ng an mit den hier beschriebenen Verfahren isolierter Gesamt-RNA läßt sich ein spezifisches Transkript nachweisen (Fig. 9).

Fig. 9 zeigt ein Ethidiumbromid-gefärbtes Agarose-Gel einer elektrophoretischen Auftrennung der NASBA-Reaktionen.
Spur 1 bis 8: NASBA-Reaktionen;
Spur 1, 2: RNA aus Mausleber in wäßriger Lösung mit der Membran Hydrolon 1,2 µm aufgereinigt;
Spur 3, 4: RNA aus HeLa-Zellen mit der Membran Sartolon aufgereinigt;
Spur 5, 6: RNA aus HeLa-Zellen mit der Membran Hydrolon 3 µm aufgereinigt; Spur 7: RNA aufgereinigt mittels RNeasy-Mini-Kit;
Spur 8: Kontrolle ohne RNA.

### Beispiel 29

### NASBA-Reaktion zur Amplifikation und Detektion von β-Aktin mRNA auf hydrophoben Membranen

Entsprechend dem Beispiel 1 werden Plastiksäulen mit kommerziell erhältlichen Membranen (Fa. Pall, Hydrolon der Porengröße 3 µm, Supor-450 PR der Porengröße 0,45 µm; Fa. Millipore, Fluoropore der Porengröße 3 µm) hergestellt.
Zur Isolierung von RNA werden unterschiedliche Mengen an HeLa-Zellen eingesetzt, die Aufreinigung der Gesamt-RNA wird wie in Beispiel 3 beschrieben durchgeführt. Die Elution wird durch Zugabe von 20 µl NASBA Reaktionspuffer durchgeführt. Die NASBA-Reaktion wird anschließend auf der Membran durchgeführt.
Die Reaktion findet unter Standardbedingungen statt (Fahy, E. et al., 1991. PCR Methods Amplic. 1, 25 - 33). Für die Amplifikation werden β-Aktin spezifische Primer eingesetzt.
Das Reaktionsgefäß wird zunächst für 5 Minuten bei 41 °C in einem Wasserbad inkubiert. Im Anschluß an diesen Schritt wird ein Enzymgemisch bestehend aus RNaseH, T7-Polymerase und AMVV-RT zugegeben und 90 Minuten bei 41 °C inkubiert. Die Amplifikate werden anschließend auf ein nicht denaturierendes Gel aufgetragen und analysiert.

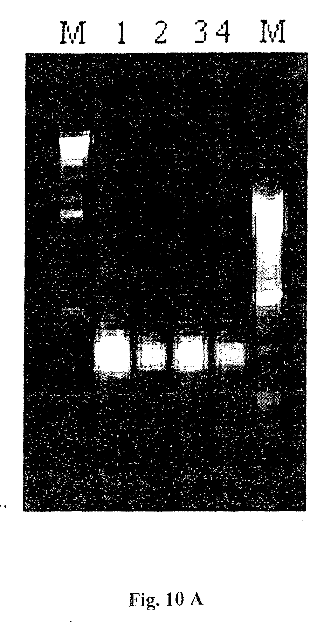

Für die eingesetzte Menge an RNA, isoliert aus 5 x 10⁵ bis 3 x 10⁴ HeLa-Zellen, an mit den hier beschriebenen Verfahren isolierter Gesamt-RNA läßt sich ein spezifisches Transkript nachweisen (Fig. 10).

Fig. 10 zeigt ein Ethidiumbromid-gefärbtes Agarose-Gel einer elektrophoretischen Auftrennung der NASBA-Reaktionen.
A: Spur 1 bis 4: RNA aus HeLa-Zellen mit der Membran Hydrolon 3 µm aufgereinigt;
Spur 1: 2,5 x 10⁵ Zellen;
Spur 2: 1,25 x 10⁵ Zellen;
Spur 3: 6 x 10⁴;
Spur 4: 3 x 10⁴ Zellen.
B: Spur 1 bis 3: RNA aus HeLa-Zellen aufgereinigt;
Spur 1: RNA aus 2,5 x 10⁵ HeLa-Zellen mit der Membran Hydrolon 3 µm aufgereinigt;
Spur 2: RNA aus 5 x 10⁵ HeLa-Zellen mit der Membran Supor-450 PR aufgereinigt;
Spur 3: RNA aus 5 x 10⁵ HeLa-Zellen mit der Membran Fluoropore 3 µm aufgereinigt.

### Beispiel 30

### Restriktion von Plasmid-DNA mit dem Emzym Aval auf einer hydrophoben Membran

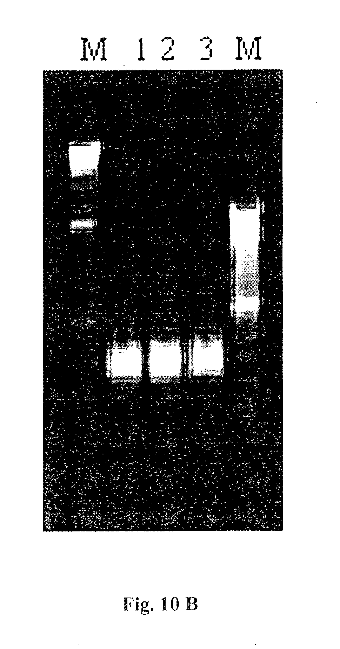

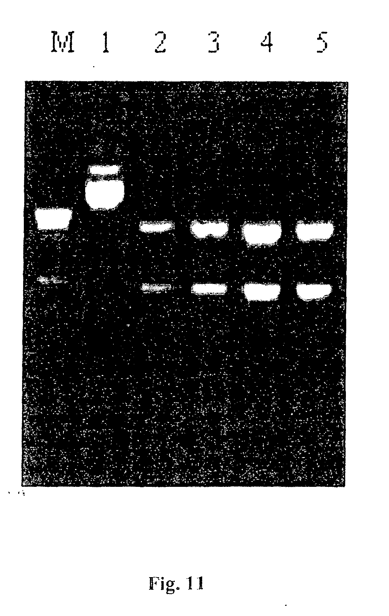

Entsprechend dem Beispiel 1 werden Plastiksäulen mit hydrophoben Membranen (beispielsweise Supor-200 PR der Fa. Pall) hergestellt.
100 µl einer Plasmid enthaltenden wäßrigen Lösung (pCMV? der Fa. Clontech) werden mit 350 µl eines Guanidinium-Isothiocyanat enthaltenden Lysepuffers (4 M GITC, 0,1 M MgSO₄, 25 mM Natrium-Acetat, pH 4) vermischt. Anschließend werden 250 µl Isopropanol zugegeben und durch Auf- und Abpipettieren gemischt. Dieses Gemisch wird dann auf die Säule aufgetragen und entsprechend Beispiel 4 durch die Membran geführt, gewaschen und getrocknet.
100 µl eines 1 x Puffers für das Restriktionsenzym Aval werden auf die Membran gegeben und
1) abgenommen, in ein neues Reaktionsgefäß überführt und anschließend ein Restriktionsenzym (beispielsweise Aval der Fa. Promega) hinzugefügt;
2) ein Restriktionsenzym (beispielsweise Aval der Fa. Promega) zum Eluat in der Säule hinzugefügt.
Die Reaktionen werden eine Stunde bei 37 °C inkubiert und anschließend auf ein nicht denaturierendes Gel aufgetragen und analysiert (Fig. 11).

Fig. 11 zeigt ein Ethidiumbromid-gefärbtes Agarose-Gel einer elektrophoretischen Auftrennung des Plasmides pCMVβ nach Restriktion mit Aval.
Spur 1: ungeschnittenes Plasmid;
Spur 2, 3: Elution mit dem Reaktionspuffer für Aval, Restriktion in neuem Gefäß;
Spur 4, 5: Restriktion mit Aval auf der Membran.

Die Aspekte der vorliegenden Erfindung sind:
1. Ein Verfahren zur Isolierung von Nukleinsäuren mit den folgenden Schritten:
   - Beschicken einer Membran mit zumindest einer Nukleinsäurenprobe;
   - Immobilisieren der Nukleinsäuren an der Membran;
   - Ablösen der immobilisierten Nukleinsäuren von der Membran; und
   - Abnehmen der abgelösten Nukleinsäuren durch die Membran hindurch, wobei die Membran Nylon, Polysulfon, Polyethersulfon, Polycarbonat, Polyacrylat, Acrylatcopolymer, Polyurethan, Polyamid, Polyvinylchlorid, Polyfluorocarbonat, Polytetrafluorethylen, Polyvinylidendifluorid, Polyethylentetrafluorethylen-Copolymerisat, Polybenzimidazole, Polyethylenchlorotrifluorethylen-Copolymerisat, Polyimide, Polyphenylensulfid, Cellulose, Cellulose-Mischester, Cellulosenitrat, Celluloseacetat, Polyacrylnitrile, Polyacrylnitril-Copolymere, Nitrocellulose, Polypropylen und/oder Polyester enthält.
2. Verfahren nach 1, **dadurch gekennzeichnet, dass** das Beschicken von oben und das Abnehmen nach unten erfolgt.
3. Verfahren nach 1 oder 2, **dadurch gekennzeichnet, dass** die Membran in einem Behälter mit einer Zuführung und einer Abführung angeordnet ist und die Membran den gesamten Querschnitt des Behälters ausfüllt.
4. Verfahren nach 1 bis 3, **dadurch gekennzeichnet, dass** die Membran beschichtet ist.
5. Verfahren nach 4, **dadurch gekennzeichnet, dass** die Membran durch die Beschichtung hydrophob gemacht ist.
6. Verfahren nach 4, **dadurch gekennzeichnet, dass** die Membran durch die Beschichtung hydrophil gemacht ist.
7. Verfahren nach 1 bis 6, **dadurch gekennzeichnet, dass** die Membran weniger als 1 mm dick ist.
8. Verfahren nach 7, **dadurch gekennzeichnet, dass** die Membran weniger als 0,5 mm, besonders bevorzugt weniger als 0,2 mm dick ist.
9. Verfahren zur Isolierung von Nukleinsäuren mit den folgenden Schritten:
   - Beschicken einer Oberfläche mit zumindest einer Nukleinsäurenprobe;
   - Immobilisieren der Nukleinsäuren auf der Oberfläche; und
   - Ablösen der immobilisierten Nukleinsäuren von der Oberfläche mit einem Elutionsmittel,
   **dadurch gekennzeichnet, dass** die Ablösung bei einer Temperatur T durchgeführt wird, wobei die Ungleichung 10°C >= T >= T_{S,EM}.gilt und T_{S,EM}.die Gefriertemperatur des Elutionsmittels ist.
10. Verfahren nach 9, **dadurch gekennzeichnet, dass** die Ablösung bei der Temperatur T durchgeführt wird, wobei gilt, dass 10°C>= T >= 5°C.
11. Verfahren nach 9, **dadurch gekennzeichnet, dass** die Ablösung bei der Temperatur T durchgeführt wird, wobei gilt, dass 10°C>= T >= 0°C.
12. Verfahren nach 9, **dadurch gekennzeichnet, dass** die Ablösung bei der Temperatur T durchgeführt wird, wobei gilt, dass 10°C>= T >= -5°C.
13. Verfahren nach 9 bis 12, **dadurch gekennzeichnet, dass** die Ablösung bei der Temperatur T durchgeführt wird,
   wobei gilt, dass 5°C>= T >= T_{S,EM·}
14. Verfahren zur Isolierung von Nukleinsäuren mit den folgenden Schritten:
   - Einstellen einer Nukleinsäurenprobe auf Bindebedingungen, die eine Immobilisierung von in der Probe enthaltenen Nukleinsäuren an eine Oberfläche erlaubt;
   - Beschicken der Oberfläche mit der Nukleinsäurenprobe; und
   - Immobilisieren der Nukleinsäuren auf der Oberfläche,
   **dadurch gekennzeichnet, dass** vor und/oder nach dem Einstellen der Bindebedingungen eine Vorreinigung erfolgt.
15. Verfahren nach 14, **dadurch gekennzeichnet, dass** die Vorreinigung durch Aussalzen erfolgt.
16. Verfahren nach 14 oder 15, **dadurch gekennzeichnet, dass** die Vorreinigung durch Filtrieren, Zentrifugation, enzymatische Behandlung, Temperatureinwirkung, Fällung, Extraktion, Homogenisieren, mechanisches Zerkleinern und/oder Binden von Kontaminanten an Oberflächen erfolgt.
17. Verfahren nach 14 bis 16, **dadurch gekennzeichnet, dass** die Bindebedingungen eine Immobilisierung von RNA ermöglichen.
18. Verfahren nach 14 bis 17, **dadurch gekennzeichnet, dass** die Bindebedingungen eine Immobilisierung von DNA ermöglichen.
19. Verfahren nach 14 bis 18, **dadurch gekennzeichnet, dass** es die weiteren Schritte aufweist:
   - Ablösen der immobilisierten Nukleinsäuren von der Oberfläche; und
   - Abnehmen der abgelösten Nukleinsäuren von der Oberfläche.
20. Verfahren nach 1 bis 13 und 19, **dadurch gekennzeichnet, dass** nach dem Ablöseschritt zumindest einmal folgender Schritt durchgeführt wird:
   - Durchführen zumindest einer chemischen Reaktion an den Nukleinsäuren.
21. Verfahren zur Durchführung einer Nukleinsäure-Amplifikationsreaktion mit den folgenden Schritten:
   - Beschicken einer Oberfläche mit zumindest einer Nukleinsäurenprobe;
   - Immobilisieren der Nukleinsäuren auf der Oberfläche; und
   - Durchführen einer Amplifikations-Reaktion mit den Nukleinsäuren.
22. Verfahren nach 21, **dadurch gekennzeichnet, dass** die Amplifikations-Reaktion nicht isothermal ist.
23. Verfahren nach 21, **dadurch gekennzeichnet, dass** die Amplifikations-Reaktion isothermal ist.
24. Verfahren nach 21 bis 23, **dadurch gekennzeichnet, dass** die Amplifikations-Reaktion eine SDA-Reaktion ("strand displacement amplification"), eine PCR oder eine RT-PCR ist.
25. Verfahren nach 21 bis 24, **dadurch gekennzeichnet, dass** vor dem Durchführen der Amplifikations-Reaktion die Nukleinsäuren mit einem geeigneten Reaktionspuffer von der Oberfläche abgelöst werden und sich das Eluat auf oder in der Membran befindet.
26. Verfahren nach 25, **dadurch gekennzeichnet, dass** es den weiteren Schritt aufweist:
   - Abnehmen der abgelösten Amplifikations-Reaktionsprodukte von der Oberfläche.
27. Verfahren nach 21 bis 24, **dadurch gekennzeichnet, dass** die Amplifikations-Reaktion in einem Reaktionspuffer durchgeführt wird, der nicht zu einer Ablösung der Nukleinsäuren von der Oberfläche führt.
28. Verfahren nach 27, **dadurch gekennzeichnet, dass** es die weiteren Schritte aufweist:
   - Ablösen der Amplifikations-Reaktionsprodukte von der Oberfläche; und
   - Abnehmen der abgelösten Amplifikations-Reaktionsprodukte von der Oberfläche.
29. Verfahren zur Durchführung von chemischen Reaktionen an Nukleinsäuren mit den folgenden Schritten:
   - Beschicken einer Oberfläche mit zumindest einer Nukleinsäurenprobe;
   - Immobilisieren der Nukleinsäuren an der Oberfläche;
   - Ablösen der immobilisierten Nukleinsäuren von der Oberfläche;
   - Durchführen zumindest einer chemischen Reaktion an den Nukleinsäuren; und
   - Abnehmen der Nukleinsäuren von der Oberfläche ohne nochmalige Immobilisierung.
30. Verfahren zur Nukleinsäureanalyse in einem Isoliergefaß mit folgenden Schritten:
   - Bereitstellen eines Isoliergefässes mit einer darin angeordneten Membran;
   - Beschicken des Isoliergefässes mit zumindest einer Nukleinsäurenprobe;
   - Immobilisieren der Nukleinsäuren auf der Membran,
   - Hindurchführen der flüssigen Bestandteile der Probe durch die Membran; und
   - Analysieren von zumindest einer Eigenschaft der Nukleinsäuren auf der im Isoliergefäß angeordneten Membran.
31. Verfahren nach 30, **dadurch gekennzeichnet, dass** nach dem Hindurchführen der flüssigen Bestandteile zumindest eine chemische Reaktion an den Nukleinsäuren durchgeführt wird.
32. Verfahren nach 30 oder 31, **dadurch gekennzeichnet, dass** die analysierte Eigenschaft eine radioaktive Markierung der Nukleinsäuren ist.
33. Verfahren nach 30 oder 32, **dadurch gekennzeichnet, dass** die analysierte Eigenschaft das Bindevermögen der Nukleinsäuren für Moleküle ist.
34. Verfahren nach 33, **dadurch gekennzeichnet, dass** die Moleküle Antikörper sind.
35. Verfahren nach 33, **dadurch gekennzeichnet, dass** die Moleküle Nukleinsäure-bindende Proteine sind.
36. Verfahren nach 33, **dadurch gekennzeichnet, dass** die Moleküle Farbstoffmoleküle sind.
37. Verfahren nach 9 bis 36, **dadurch gekennzeichnet, dass** das Beschicken von oben erfolgt.
38. Verfahren nach 1 bis 13, 19, 20, 25, 26, 28 und 29, **dadurch gekennzeichnet, dass** zwischen dem Immobilisierungs- und dem Ablöseschritt ein Waschen der immobilisierten Nukleinsäuren mit zumindest einem Waschpuffer erfolgt.
39. Verfahren nach 38, **dadurch gekennzeichnet, dass** das Waschen für jeden Waschpuffer folgende Schritte umfasst:
   - Aufbringen einer vorbestimmten Menge an Waschpuffer auf die Oberfläche; und
   - Hindurchführen des Waschpuffers durch die Oberfläche.
40. Verfahren nach 1 bis 13, 19, 20, 25, 26, 28, 29 und 38 bis 39, **dadurch gekennzeichnet, dass** zum Ablösen der Nukleinsäuren eine wässerige Salz- oder Pufferlösung eingesetzt wird.
41. Verfahren nach 1 bis 13, 19, 20, 25, 26, 28, 29 und 38 bis 39, **dadurch gekennzeichnet, dass** zum Ablösen der Nukleinsäuren Wasser eingesetzt wird.
42. Verfahren nach einem der vorstehenden Aspekte, **dadurch gekennzeichnet, dass** das Beschicken und Immobilisieren der Nukleinsäuren folgende Schritte umfasst:
   - Mischen der zumindest einen Nukleinsäurenprobe mit einem Immobilisierungspuffer,
   - Beschicken der zumindest einen Nukleinsäurenprobe mit dem Immobilisierungspuffer auf die Oberfläche; und
   - Hindurchführen der füssigen Bestandteile durch die Oberfläche in im wesentlichen der Richtung der Beschickung.
43. Verfahren nach einem der vorstehenden Aspekte, **dadurch gekennzeichnet, dass** zumindest einer der Schritte durch einen Automaten vollautomatisch durchgeführt wird.
44. Verfahren nach 43, **dadurch gekennzeichnet, dass** alle Schritte des Verfahrens durch einen Automaten in gesteuerter Abfolge durchgeführt werden.
45. Verfahren nach einem der vorstehenden Aspekte, **dadurch gekennzeichnet, dass** eine Mehrzahl von Nukleinsäureisolierungen oder Reaktionen gleichzeitig durchgeführt werden.
46. Verfahren nach einem der vorstehenden Aspekte, **dadurch gekennzeichnet, dass** zum Immobilisieren der Nukleinsäuren wässerige Salzlösungen von Metall- und/oder Ammoniumkationen mit Mineralsäuren eingesetzt werden.
47. Verfahren nach 46, **dadurch gekennzeichnet, dass** zum Immobilisieren der Nukleinsäuren Alkali- und/oder Erdalkalihalogenide und/oder -sulfate und/oder -phosphate eingesetzt werden.
48. Verfahren nach 47, **dadurch gekennzeichnet, dass** zum Immobilisieren der Nukleinsäuren Halogenide des Natriums, Lithiums und/oder Kaliums und/oder Magnesiumsulfat eingesetzt werden.
49. Verfahren nach 1 bis 45, **dadurch gekennzeichnet, dass** zum Immobilisieren der Nukleinsäuren wässerige Lösungen von Salzen von ein- oder mehrbasigen oder polyfunktionellen organischen Säuren mit Alkali- oder Erdalkalimetallen eingesetzt werden.
50. Verfahren nach 49, **dadurch gekennzeichnet, dass** zum Immobilisieren der Nukleinsäuren wässerige Lösungen von Salzen des Natriums, des Kaliums oder des Magnesiums mit organischen Dicarbonsäuren eingesetzt werden.
51. Verfahren nach 50, **dadurch gekennzeichnet, dass** die organische Dicarbonsäure Oxalsäure, Malonsäure und/oder Bemsteinsäure ist.
52. Verfahren nach 49, **dadurch gekennzeichnet, dass** zum Immobilisieren der Nukleinsäuren wässerige Lösungen von Salzen des Natriums oder des Kaliums mit einer Hydroxy- oder Polyhydroxycarbonsäure eingesetzt werden.
53. Verfahren nach 52, **dadurch gekennzeichnet, dass** die Polyhydroxycarbonsäure Zitronensäure ist.
54. Verfahren nach 1 bis 45, dadurch gekenneichnet, dass zum Immobilisieren der Nukleinsäuren Hydroxylderivate von aliphatischen oder acyclischen gesättigten oder ungesättigten Kohlenwasserstoffen eingesetzt werden.
55. Verfahren nach 54, **dadurch gekennzeichnet, dass** als Hydroxylderivate C1-C5-Alkanole eingesetzt werden.
56. Verfahren nach 55, **dadurch gekennzeichnet, dass** als C1-C5-Alkanole Methanol, Ethanol, n-Propanol, tert.-Butanol und/oder Pentanole eingesetzt werden.
57. Verfahren nach 54, **dadurch gekennzeichnet, dass** als Hydroxylderivat ein Aldit eingesetzt wird.
58. Verfahren nach 1 bis 45, **dadurch gekennzeichnet, dass** zum Immobilisieren der Nukleinsäuren ein Phenol oder Polyphenol eingesetzt wird.
59. Verfahren nach einem der vorstehenden Aspekte, **dadurch gekennzeichnet, dass** zum Immobilisieren der Nukleinsäuren zumindest ein chaotropes Agens eingesetzt wird.
60. Verfahren nach 59, **dadurch gekennzeichnet, dass** das chaotrope Agens ein Salz aus der Gruppe der Trichloracetate, Thiocyanate, Perchlorate, lodide oder Guanidinium-Hydrochlorid, Guanidinium-iso-thiocyanat oder Harnstoff ist.
61. Verfahren nach 59 oder 60, **dadurch gekennzeichnet, dass** 0,01 molare bis 10 molare wässerige Lösungen des zumindest einen chaotropen Agens allein oder in Kombination mit anderen Salzen zum Immobilisieren der Nukleinsäuren eingesetzt werden.
62. Verfahren nach 61, **dadurch gekennzeichnet, dass** 0,1 molare bis 7 molare wässerige Lösungen des zumindest einen chaotropen Agens allein oder in Kombination mit anderen Salzen zum Immobilisieren der Nukleinsäuren eingesetzt werden.
63. Verfahren nach 62, **dadurch gekennzeichnet, dass** 0,2 molare bis 5 molare wässerige Lösungen des zumindest einen chaotropen Agens allein oder in Kombination mit anderen Salzen zum Immobilisieren der Nukleinsäuren eingesetzt werden.
64. Verfahren nach 59 bis 63, **dadurch gekennzeichnet, dass** eine wässerige Lösung von Natriumperchlorat, Guanidinium-Hydrochlorid, Guanidinium-iso-thiocyanat, Natriumiodid und/oder Kaliumiodid zum Immobilisieren der Nukleinsäuren eingesetzt wird.
65. Verfahren nach 38 bis 64, **dadurch gekennzeichnet, dass** zum Waschen der immobilisierten Nukleinsäuren eine Salz- oder eine Pufferlösung gemäß 46 bis 64 eingesetzt wird.
66. Verfahren nach 9 bis 65, **dadurch gekennzeichnet, dass** die Oberfläche eine Membran ist.
67. Verfahren nach 1 bis 8 und 66, **dadurch gekennzeichnet, dass** die Membran eine hydrophobe Membran ist.
68. Verfahren nach 67, **dadurch gekennzeichnet, dass** die hydrophobe Membran aus einem Polymer mit polaren Gruppen aufgebaut ist.
69. Verfahren nach 67 oder 68, **dadurch gekennzeichnet, dass** die Membran eine hydrophile Membran mit einer hydrophobisierten Oberfläche ist.
70. Verfahren nach 67 bis 69, **dadurch gekennzeichnet, dass** die Membran aus Nylon, einem Polysulfon, Polyethersulfon, Polycarbonat, Polypropylen, Polyacrylat Acrylatcopolymeren, Polyurethan, Polyamid, Polyvinylchlorid, Polyfluorocarbonat, Polytetrafluoroethylen, Polyvinylidendifluorid, Polyethylentetrafluoroethylen-Copolymerisat, einem Polyethylenchlorotrifluoroethylen-Copolymerisat, Celluloseacetat, Nitrocellulose, Polybenzimidazole, Polyimide, Polyacrylnitrile, Polyacrylnitril-Copolymere, Cellulosemischester, Cellulosenitrat, oder Polyphenylensulfid besteht.
71. Verfahren nach 69 oder 70, **dadurch gekennzeichnet, dass** die Membran aus einem hydrophobisierten Nylon besteht.
72. Verfahren nach 69 bis 71, **dadurch gekennzeichnet, dass** die Membran mit einem Hydrophobisierungsmittel aus der Gruppe der Paraffine, Wachse, Metallseifen, ggf. mit Zusätzen an Aluminium bzw. Zirkoniumsalzen, quartären organischen Verbindungen, Harnstoffderivate, fettstoffmodifizierten Melaminharze, Silicone, zinkorganischen Verbindungen und/oder mit Glutardialdehyd beschichtet ist.
73. Verfahren nach 1 bis 8 und 66, **dadurch gekennzeichnet, dass** die Membran eine hydrophile oder hydrophilisierte Membran ist.
74. Verfahren nach 73, **dadurch gekennzeichnet, dass** die Membran aus hydrophilisiertem Nylon, Polyethersulfon, Polycarbonat, Polyacrylat, Acrylatcopolymeren, Polyurethan, Polyamid, Polyvinylchlorid, Polyfluorocarbonat, Polytetrafluoroethylen, Polyvinylidendifluorid, Polyethylentetrafluoroethylen-Copolymerisat, einem Polyethylenchlorotrifluoroethylen-Copolymerisat, Celluloseacetat, Polypropylen, Nitrocellulose, Polybenzimidazole, Polyimide, Polyacrylnitrile, Polyacrylnitril-Copolymere, Cellulosemischester, Polyester, Polysulfon, Cellulosenitrat oder Polyphenylensulfid besteht.
75. Verfahren nach 1 bis 8 und 66 bis 74, **dadurch gekennzeichnet, dass** die Membran einen Porendurchmesser von 0,001 bis 50 Mikrometer, vorzugsweise 0,01 bis 20 Mikrometer, besonders bevorzugt 0,05 bis 10 Mikrometer besitzt.
76. Verfahren nach 9 bis 27, **dadurch gekennzeichnet, dass** die Oberfläche ein hydrophobes Vlies ist.
77. Automat, **dadurch gekennzeichnet, dass** er das Verfahren nach 1 bis 76 ausführen kann.
78. Automat nach 77, **dadurch gekennzeichnet, dass** er mit zumindest eine Saugvorrichtung ausgestattet ist, die das Zugeben von Puffern und Lösungen auf die Oberfläche und von der Oberfläche weg ausführt oder ausführen kann.
79. Isoliergefäß zur Isolierung von Nukleinsäuren mit
   zumindest einem zylinderförmigem Oberteil mit einer oberen Öffnung, einer unteren Öffnung und einer Membran, die an der unteren Öffnung angeordnet ist und den gesamten Querschnitt des Oberteils ausfüllt;
   einem Unterteil mit einem absorbierenden Material; und
   einem Mechanismus zur Verbindung zwischen Oberteil und Unterteil,
   wobei bei hergestellter Verbindung die Membran in Kontakt mit dem absorbierenden Material ist und bei nicht hergestellter Verbindung die Membran nicht in Kontakt mit dem absorbierenden Material ist.
80. Isoliergefäß nach 79, **dadurch gekennzeichnet, dass** das Unterteil ein Zylinder gleichen Durchmessers wie das Oberteil ist.
81. Isoliergefäß nach 79 oder 80, **dadurch gekennzeichnet, dass** der Mechanismus ein Anschluß ist, der eine räumliche Trennung von Oberteil und Unterteil erlaubt.
82. Isoliergefäß nach 81, **dadurch gekennzeichnet, dass** der Anschluß ein Bajonettanschluß ist.
83. Isoliergefäß nach 81, **dadurch gekennzeichnet, dass** der Anschluß ein Schraubanschluß ist.
84. Isoliergefäß nach 79 oder 80, **dadurch gekennzeichnet, dass** der Mechanismus ein Schieber ist, der zwischen absorbierendem Material und Membran einschiebbar ist.
85. Isoliergefäß nach 79 oder 80, **dadurch gekennzeichnet, dass** der Mechanismus eine Sollbruchstelle zwischen Oberteil und Unterteil ist.
86. Isoliergefäß nach 79 bis 85, **dadurch gekennzeichnet, dass** das Oberteil ein Röhrchen ist, welches in Reaktionsgefäßhalter einstellbar ist.
87. Isoliergefäß nach 79 bis 86, dadurch gekennzeichnet, dass Oberteil und Unterteil ein Röhrchen bilden, welches in Reaktionsgefäßbehälter einstellbar ist.
88. Isoliergefäß nach 79 bis 86, **dadurch gekennzeichnet, dass** mehrere Oberteile auf einem Unterteil angeordnet sind.
89. Isoliergefäß nach 79 bis 88, **dadurch gekennzeichnet, dass** das absorbierende Material einen Schwamm aufweist.
90. Isoliergefäß nach 79 bis 89, **dadurch gekennzeichnet, dass** das absorbierende Material ein Granulat enthält.
91. Verwendung des Isoliergefässes nach 79 bis 90 zur Analyse der Eigenschaften von Nukleinsäuren.
92. Verwendung des Isoliergefässes nach 79 bis 90 zur Isolierung von Nukleinsäuren.
93. Verwendung eines Isoliergefässes nach einem der Aspekte 79 bis 92 als Reaktionsgefäß zur dauerhaften Immobilisierung von Nukleinsäuren an eine Membran, die den gesamten Querschnitt des Isoliergefässes ausfüllt.
94. Isoliergefäß zur Isolierung von Nukleinsäuren mit
   zumindest einem Oberteil mit einer oberen Öffnung, einer unteren Öffnung und einer Membran, die an der unteren Öffnung angeordnet ist und den gesamten Querschnitt des Oberteils ausfüllt;
   einem Unterteil mit einem absorbierenden Material;
   und einem das Oberteil zumindest im Bereich der Membran umgebenden Mantel zur Aufnahme eines Kühlmittels.
95. Isoliergefäß nach 94, **dadurch gekennzeichnet, dass** der Mantel zwei Kompartimente aufweist, die voneinander durch eine mechnisch zerstörbare Trennwand getrennt sind und jedes der Kompartimente eine Lösung enthält,
   wobei bei Mischung der beiden Lösungen nach Zerstörung der Trennwand das Kühlmittel entsteht.
96. Verwendung von Celluloseacetat, nicht carboxyliertem, hydrophoben Polyvinylidendifluorid, oder massivem, hydrophobem Polytetrafluorethylen als Material zur Anlagerung und Isolierung von Nukleinsäuren.
97. Verwendung nach 96, **dadurch gekennzeichnet, dass** das Material in Membranform verwendet wird.
98. Verwendung nach 96, **dadurch gekennzeichnet, dass** das Material als Granulat verwendet wird.
99. Verwendung nach 96, **dadurch gekennzeichnet, dass** das Material in Faserfom verwendet wird.
100. Verwendung nach 99, **dadurch gekennzeichnet, dass** die Fasern als Vlies angeordnet sind.
101. Kit zur Nukleinsäurenisolierung mit
   - einem Immobilisierungspuffer;
   - einem Elutionspuffer; und
   - zumindest einem Isoliergefäß nach 79 bis 89 oder 94.
102. Kit nach 101, **dadurch gekennzeichnet, dass** es weiterhin umfasst einen Waschpuffer.
103. Kit nach 101oder 102, **dadurch gekennzeichnet, dass** es weiterhin umfasst einen Lysepuffer.
104. Kit nach 101 bis 103, **dadurch gekennzeichnet, dass** es zur Durchführung der Verfahren nach 1 bis 76 geeignet ist.

## Patentansprüche

1. Verfahren zur Isolierung von Nukleinsäuren mit den folgenden Schritten:
- Beschicken einer Membran mit zumindest einer Nukleinsäurenprobe;
- Immobilisieren der Nukleinsäuren an der Membran;
- Ablösen der immobilisierten Nukleinsäuren von der Membran; und
- Abnehmen der abgelösten Nukleinsäuren durch die Membran hindurch, wobei die Membran Nylon, Polysulfon, Polyethersulfon, Polycarbonat, Polyacrylat, Acrylatcopolymer, Polyurethan, Polyamid, Polyvinylchlorid, Polyfluorocarbonat, Polytetrafluorethylen, Polyvinylidendifluorid, Polyethylentetrafluorethylen-Copolymerisat, Polybenzimidazole, Polyethylenchlorotrifluorethylen-Copolymerisat, Polyimide, Polyphenylensulfid, Cellulose, Cellulose-Mischester, Cellulosenitrat, Celluloseacetat, Polyacrylnitrile, Polyacrylnitril-Copolymere, Nitrocellulose, Polypropylen und/oder Polyester enthält.

2. Verfahren zur Isolierung von Nukleinsäuren mit den folgenden Schritten:
- Einstellen einer Nukleinsäurenprobe auf Bindebedingungen, die eine Immobilisierung von in der Probe enthaltenen Nukleinsäuren an eine Oberfläche erlaubt;
- Beschicken der Oberfläche mit der Nukleinsäurenprobe; und
- Immobilisieren der Nukleinsäuren auf der Oberfläche,
**dadurch gekennzeichnet, dass** vor und/oder nach dem Einstellen der Bindebedingungen eine Vorreinigung erfolgt.

3. Verfahren nach Anspruch 2, **dadurch gekennzeichnet, dass** es die weiteren Schritte aufweist:
- Ablösen der immobilisierten Nukleinsäuren von der Oberfläche; und
- Abnehmen der abgelösten Nukleinsäuren von der Oberfläche.

4. Verfahren nach einem der Ansprüche 1 oder 3, **dadurch gekennzeichnet, dass** nach dem Ablöseschritt zumindest einmal folgender Schritt durchgeführt wird:
- Durchführen zumindest einer chemischen Reaktion an den Nukleinsäuren.

5. Verfahren zur Durchführung einer Nukleinsäure-Amplifikationsreaktion mit den folgenden Schritten:
- Beschicken einer Oberfläche mit zumindest einer Nukleinsäurenprobe;
- Immobilisieren der Nukleinsäuren auf der Oberfläche; und
- Durchführen einer Amplifikations-Reaktion mit den Nukleinsäuren.

6. Verfahren nach Anspruch 5, **dadurch gekennzeichnet, dass** vor dem Durchführen der Amplifikations-Reaktion die Nukleinsäuren mit einem geeigneten Reaktionspuffer von der Oberfläche abgelöst werden und sich das Eluat auf oder in der Membran befindet.

7. Verfahren nach Anspruch 6, **dadurch gekennzeichnet, dass** es den weiteren Schritt aufweist:
- Abnehmen der abgelösten Amplifikations-Reaktionsprodukte von der Oberfläche.

8. Verfahren nach Anspruch 5, **dadurch gekennzeichnet, dass** die Amplifikations-Reaktion in einem Reaktionspuffer durchgeführt wird, der nicht zu einer Ablösung der Nukleinsäuren von der Oberfläche führt.

9. Verfahren nach Anspruch 8, **dadurch gekennzeichnet, dass** es die weiteren Schritte aufweist:
- Ablösen der Amplifikations-Reaktionsprodukte von der Oberfläche; und
- Abnehmen der abgelösten Amplifikations-Reaktionsprodukte von der Oberfläche.

10. Verfahren zur Durchführung von chemischen Reaktionen an Nukleinsäuren mit den folgenden Schritten:
- Beschicken einer Oberfläche mit zumindest einer Nukleinsäurenprobe;
- Immobilisieren der Nukleinsäuren an der Oberfläche;
- Ablösen der immobilisierten Nukleinsäuren von der Oberfläche;
- Durchführen zumindest einer chemischen Reaktion an den Nukleinsäuren; und
- Abnehmen der Nukleinsäuren von der Oberfläche ohne nochmalige Immobilisierung.

11. Verfahren zur Nukleinsäureanalyse in einem Isoliergefaß mit folgenden Schritten:
- Bereitstellen eines Isoliergefässes mit einer darin angeordneten Membran;
- Beschicken des Isoliergefässes mit zumindest einer Nukleinsäurenprobe;
- Immobilisieren der Nukleinsäuren auf der Membran,
- Hindurchführen der flüssigen Bestandteile der Probe durch die Membran; und
- Analysieren von zumindest einer Eigenschaft der Nukleinsäuren auf der im Isoliergefäß angeordneten Membran.

12. Verfahren nach einem der vorstehenden Ansprüche, **dadurch gekennzeichnet, dass** zumindest einer der Schritte durch einen Automaten vollautomatisch durchgeführt wird.

13. Isoliergefäß zur Isolierung von Nukleinsäuren mit
zumindest einem zylinderförmigem Oberteil mit einer oberen Öffnung, einer unteren Öffnung und einer Membran, die an der unteren Öffnung angeordnet ist und den gesamten Querschnitt des Oberteils ausfüllt;
einem Unterteil mit einem absorbierenden Material; und
einem Mechanismus zur Verbindung zwischen Oberteil und Unterteil,
wobei bei hergestellter Verbindung die Membran in Kontakt mit dem absorbierenden Material ist und bei nicht hergestellter Verbindung die Membran nicht in Kontakt mit dem absorbierenden Material ist.

14. Verwendung eines Isoliergefässes nach Anspruch 13 als Reaktionsgefäß zur dauerhaften Immobilisierung von Nukleinsäuren an eine Membran, die den gesamten Querschnitt des Isoliergefässes ausfüllt.

15. Isoliergefäß zur Isolierung von Nukleinsäuren mit
zumindest einem Oberteil mit einer oberen Öffnung, einer unteren Öffnung und einer Membran, die an der unteren Öffnung angeordnet ist und den gesamten Querschnitt des Oberteils ausfüllt;
einem Unterteil mit einem absorbierenden Material;
und einem das Oberteil zumindest im Bereich der Membran umgebenden Mantel zur Aufnahme eines Kühlmittels.

16. Kit zur Nukleinsäurenisolierung mit
- einem Immobilisierungspuffer;
- einem Elutionspuffer; und
- zumindest einem Isoliergefäß nach einem der Ansprüche 14 oder 15.
